# EUROPEAN PATENT SPECIFICATION

(11) **EP 2 689 249 B1**
(45) Date of publication and mention of the grant of the patent: **25.05.2016**
(21) Application number: 12760950.1
(22) Date of filing: 22.03.2012
(51) Int. Cl.: G01N 33/574

(54) **ASSAY FOR SCREENING COMPOUNDS THAT SELECTIVELY DECREASE THE NUMBER OF CANCER STEM CELLS**
ASSAY ZUM SCREENING VON VERBINDUNGEN ZUR SELEKTIVEN VERMINDERUNG DER ANZAHL VON KREBSSTAMMZELLEN
ESSAI DE CRIBLAGE DE COMPOSÉS QUI DIMINUENT DE MANIÈRE SÉLECTIVE LE NOMBRE DE CELLULES SOUCHES CANCÉREUSES

(30) Priority: 24.03.2011 US 201161467265 P
(43) Date of publication of application: 29.01.2014
(73) Proprietor: THE ROGOSIN INSTITUTE, New York, NY 10021 (US)
(72) Inventor: SMITH, Barry, New York, NY 10021 (US); CORDON-CARDO, Carlos, New York, NY 10027 (US); PETRYLAK, Daniel, New York, NY 10027 (US); DOMENECH, Josep, New York, NY 10027 (US); MARTIN, Mireia, Castilla, New York, NY 10027 (US)
(74) Representative: Zaccaro, Elisabetta
(86) International application number: PCT/US2012/030103
(87) International publication number: WO 2012/129393

(56) References cited:
- US-A1- 2008 118 432
- US-A1- 2008 132 423
- US-A1- 2011 020 221
- GALMOZZI E ET AL: "Cancer stem cells and therapeutic perspectives", CURRENT MEDICINAL CHEMISTRY, BENTHAM SCIENCE PUBLISHERS, NL, vol. 13, no. 6, 1 January 2006 (2006-01-01), pages 603-607, XP009107987, ISSN: 0929-8673, DOI: 10.2174/092986706776055661
- PÉREZ-CARO AND I SANCHEZ-GARCIA M: "killing time for cancer stem cells (CSC): discovery and development of selective CSC inhibitors", CURRENT MEDICINAL CHEMISTRY, BENTHAM SCIENCE PUBLISHERS, NL, vol. 13, 1 January 2006 (2006-01-01), pages 1719-1725, XP002663777, ISSN: 0929-8673
- JIANGBING ZHOU ET AL: "Cancer stem/progenitor cell active compound 8-quinolinol in combination with paclitaxel achieves an improved cure of breast cancer in the mouse model", BREAST CANCER RESEARCH AND TREATMENT, KLUWER ACADEMIC PUBLISHERS, BO, vol. 115, no. 2, 28 May 2008 (2008-05-28), pages 269-277, XP019671272, ISSN: 1573-7217
- PIYUSH B. GUPTA ET AL: "Identification of Selective Inhibitors of Cancer Stem Cells by High-Throughput Screening", CELL, vol. 138, no. 4, 1 August 2009 (2009-08-01), pages 645-659, XP55001903, ISSN: 0092-8674, DOI: 10.1016/j.cell.2009.06.034
- WINQUIST R J ET AL: "Cancer stem cells as the relevant biomass for drug discovery", CURRENT OPINION IN PHARMACOLOGY, ELSEVIER SCIENCE PUBLISHERS, NL, vol. 10, no. 4, 1 August 2010 (2010-08-01) , pages 385-390, XP027189647, ISSN: 1471-4892 [retrieved on 2010-07-12]
- B. H. SMITH ET AL: "Three-Dimensional Culture of Mouse Renal Carcinoma Cells in Agarose Macrobeads Selects for a Subpopulation of Cells with Cancer Stem Cell or Cancer Progenitor Properties", CANCER RESEARCH, vol. 71, no. 3, 1 February 2011 (2011-02-01), pages 716-724, XP55050161, ISSN: 0008-5472, DOI: 10.1158/0008-5472.CAN-10-2254
- ULASOV ET AL.: 'Inhibition of Sonic Hedgehog and Notch Pathways Enhances Sensitivity of CD133+ Glioma Stem Cells to Temozolomide Therapy.' MOL MED. vol. 17, 15 October 2010, pages 103 - 112, XP055127709
- Fabio Morandi ET AL: "Immunogenicity of Human Mesenchymal Stem Cells in HLA-Class I-Restricted T-Cell Responses Against Viral or Tumor-Associated Antigens", Stem Cells, vol. 26, no. 5, 1 May 2008 (2008-05-01), pages 1275-1287, XP055187877, ISSN: 1066-5099, DOI: 10.1634/stemcells.2007-0878

## Description

### FIELD OF THE INVENTION

This invention is directed to, *inter alia,* methods that are useful, for example, for identifying agents that selectively decrease the number of cancer stem cells in a population of cells.

### BACKGROUND

Cancer is a term used to define a group of diseases characterized by unregulated cell proliferation, aberrant differentiation, and defective apoptosis. These neoplastic diseases may all have in common an initial transforming event that is manifold in nature (e.g., viral infection, chemical carcinogens, etc.) and impacts a unique tissue cell, the so-called "adult stem cell." It is believed that these transforming events generate a "tumor/cancer stem cell (CSC)" that is responsible for tumor initiation and the hierarchical organization of cancer. However, such cell has not yet been definitively identified nor subsequently characterized.

Tumors consist of heterogeneous populations of cells that differ in growth capacities, morphology and marker expression. The cancer stem cell hypothesis posits that in addition to CSCs, tumors comprise a small population of transit amplifying clonogens, and large sets of differentiated malignant cells (Reya *et al.,* 2008; Jordan *et al.,* 2006; Dalerba *et al.,* 2007; Vermeulen *et al.,* 2008). Cancer stem cells, like their normal stem cell counterparts, should be undifferentiated and display asymmetrical cell division, resulting in self-renewal and production of differentiated clonogens, thus contributing to tumor heterogeneity. In addition, CSCs have been postulated to be refractory to standard chemotherapy treatments, though such treatments frequently result in eradication of the fastest dividing cells, resulting in tumor response, but not in CSC depletion (Gupta *et al.,* 2009; Sharma *et a*/., 2010; Bao *et al.,* 2006; Trumpp *et al.,* 2008; Dean *et al.,* 2005; Costello *et al.,* 2000; Guzman *et al.,* 2002). This model explains why standard human cancer chemotherapy frequently results in initial tumor shrinkage, though most cancers eventually recur due to regeneration by surviving CSCs. Thus from a clinical point of view, the identification and molecular characterization of CSCs has fundamental implications for cancer diagnosis, prognosis and novel therapeutic approaches, including targeted treatments.

In solid tumors, CSCs have been previously claimed to be identified by cell surface immunophenotyping and tumor initiating capacity. These putative CSC subpopulations were shown to display a CD44⁺/CD24^{-/low} and α6-integrin phenotype in breast cancer (Al-Hajj *et al.,* 2003; Cariati *et al.,* 2008; Fillmore *et al.,* 2008; Ponti *et al.,* 2005), a CD133⁺/Nestin⁺ phenotype in brain tumors (Singh *et al.,* 2004), and a CD133⁺ phenotype in colon cancer (Ricci-Vitiani *et al.,* 2007; O'Brien *et al.,* 2007), among others. In prostate cancer, a similar subpopulation of putative CSCs that expresses high levels of CD44 has also been described (Collins *et al.,* 2005; Patrawala *et al.,* 2006; Li *et al.,* 2008; Patrawala *et al.,* 2007). Perhaps the most challenging issue facing the field is that the described CSC subpopulations do not always fulfill the classical properties that define "stemness", mainly the ability to generate differentiated progeny by asymmetrical cell division. Furthermore, the existence of the above-mentioned surface phenotype subpopulations vary dramatically in tumor tissue samples from patients with the same histopathological diagnosis. In some cases, these subpopulations are relatively rare, whereas in others they constitute a large fraction of tumor mass (Quintana *et al.,* 2008; Rosen *et al.,* 2009; Chen *et al.,* 2010; Shmelkov *et al.,* 2008). These observations highlight the complexity of a consistent identification of CSCs.

Lack of consistency in identifying CSCs by their characteristics such as stemness has made a reliable search for agents targeting CSCs untenable. In particular, identification of agents that may modulate differentiation or other processes affecting the percentage of CSCs in a population of cells has not been feasible.

### SUMMARY OF THE INVENTION

One embodiment of the present invention is a method for identifying an agent that selectively decreases the number of cancer stem cells (CSCs). This method comprises:
a. contacting a CSC from a population of cells with a candidate agent; and
b. determining whether the candidate agent reduces the survival or growth of the CSC or increases differentiation of the CSC relative to a CSC that has not been contacted with the candidate agent, wherein the CSC is a cancer cell that is HLA⁻.

Another embodiment of the invention is a high throughput screening method for identifying agents that selectively decrease the percentage of cancer stem cells (CSCs) in a population of cells. This screening method comprises:
a. contacting a CSC from a population of cells with a candidate agent;
b. determining whether the candidate agent reduces the survival or growth or increases the differentiation of the CSC relative to a CSC that has not been contacted with the candidate agent by:
   i. comparing the percentage of CSCs in a population of cells, which is treated with the candidate agent to the percentage of CSCs in a population of cells, which is not treated with the candidate agent;
   ii. determining the percentage of viable cells in the treated and untreated population of cells,
   iii. determining the percentage of non-CSC cells in the treated and untreated population of cells; and
   iv. determining the percentage of dividing cells in the treated
and untreated population of cells,
wherein a candidate agent that (1) decreases the percentage of CSCs and decreases cell viability; (2) decreases the percentage of CSCs without a decrease in cell viability; (3) decreases the percentage of CSCs and increases the percentage of non-CSCs; (4) decreases the percentage of CSCs and decreases cell division; or (5) decreases the percentage of CSCs without a decrease in cell division is an agent that selectively decreases the percentage of CSCs; wherein the CSC is a cancer cell that is HLA⁻.

Another embodiment of the invention is a high throughput screening method for identifying agents that selectively decrease the percentage of cancer stem cells (CSCs) in a population of cells, wherein the CSCs are refractory to standard chemotherapy. This screening method comprises:
a. contacting a CSC from a population of cells with a candidate agent, wherein the CSC has the following properties: HLA I⁻ and HLA II⁻;
b. determining whether the candidate agent reduces the survival or growth or increases differentiation of the CSC relative to a CSC that has not been contacted with the candidate agent by:
   i. comparing, using flow cytometry, the percentage of CSCs in the population of cells, which is treated with the candidate agent to the percentage of CSCs in a population of cells, which is not treated with the candidate agent;
   ii. determining, using a cell viability assay, the percentage of viable cells in the treated and untreated population of cells,
   iii. determining, using a cell differentiation assay, the percentage of non-CSC cells in the treated and untreated population of cells; and
   iv. determining, using a cell division assay, the percentage of dividing cells in the treated and untreated population of cells,
   wherein a candidate agent that (1) decreases the percentage of CSCs and decreases cell viability; (2) decreases the percentage of CSCs without a decrease in cell viability; (3) decreases the percentage of CSCs and increases the percentage of non-CSCs; (4) decreases the percentage of CSCs and decreases cell division; or (5) decreases the percentage of CSCs without a decrease in cell division is an agent that selectively decreases the percentage of CSCs.

A further embodiment is an agent for selectively killing CSCs identified by any methods of the present invention.

An additional embodiment is a pharmaceutical composition. This pharmaceutical composition comprises a pharmaceutically acceptable carrier and an agent as disclosed herein.

### BRIEF DESCRIPTION OF THE DRAWINGS

This patent or application file contains at least one drawing executed in color. Copies of this patent or patent application publication with color drawing(s) will be provided by the Office upon request and payment of the necessary fee.

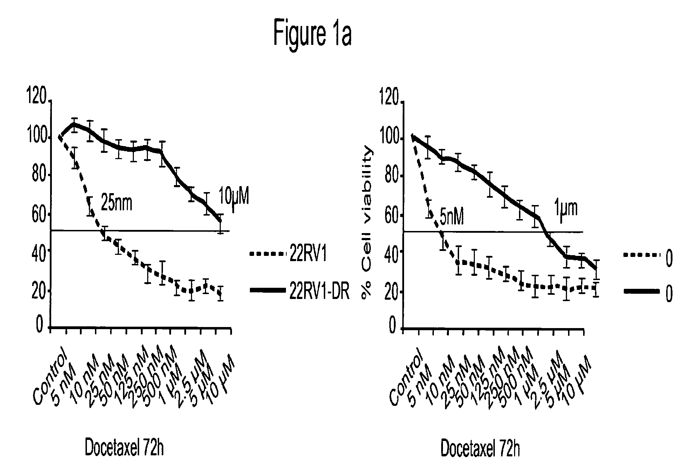

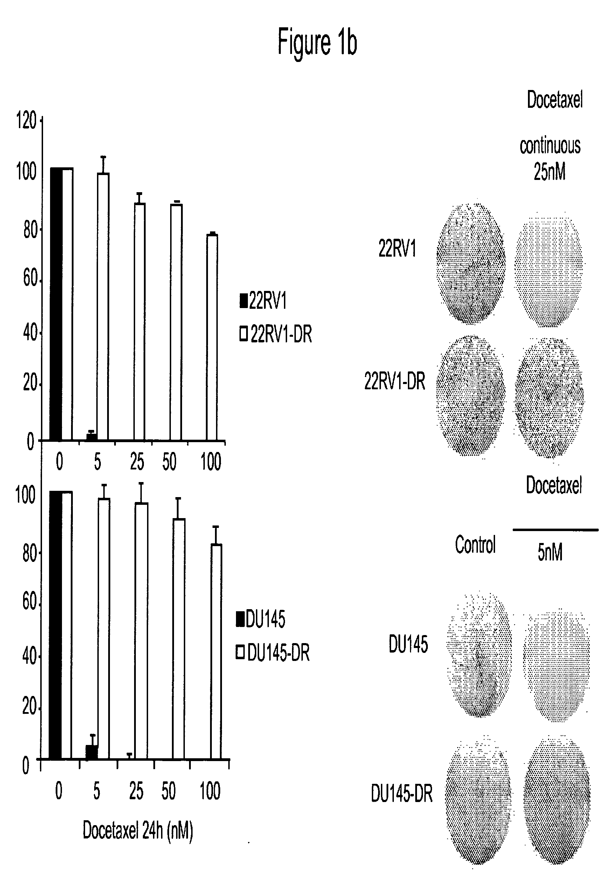

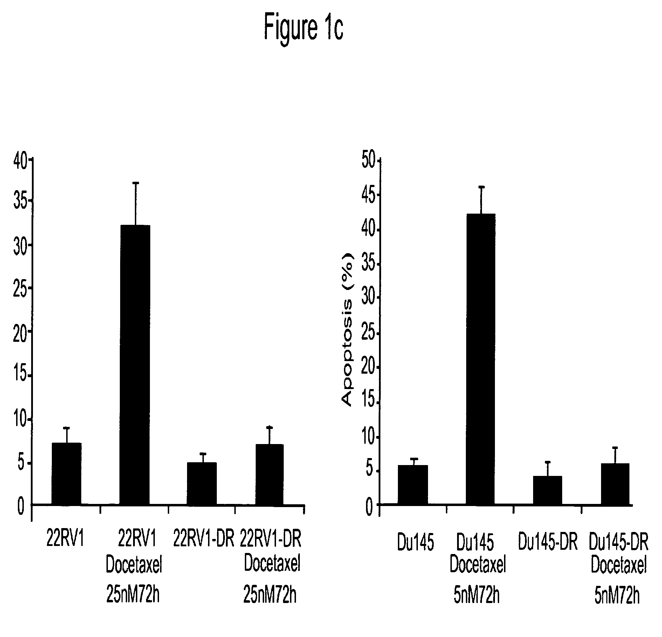

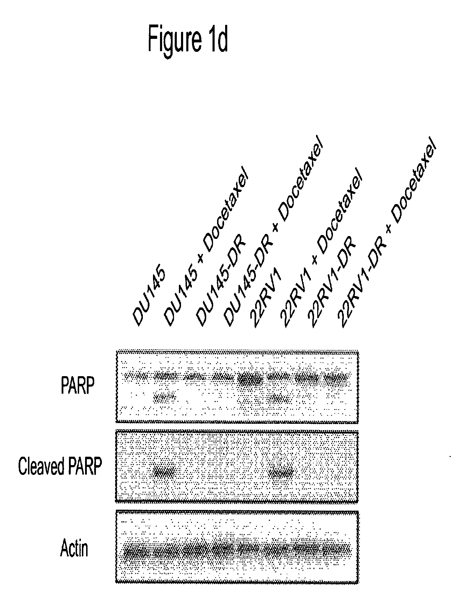

Figure 1 shows characterization of Docetaxel acquired resistance in hormone independent prostate cancer cells. Figure 1 a shows the results of cell viability assays (MTs) in the parental cells (22RVI (left panel) and DU145 (right panel)) and Docetaxel acquired resistant cells (22RVI-DR and DUI45-DR) treated with increasing doses of Docetaxel. A red line designates the IC₅₀ concentration of the drug (Docetaxel) for sensitive and resistant cells. 22RV1-DR Docetaxel IC₅₀ increased from 25nM to 10µM (400 fold increase) and DU145-DR Docetaxel IC₅₀ increased from 5nM to 1µM (200 fold increase). The left panels of Figure 1b show quantitative analysis of colony formation assays in parental and Docetaxel resistant cells treated with increasing doses of Docetaxel for 24 hours. The right panels of Figure 1b show representative colony formation assays of cells treated continuously for 21 days with Docetaxel (25nM in 22RV1 cells and 5nM in DU145 cells). No colonies were observed after continuous administration of Docetaxel in the sensitive cells, whereas colonies were observed in the resistant cells. Similar effects were observed after 24 hour exposure to the drug. Figure 1c shows the results of flow cytometry assessment of apoptosis by annexin-V and propidium iodide staining of cells exposed to DMSO (control) and Docetaxel. Figure 1d shows a Western blot analysis of PARP cleavage of various cell types as indicated. Results shown in Figure 1 (c) and (d) demonstrate that the acquired Docetaxel resistance phenotype is linked to a lack of Docetaxel apoptotic response.

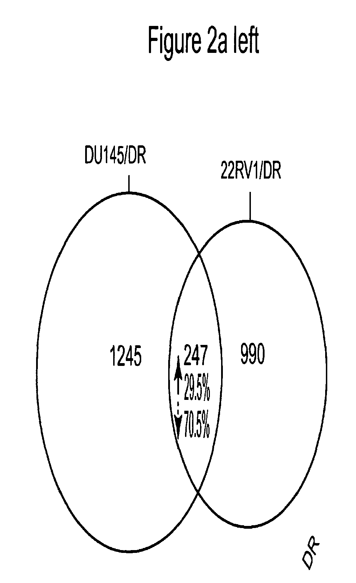

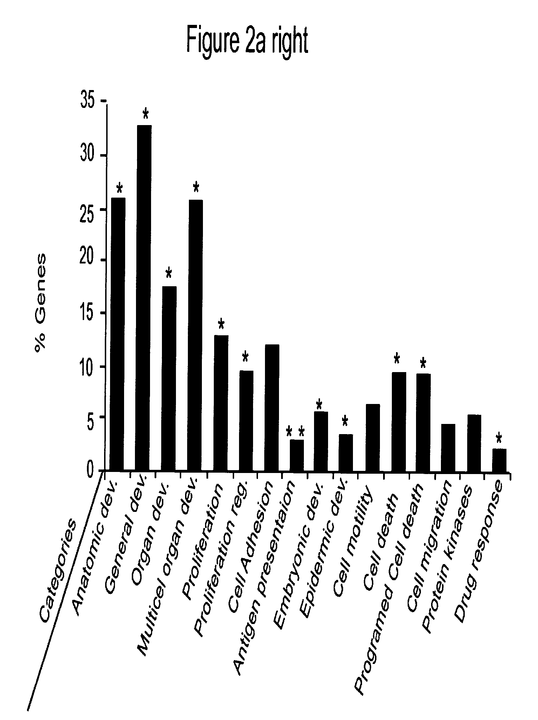

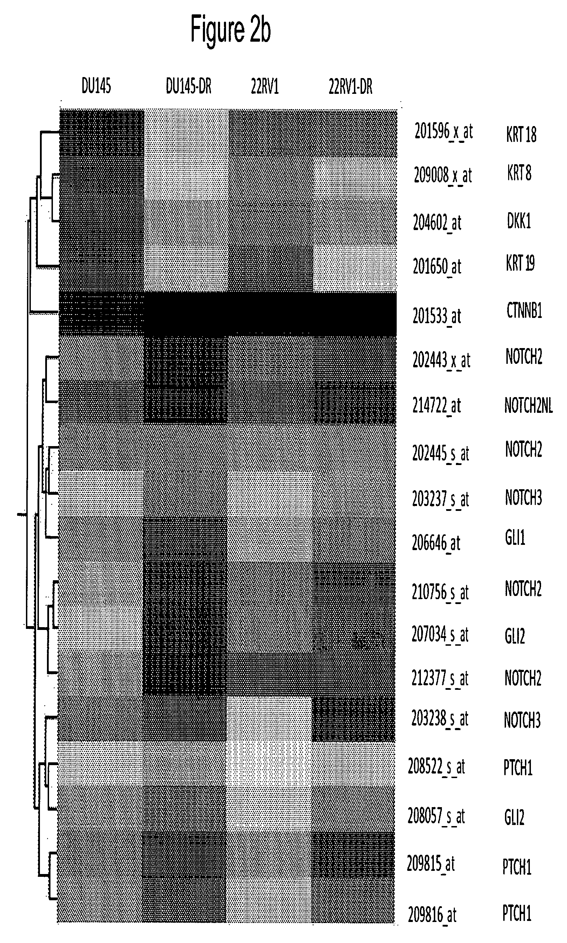

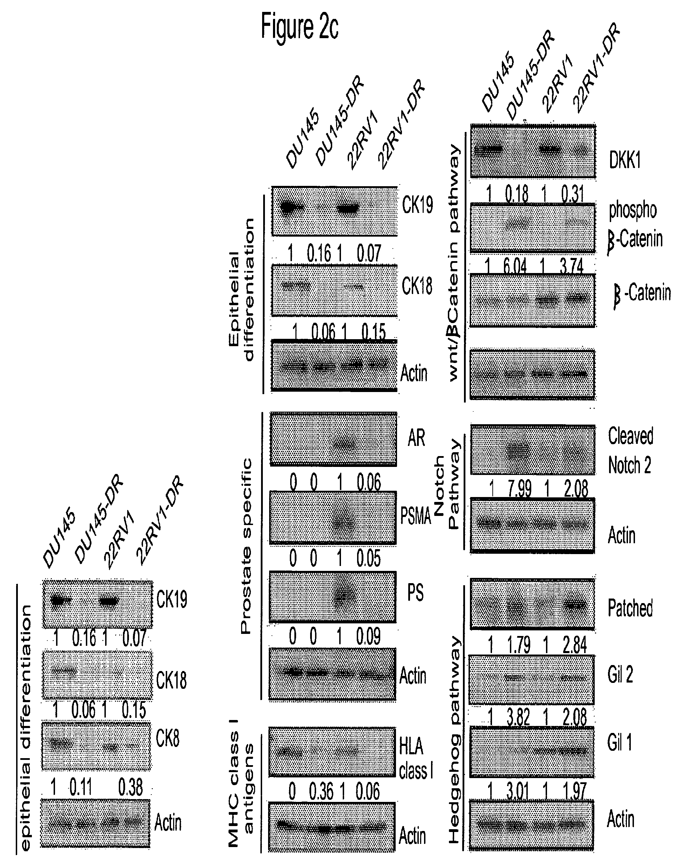

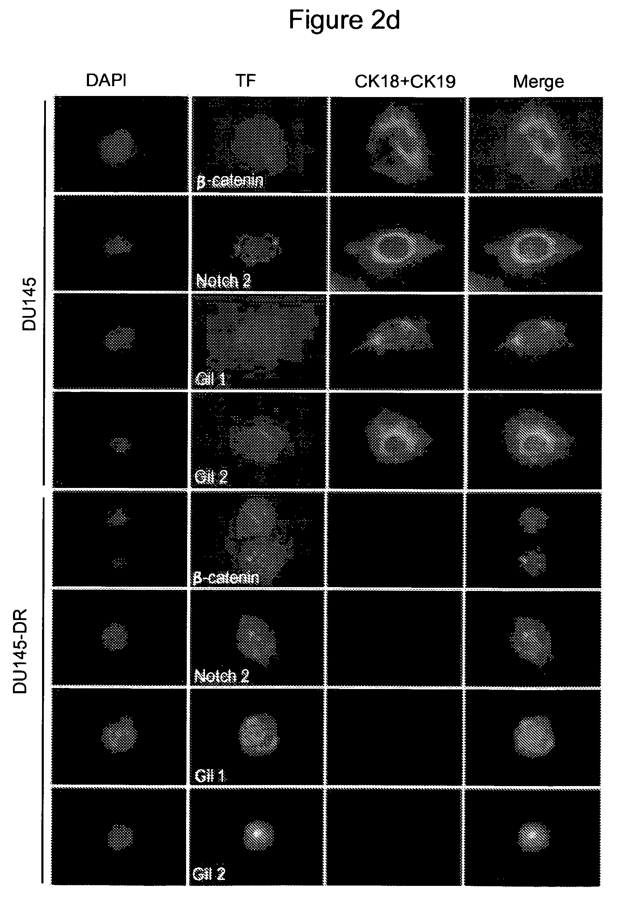

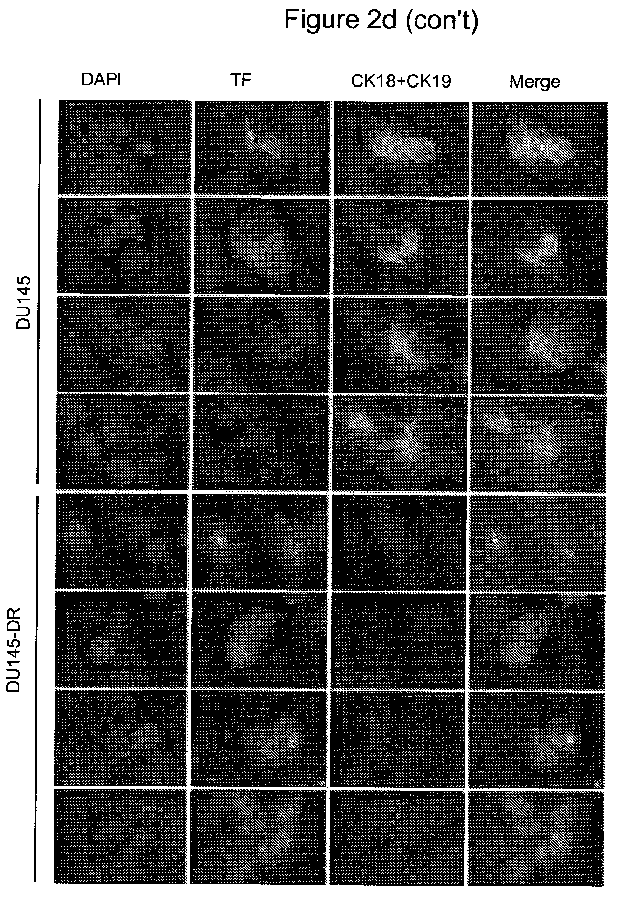

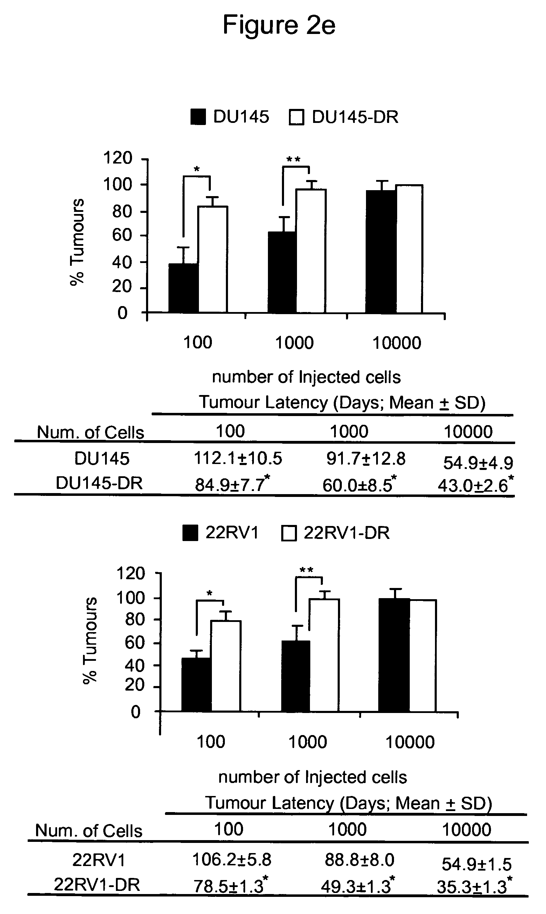

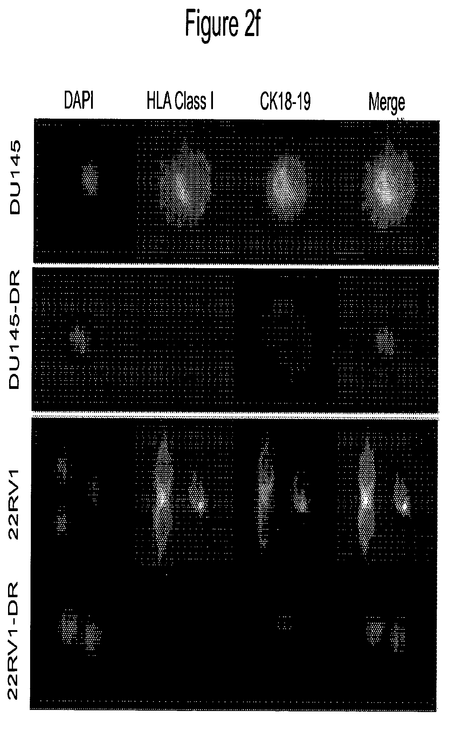

Figure 2 shows phenotypical characterization and tumor initiating capacity of Docetaxel resistant cells. Figure 2a left shows a Venn diagram of genes with at least 2 fold increase (↑) or decrease (↓) in transcript expression in the process of acquiring Docetaxel resistance in DU145 and 22RV1 cells. Overlapping genes = 247. Figure 2a right is a histogram representing the gene ontology (GO) of the 247 overlapping genes. Categories with statistical significance (p≤ 0.01) are represented. GO categories marked with a black asterisk (*) relate to cell proliferation, cell death and response to drugs. GO categories marked with a red asterisk (*) relate to developmental processes. Figure 2b shows the heat map of developmental genes, organized by hierarchical clustering using Cluster and Treeview. Color coding in red indicates high levels of expression and coding in green indicates low levels of expression. The signal values have been log2 transformed. Figure 2c shows immunoblotting and protein quantification of cell lysates of matched parental and Docetaxel resistant cells for epithelial differentiation markers, prostate related markers, MHC class I antigens, WNT/ β-catenin pathway proteins, NOTCH signaling protein and Hedgehog signaling pathway proteins. Figure 2d shows immunofluorescence analyses in DU145 (2d) and 22RV1 (2d con't) parental and Docetaxel resistant cells of the expression and subcellular localization of CKs and HLA class I antigens, as well as various transcription factors (dephosphorylated β-catenin, cleaved NOTCH 2, Gli1 and Gli2). Nuclear localization of developmental transcription factors in Docetaxel resistant cells is seen upon comparison to their parental cells. A lack of expression of CKs is shown. Figure 2e provides histograms showing the tumour initiating capacity and tables summarizing tumor initiating capacity and tumor latencies after injection of parental and Docetaxel resistant cells in NOD/SCID mice. *corresponds to p<0.001 and **corresponds to p <0.05. Figure 2f shows immunofluorescence analyses in DU145 and 22RV1 parental and Docetaxel resistant cells of the expression and subcellular localization of CKs and HLA class I antigens.

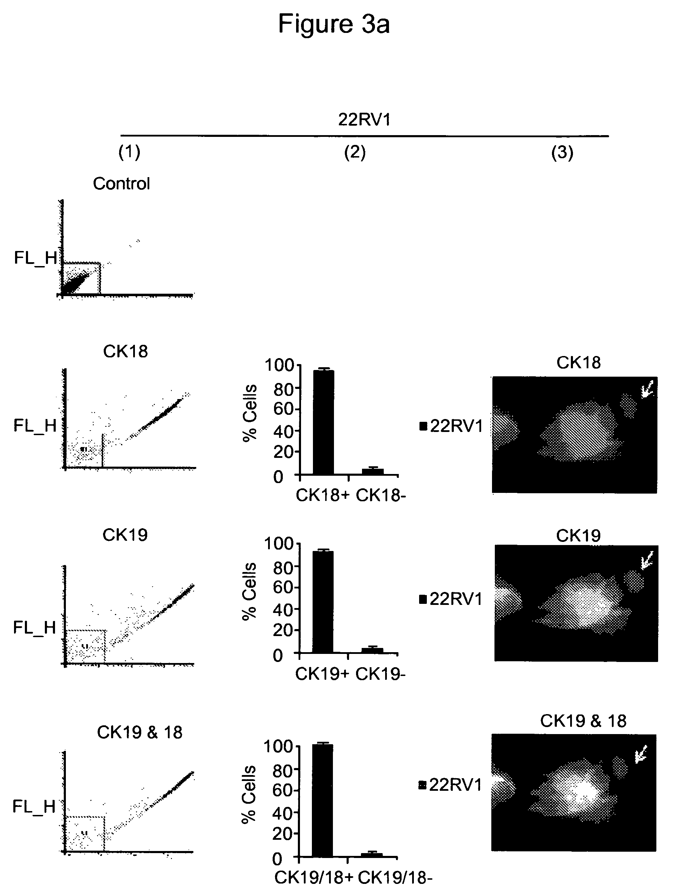

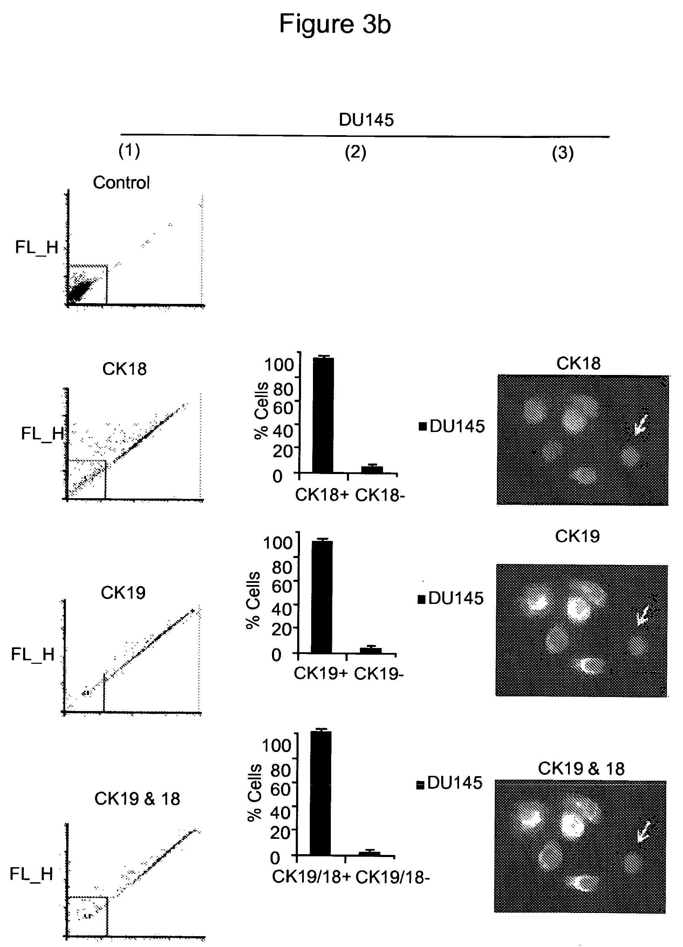

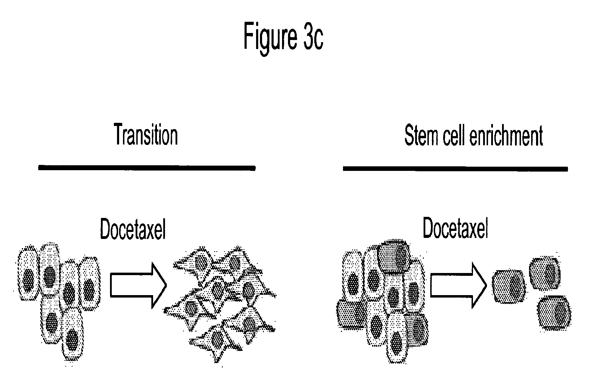

Figure 3 shows identification of a cytokeratin negative subpopulation in parental Docetaxel sensitive cells. Figures 3a and 3b show flow cytometry and immunofluorescence analysis of CK (18 and 19) expression in parental cells (22RVI and DUI45). Plot of flow cytometry analysis shown in column (1) for control (unstained), CK18, CK19 and CK18+19 stained cells are representative of the population. Column (2) shows the quantification of CK- cells in three independent experiments. Column (3) shows immunofluorescence staining for CK 18 (red), CK19 (green) and CK18+19. White arrow indicates a CK negative cell in both parental cell lines. Figure 3c is a schematic representation of the two initial working hypotheses: transition versus cancer stem cell enrichment by Docetaxel.

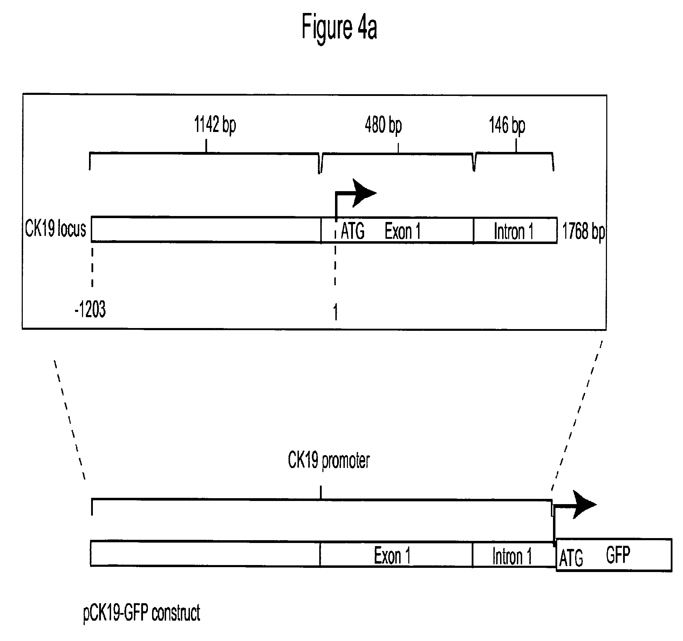

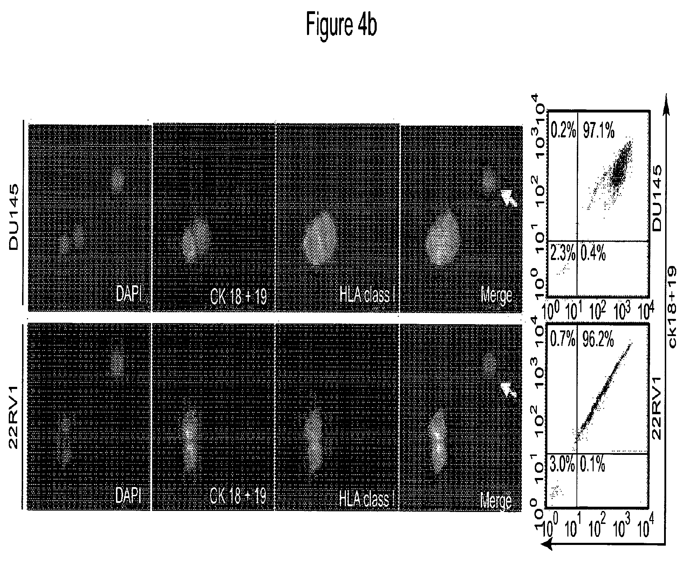

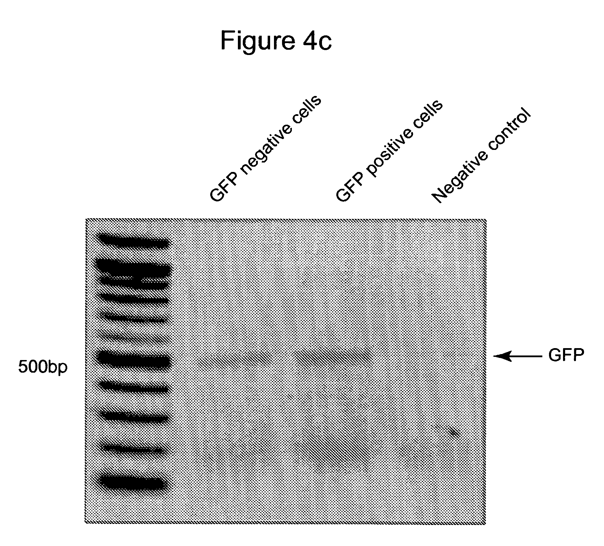

Figure 4 shows generation and validation of the plasmid containing the promoter of CK19 driving GFP expression and that chemotherapy enriches for cancer stem cells with a CK-negative/HLA-negative phenotype. Figure 4a shows a schematic illustration of the generated CK19 promoter-GFP reporter construct. A region of 1768 bp corresponding to the human Cytokeratin 19 promoter was amplified by PCR using genomic DNA from DU145 cells. The promoter region includes 1142 bp of the 5' UTR region, 480 bp belonging to Exon 1 and 146 bp belonging to Intron 1. The PCR product was cloned into pEGFPN1. The resulting construct has the CK19 promoter upstream the GFP protein coding region and regulates its expression. Figure 4b shows immunofluorescence co-staining for CKs (red) and HLA class I (green) of DU145 and 22RV1 parental cells. White arrow in the merge panel points to a cell with a CK-negative/HLA class I-negative phenotype. Representative flow cytometry analysis shows two distinct populations of cells: a major population of CK-positive/HLA-positive cells and a smaller population of cells with a CK-negative/HLA-negative phenotype. Figure 4c shows the results of PCR of sorted GFP+ and GFP- cells stably transfected with the plasmid. PCR confirms the stable integration of the CK19 reporter construct in GFP+ and GFP- DU145 sorted cells. A fragment of the GFP sequence was PCR amplified using genomic DNA as a template from sorted GFP+ and GFP- DU145 cells stably transfected with the reporter plasmid. As a negative PCR control, untransfected parental DU145 cells were used.

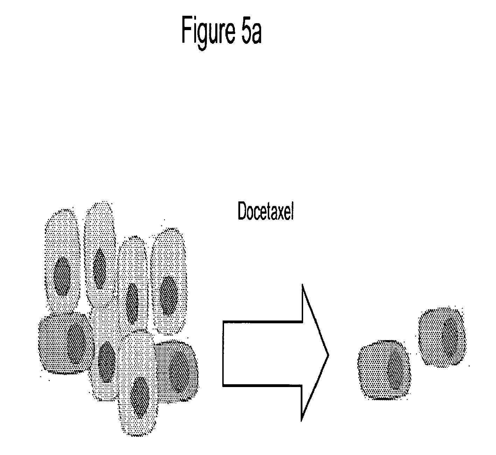

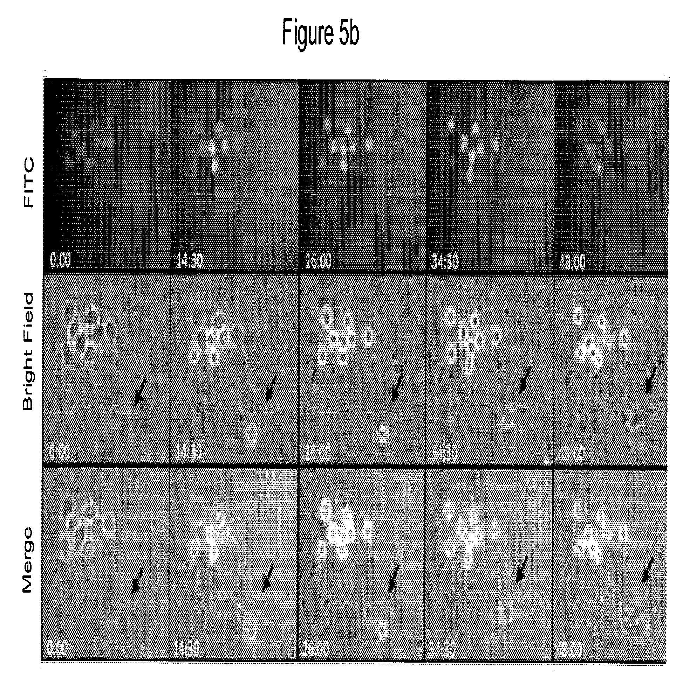

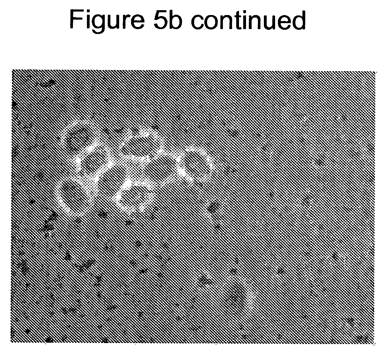

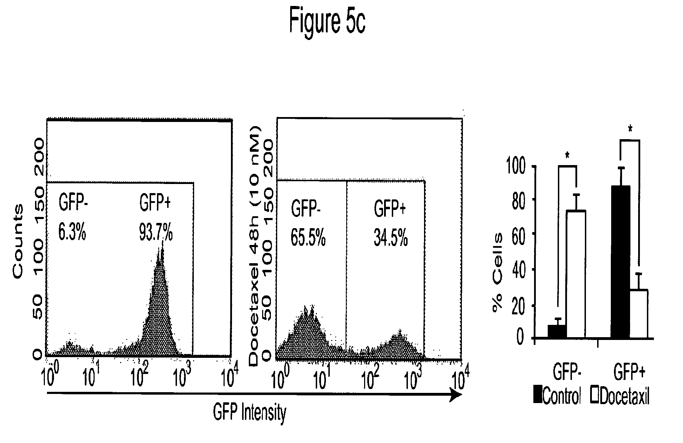

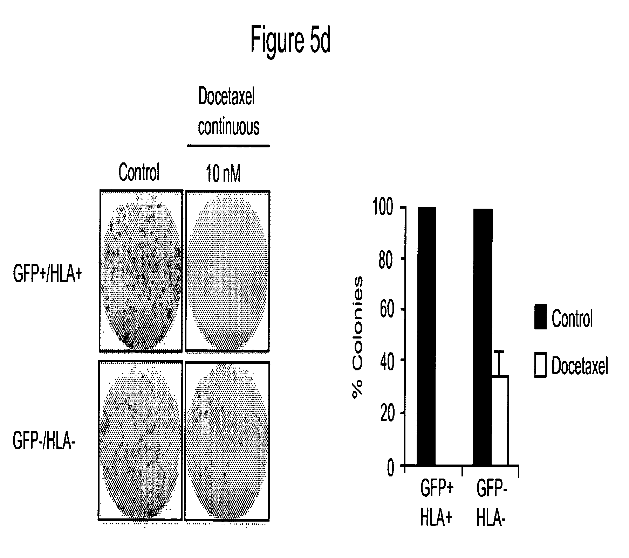

Figure 5 shows that chemotherapy enriches for cancer stem cells with a CK19-negative, GFP negative phenotype. Figure 5a shows a schematic representation of the working hypothesis. Figure 5b shows representative serial imaging of stably transfected cells treated with Docetaxel (10 nM) at various time points as indicated. Unsorted DU145-CK19 promoter-GFP stable cells were treated with Docetaxel (10nM) for 48h and filmed by time-lapse microscopy to study their behavior. Serial images of a representative experiment are included where a black arrow points to a GFP- cell that undergoes cell division and survives under chemotherapy, while GFP expressing cells start dying after mitotic arrest. Figure 5c shows representative flow cytometry analysis and quantification of the percentage of GFP- and GFP+ cells surviving after 48 hours of Docetaxel treatment. Cells from panel (c) were analyzed for GFP expression by flow cytometry after 48 hours of 10nM Docetaxel treatment. Representative plots and quantification of three independent experiments show that there is a shift in the percentage of GFP- and GFP+ populations of cells surviving treatment as compared to untreated cells (control). Figure 5d shows a representative colony formation assay and quantification of sorted GFP-/HLA- and GFP+/HLA+ cells continuously treated with Docetaxel (10 nM). Three independent experiments of GFP/HLA sorted DU145-CK19 promoter-GFP stable cells (GFP+/HLA+ and GFP-/HLA-) continuously cultured with 10nM Docetaxel or in absence of the drug (control) were performed. *corresponds to p<0.001.

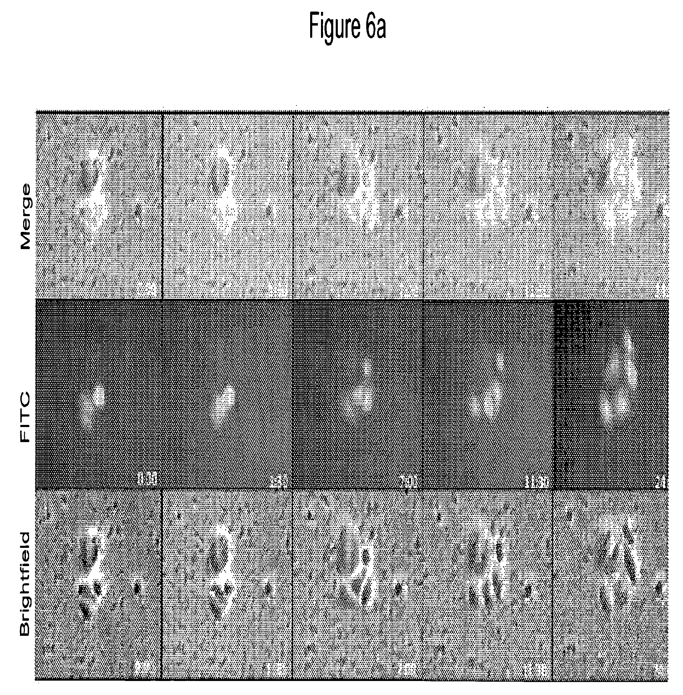

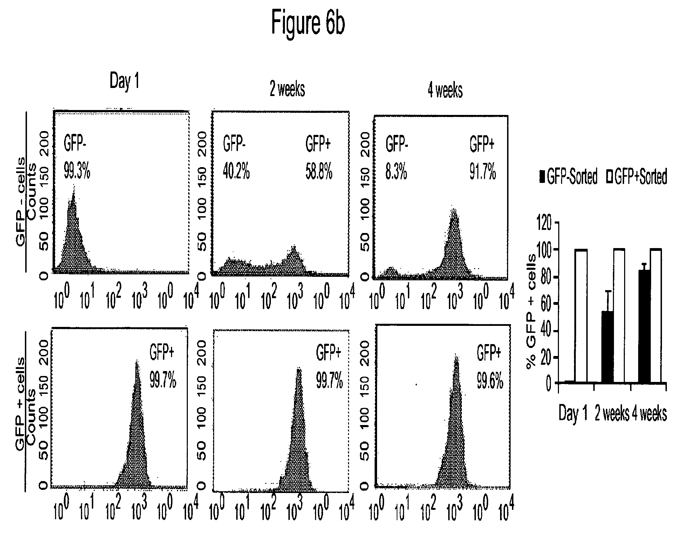

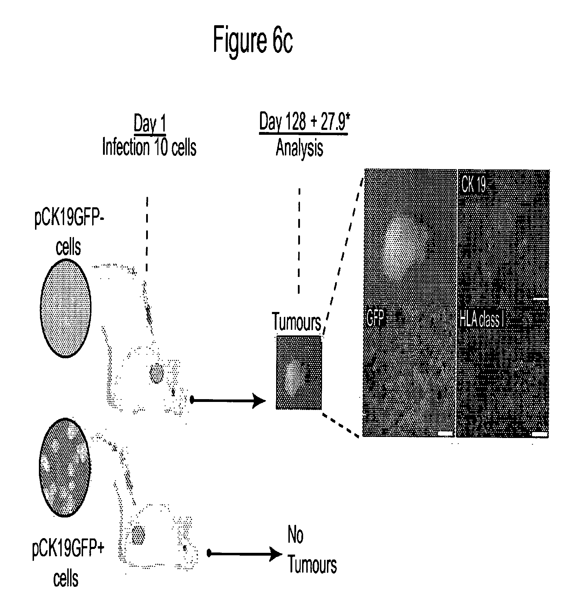

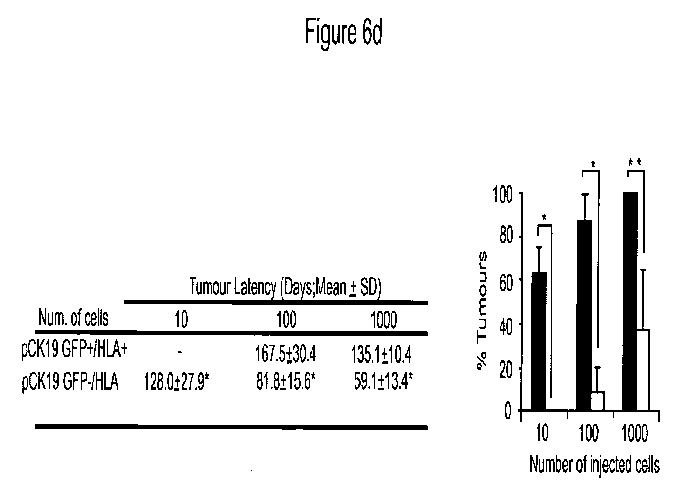

Figure 6 shows the characterization of cancer stem cell features: asymmetrical cell division, tumor initiation and differentiation. Figure 6a shows representative serial imaging of stably transfected cells that depicts a GFP- (CK-negative/HLA class I-negative) cell undergoing an asymmetrical division. Unsorted and untreated DU145-CK19 promoter-GFP stable cells were filmed by time-lapse microscopy during 24 hours. Serial images of a representative experiment show a GFP-(CK-negative) cell dividing asymmetrically and producing a GFP+ daughter cell. Figure 6b shows representative flow cytometry analysis and quantification of GFP populations of cells derived from GFP-negative and GFP-positive sorted cells at different time points. DU145-CK19 promoter-GFP stable cells were GFP sorted and GFP+ and GFP-cells were plated separately and grown for different time periods. Representative flow cytometry analysis plot and quantification of three independent experiments of cultured cells derived from GFP- and GFP+ sorted cells at different time points (Day 1, 2 weeks, 4 weeks). Figure 6c shows a schematic representation of the experimental design and results, as well as immunofluorescence analysis of differentiation markers (GFP, CKs and HLA class I) in a tumor xenograft generated from GFP-negative/CK-negative/HLA class I-negative cells. The schematic representation of the experimental design shows injection of GFP/HLA sorted DU145-CK19 promoter-GFP stable cells into NOD/SCID mice and the resulting tumours. Three independent experiments that included 8 mice for each sorted cell population (e.g., GFP-negative/HLA class I-negative) and cell dilution (10, 100 and 1000 cells) were performed. Representative immunofluorescence analysis of differentiation markers (GFP, CKs and HLA class I) in a xenograft tumour generated from GFP-negative/CK-negative/HLA class I-negative cells is shown. Figure 6d shows tumor latencies and quantitation of tumor initiating capacity after injection of GFP+/HLA+ and GFP-/HLA- sorted cells in NOD/SCID mice. *corresponds to p<0.001 and **corresponds to p <0.05. Bar corresponds to 100µm.

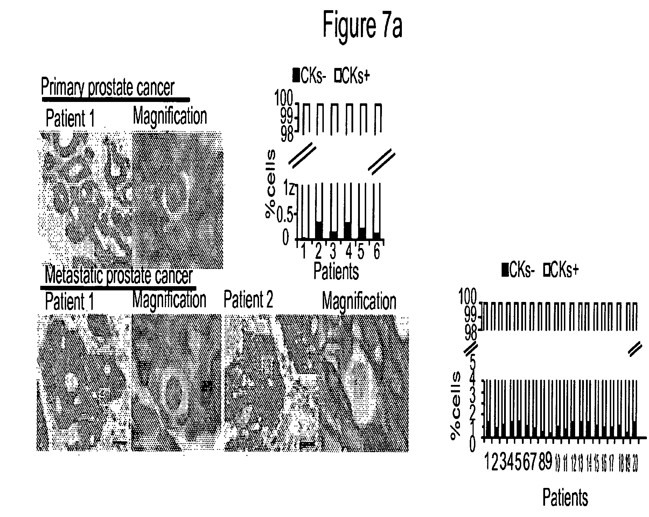

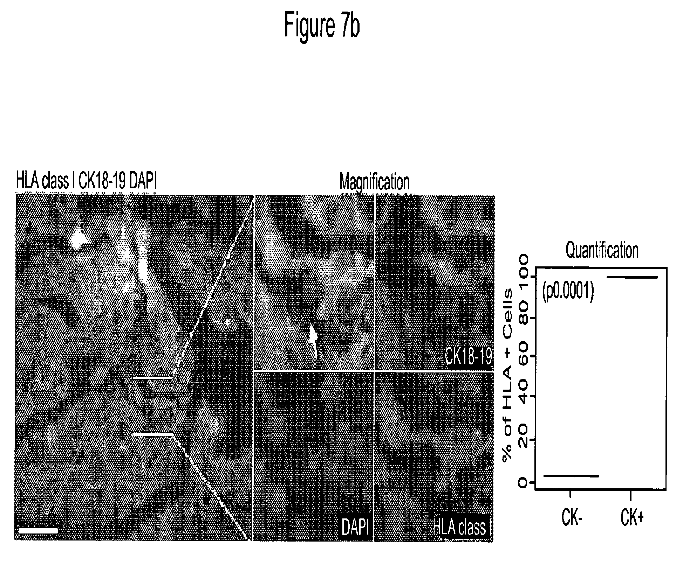

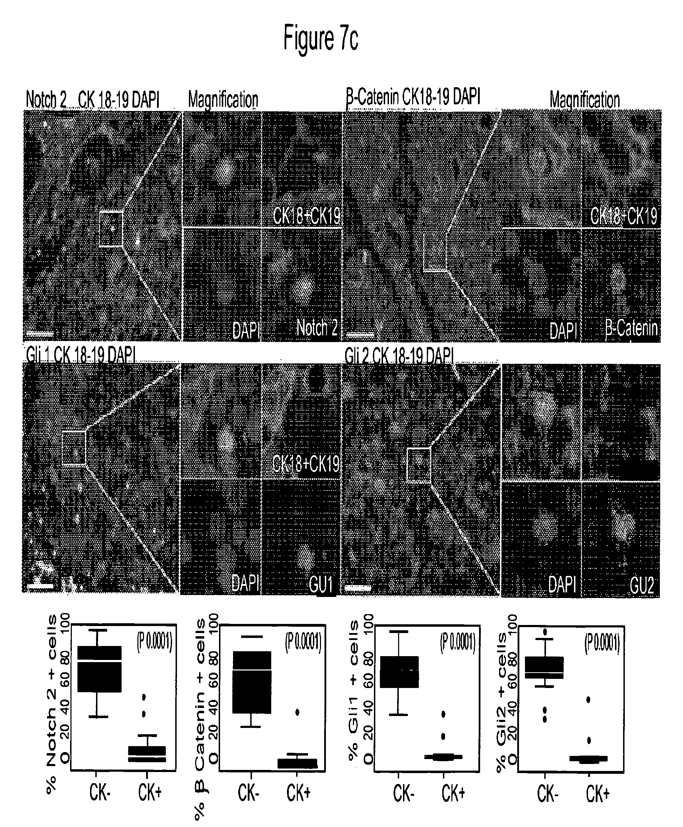

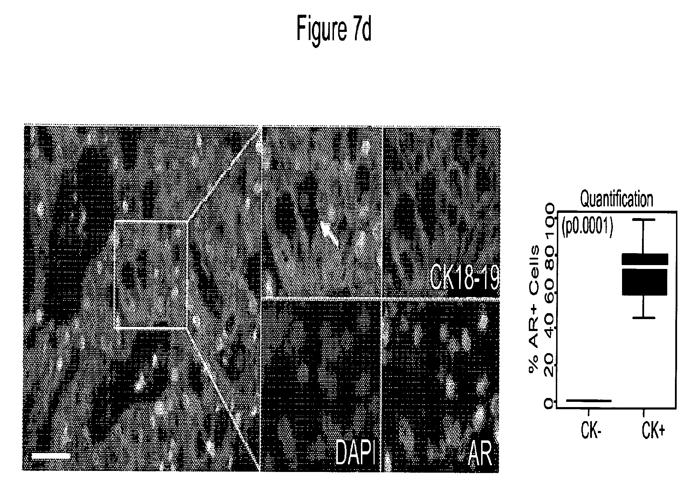

Figure 7 shows the identification of cancer stem cells in human primary and metastatic prostate cancer tissues. Figure 7a shows CK18 and CK19 immunohistochemical expression in representative tissue samples from two patients (Patient 1 with matched primary and metastatic tumors). The histograms show the percentage of CK-negative and positive cells in primary (n=6) and metastatic (n=20) tissue samples from independent patients. Figures 7b-7d show quantification of CK-negative and positive cells in the analyzed tissue samples, as well as representative immunofluorescence based co-expression analysis of CKs (CK18+19) and HLA class I antigens (Figure 7b), transcription factors (cleaved Notch-2, active β-catenin, Gli1 and Gli2) (Figure 7c) and androgen receptor (AR) (Figure 7d). Nuclear staining of transcription factors is shown in the identified CK-negative/HLA-negative tumour cells. Bar corresponds to 100µm.

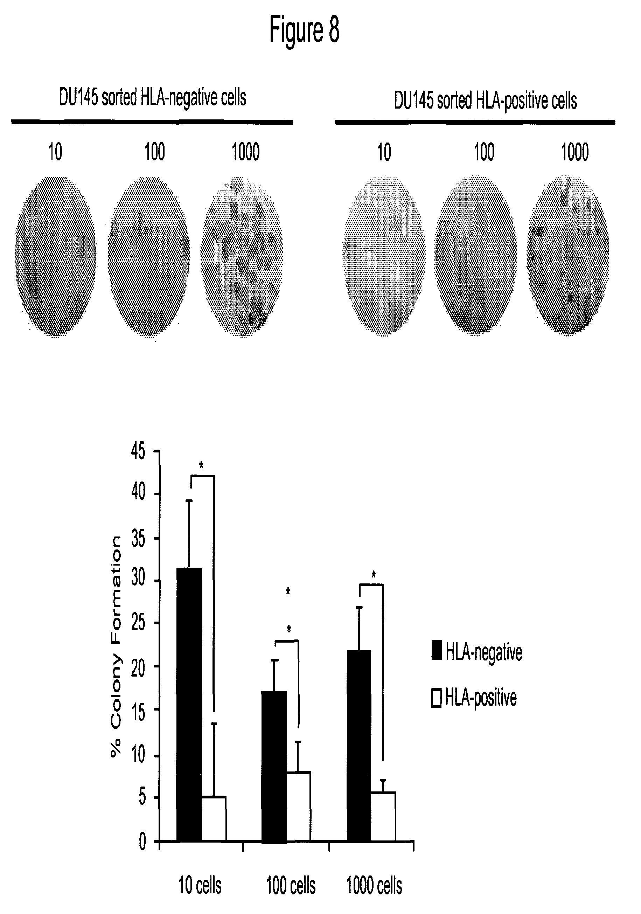

Figure 8 shows the clonability capacity of HLA class I sorted cells. Representative dilution (10, 100 and 1000 cells) colony formation assay of DU145 HLA class I sorted cells and quantification of three independent experiments were performed. HLA class I-negative sorted cells showed a statistically significant higher colony formation when compared to HLA-class I-positive sorted cells. *corresponds to p<0.05.

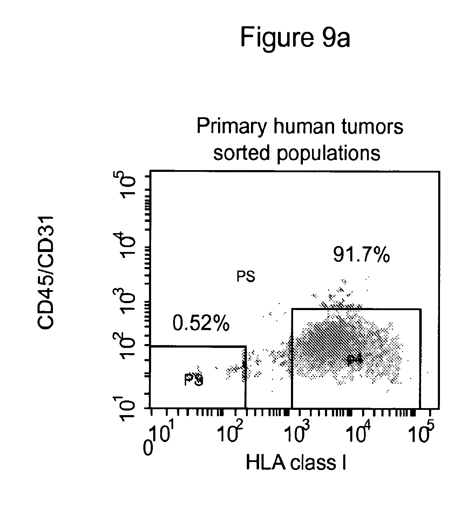

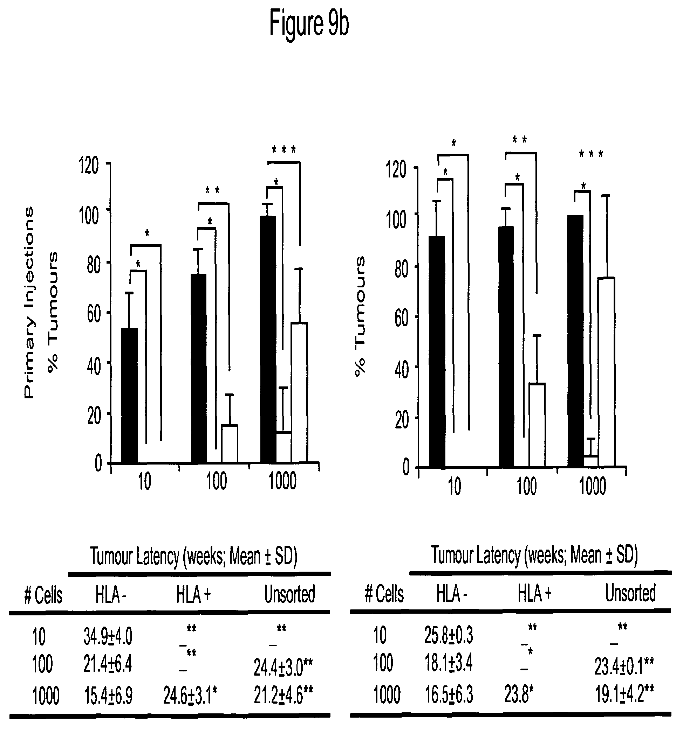

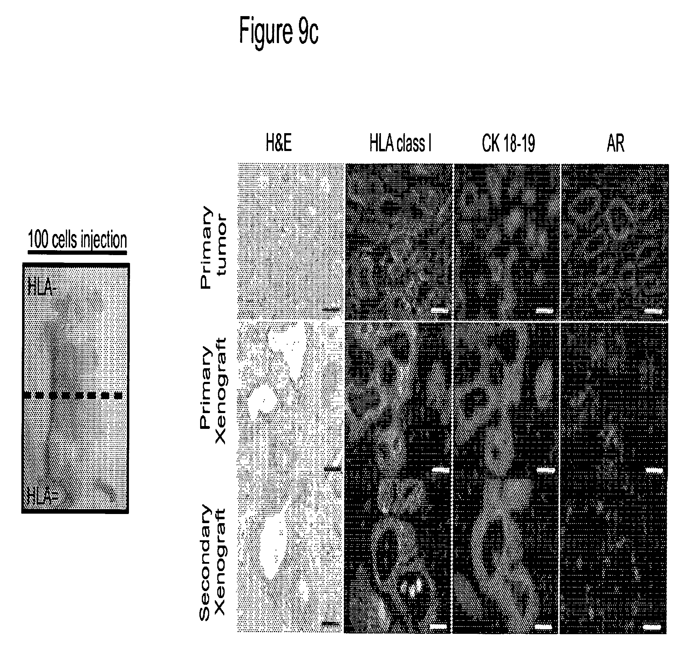

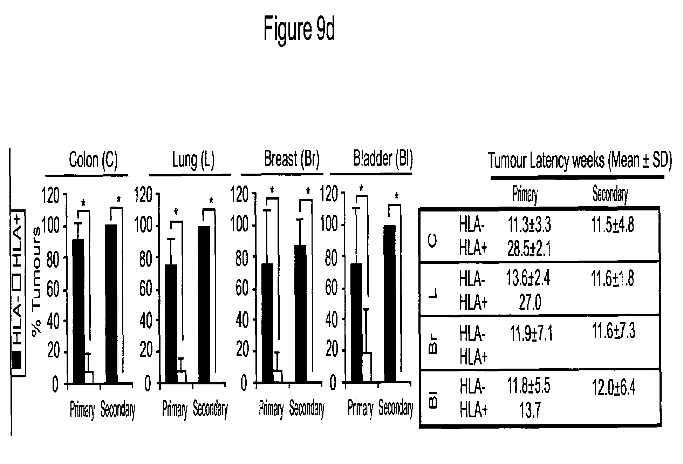

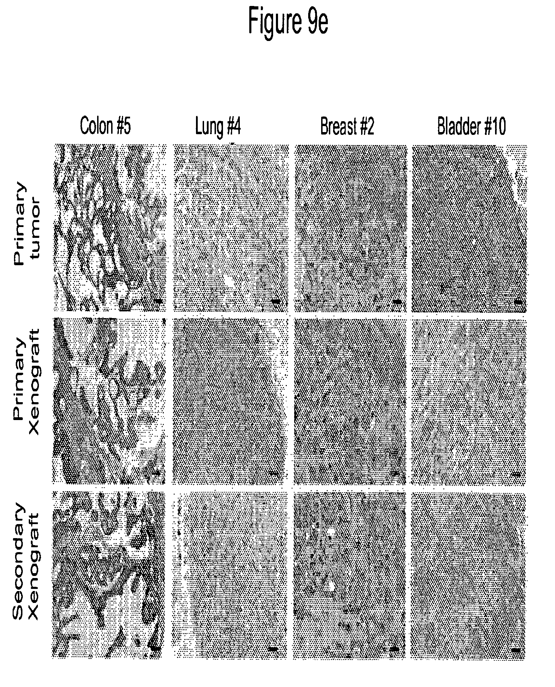

Figure 9 shows that HLA class I-negative epithelial tumor cells from different fresh human cancer types have tumor initiating capacity in NOD/SCID mice. Figure 9a shows a representative sorting diagram of HLA class I-negative and positive tumor cells. Figure 9b shows tumor initiating capacity and tumor latencies after dilution assays of human prostate cancer HLA sorted cells. Graphs and corresponding tables summarize the tumour initiating capacity and tumour latency of different dilutions (10, 100 and 1000 injected cells) of human prostate cancer HLA sorted cells (HLA- and HLA+) and unsorted cells, directly from fresh human samples (primary injections) and derived xenografts (secondary injections) in NOD/SCID mice. Four mice for each sorted cell population and cell dilution were injected twice in the upper flanks (HLA-negative) and lower flanks (HLA-positive). Figure 9c shows representative tumor xenograft formation in a NOD/SCID mouse injected with 10² HLA class I-negative (up) and HLA class I-positive (down) cells. Tumors arising from the injection were confirmed to be prostate cancer by histological (H&E) and immunofluorescence (CKs, androgen receptor (AR), and prostate specific membrane antigen (PSMA)) studies performed in human primary tumour and arising xenograft tumours from primary and secondary injections. Figure 9d shows histograms representing tumor initiating capacity and tumor latencies after dilution assays of other human cancers (Colon, Lung, Breast and Bladder) HLA sorted cells. Primary and secondary injections of 100 HLA sorted cells from the other human cancer types were done. Four mice for each sorted cell population and cell dilution were injected twice in the upper flanks (HLA-negative) and lower flanks (HLA-positive). Figure 9e shows histological (H&E) characterization of human primary and matched derived xenografts. *corresponds to p<0.001, **corresponds to p <0.05 and ***corresponds to p>0.05. Bar corresponds to 100 µm.

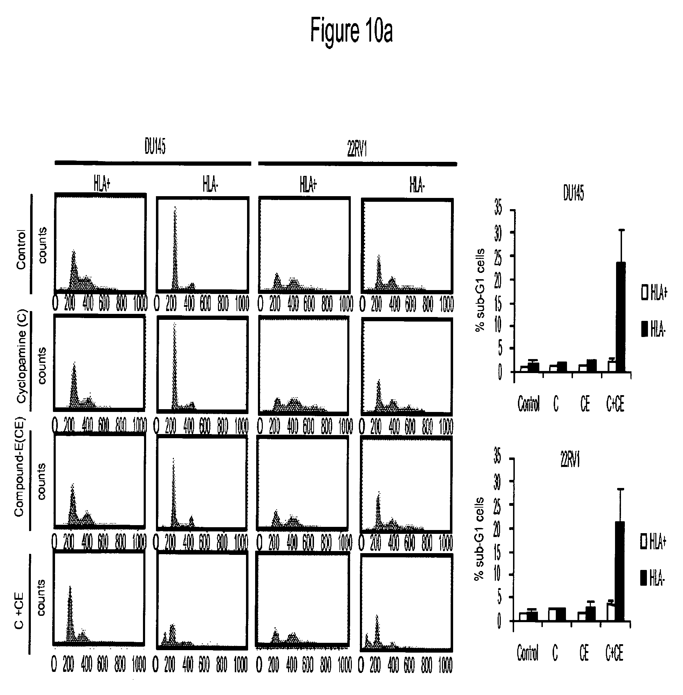

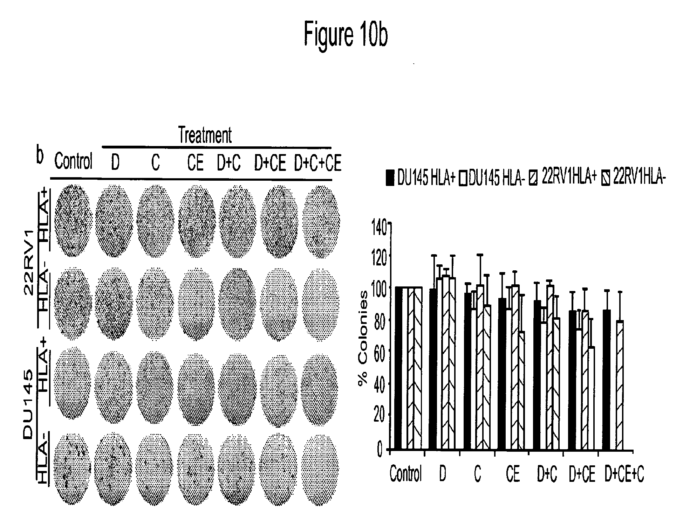

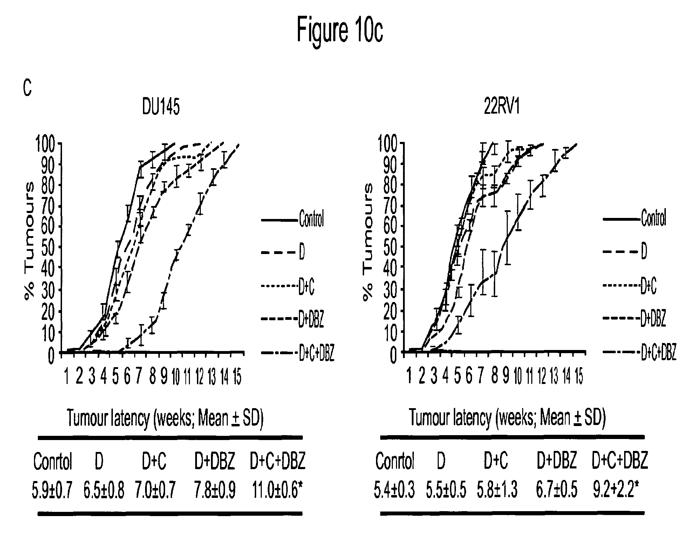

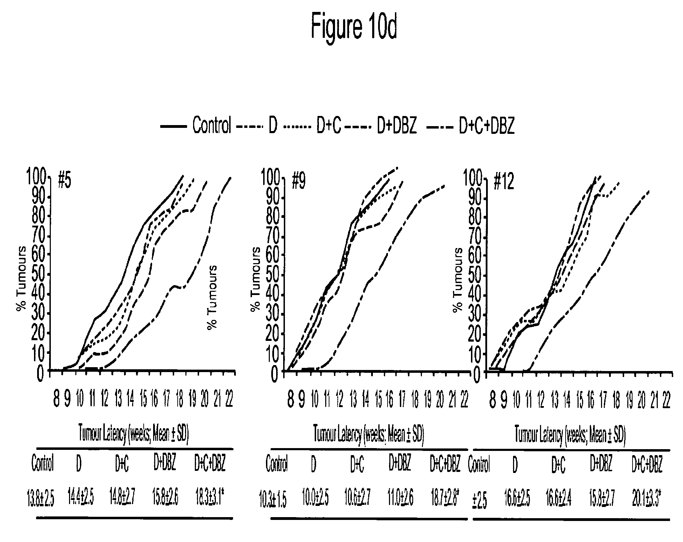

Figure 10 shows *in vitro* and *in vivo* effects of NOTCH and Hedgehog pathway inhibition in the identified cancer stem cells. Figure 10a shows representative cell cycle analysis and quantification of the observed sub-G1 effects in parental (DU145 and 22RV1) HLA-negative and positive sorted tumor cells when exposed during 72 hours to Cyclopamine (C), Compound-E (CE) alone or in combination (C+CE). Figure 10b shows representative colony formation assays and quantification of parental (DU145 and 22RV1) sorted HLA-negative and positive tumor cells when exposed continuously to the Dexamethasone (D) and the combination of the same drugs as in Figure 10a. Figure 10c shows tumor initiating capacity and latencies after injection of 10³ 22RV1 and DU145 HLA-negative sorted cells in NOD/SCID mice exposed to vehicle solution (control), Dexamethasone (D), Cyclopamine plus dexamethasone (D+C), DBZ plus dexamethasone (D+DBZ) or in triple combination (D+C+DBZ). Three independent experiments that included 8 mice for each HLA sorted cell line and treatment (*e.g*., Cyclopamine) were performed. Figure 10d shows tumor initiating capacity and latencies after injection of 10³ HLA-negative sorted cells from human prostate cancer xenografts #5, #9 and #12 in NOD/SCID mice exposed to the same drugs and concentrations as in Figure 10c. The experiment included 8 mice for each prostate cancer case and treatment. *corresponds to p <0.05.

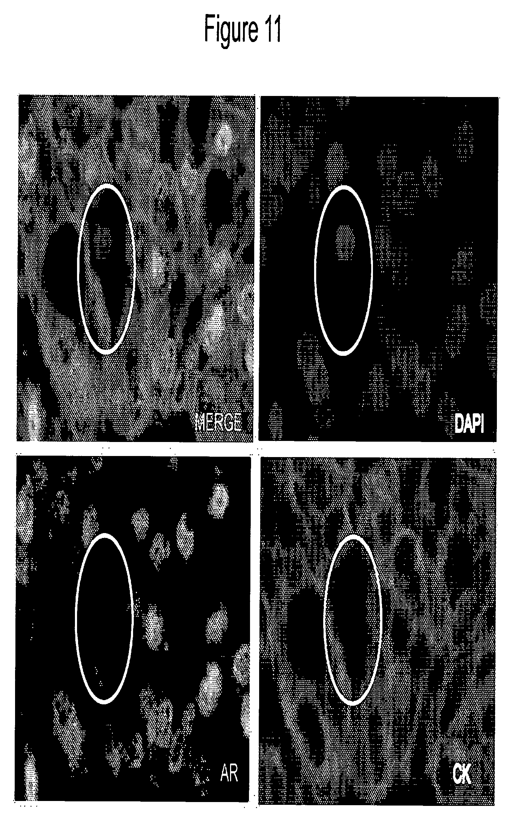

Figure 11 shows that tumor cells that lacked cytokeratins displayed a negative AR phenotype.

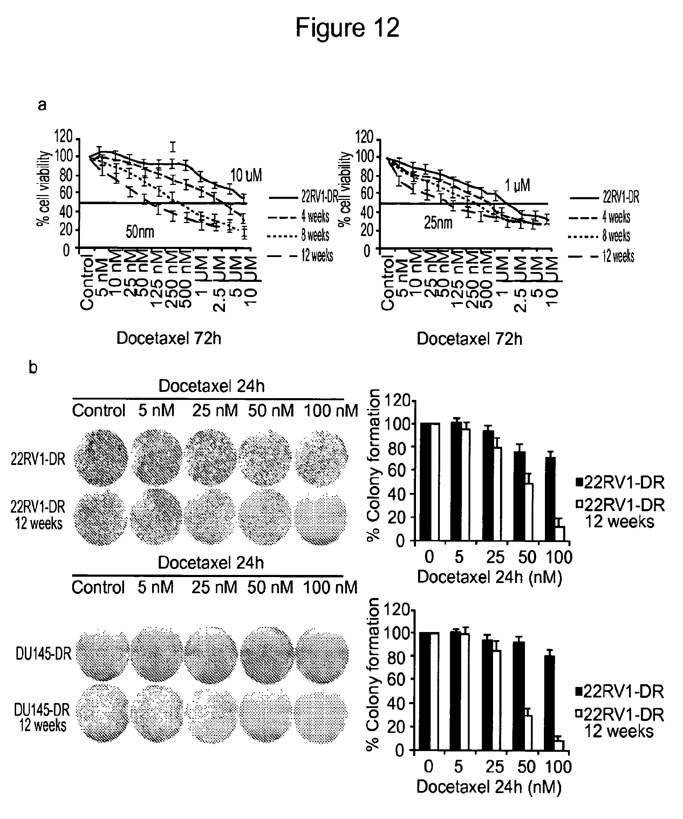

Figure 12 shows the reversibility of acquired Docetaxel resistance in prostate cancer cells. Figure 12a shows quantitation of percent cell viability from cell viability assays (MTs) in Docetaxel resistant cells (22RV1-DR and DU-145-DR) and Docetaxel resistant cells cultured without drug during various time periods (4, 8 and 12 weeks). A red line indicates the IC₅₀ concentration of the drug (Docetaxel) for acquired resistant and reversed resistant cells. Acquired Docetaxel resistant cells cultured without drug become progressively more sensitive to Docetaxel, in a time dependent manner. After 12 weeks of drug withdrawal 22RV1-DR Docetaxel IC₅₀ decreased from 10µM to 50nM and DU145-DR Docetaxel IC₅₀ decreased from 1µM to 25nM. Figure 12b right panel shows quantitative analysis of colony formation assays of Docetaxel resistant cells and Docetaxel resistant cells cultured without drug treated with increasing doses of Docetaxel for 24 hours. The left panel of Figure 12b shows representative colony formation assay of cells treated continuously with Docetaxel. The results confirm the reversibility of acquired Docetaxel resistance because reversed resistant cells form fewer colonies when treated with Docetaxel.

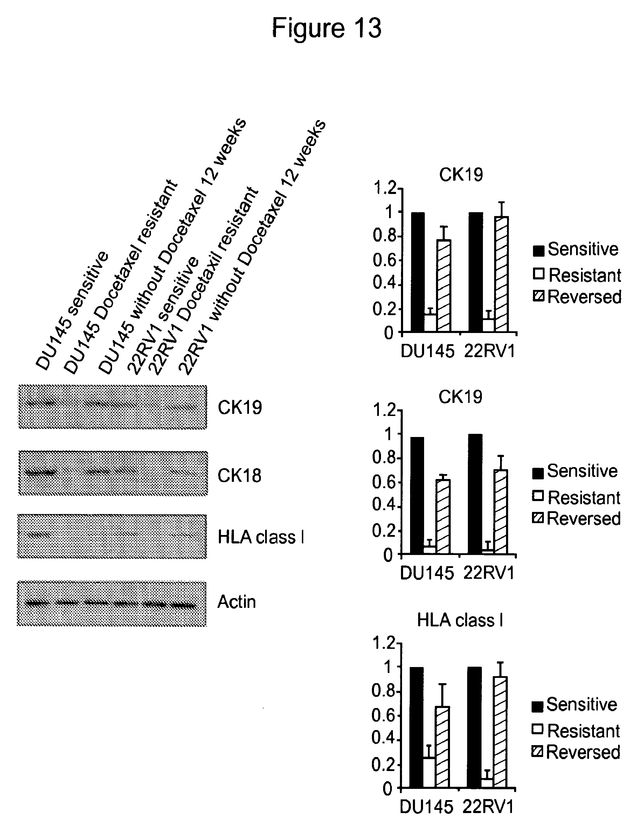

Figure 13 shows Docetaxel resistance reversibility linked to a recovery in the differentiated cell phenotype in DU145 and 22RV1 cells. The left panel of Figure 13 shows western blot analysis and the right panels of Figure 13 show histogram protein quantification of the expression of epithelial differentiation markers (CK18 and CK19) and HLA class I antigens in parental sensitive cells, Docetaxel acquired resistant cells and Docetaxel reversed resistant cells. Reversed resistant cells display higher protein expression levels than Docetaxel acquired resistant cells, achieving similar levels than those observed in parental sensitive cells.

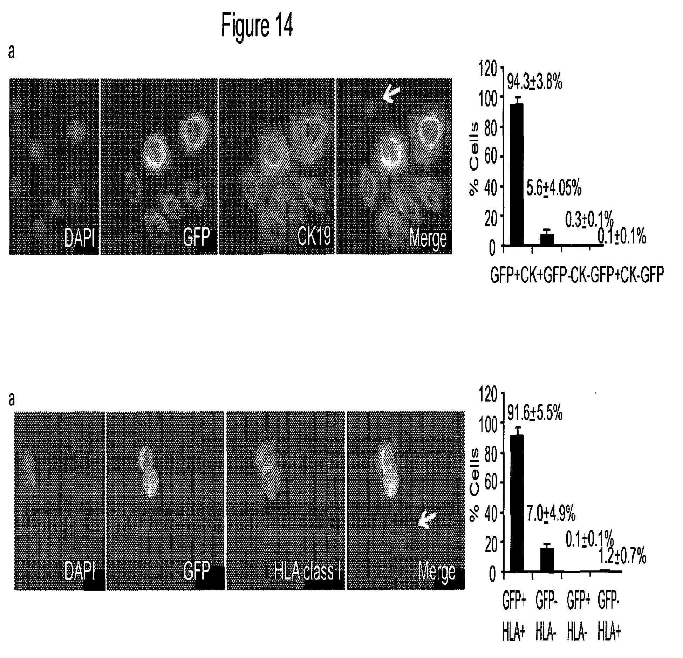

Figure 14 shows generation and validation of the plasmid containing the promoter of CK19 driving GFP expression. Figure 14a shows immunofluorescence staining for CK19 (red) and GFP (green) of DU145 parental cells stably transfected with the pCK19-GFP plasmid. A white arrow in the merge panel points to a cell lacking the expression of CK19 and GFP. Flow cytometry quantification confirms that there is a co-expression of endogenous CK and GFP, thus validating the use of GFP expression as a read out of CK expression in this stable cell line. Figure 14b shows immunofluorescence staining for HLA class I (red) and GFP (green) of DU145 parental cells stably transfected with the pCK19-GFP plasmid. A white arrow in the merge panel points to a cell lacking the expression of HLA class I and GFP. Flow cytometry quantification confirms the co-expression of GFP and HLA-class I antigens. Cells that express GFP are HLA class I-positive, whereas cells that do not express GFP are also HLA class I-negative.

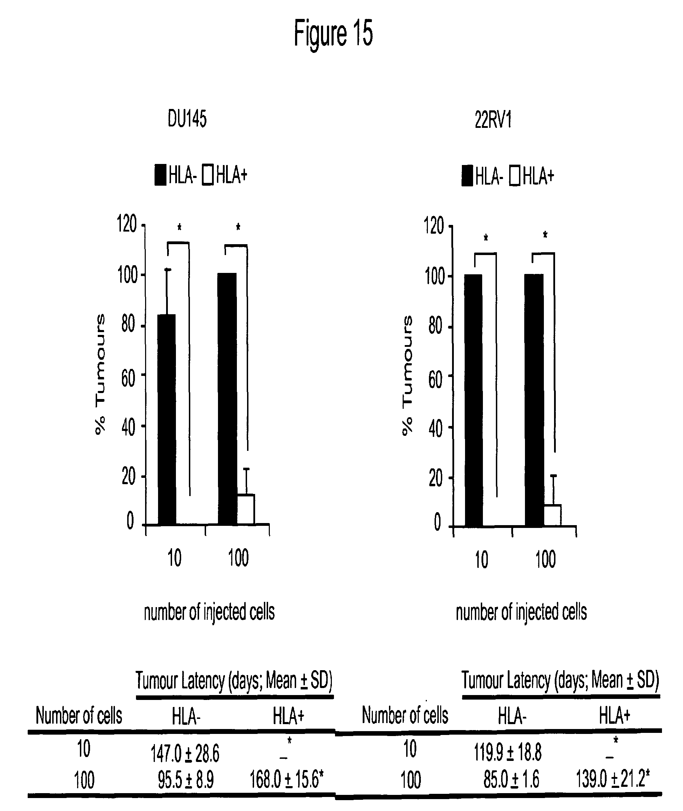

Figure 15 shows tumour initiating capacity of HLA class I sorted cells. Parental DU145 and 22RV1 cells were sorted by HLA marker expression. Two different cell dilutions (10 and 100 cells) of HLA-negative and HLA-positive populations were injected in NOD/SCID mice. Three independent experiments that included 8 mice for each sorted cell line and cell dilution were performed. The graphs of the upper panels and the corresponding tables of the lower panels summarize the tumour initiating capacity and tumour latency, respectively, of these three independent experiments. In both cell lines, only the injection of 10 HLA-negative sorted cells show tumourigenic capacity, whereas 10 HLA-positive cells do not form tumours. *corresponds to p<0.0001.

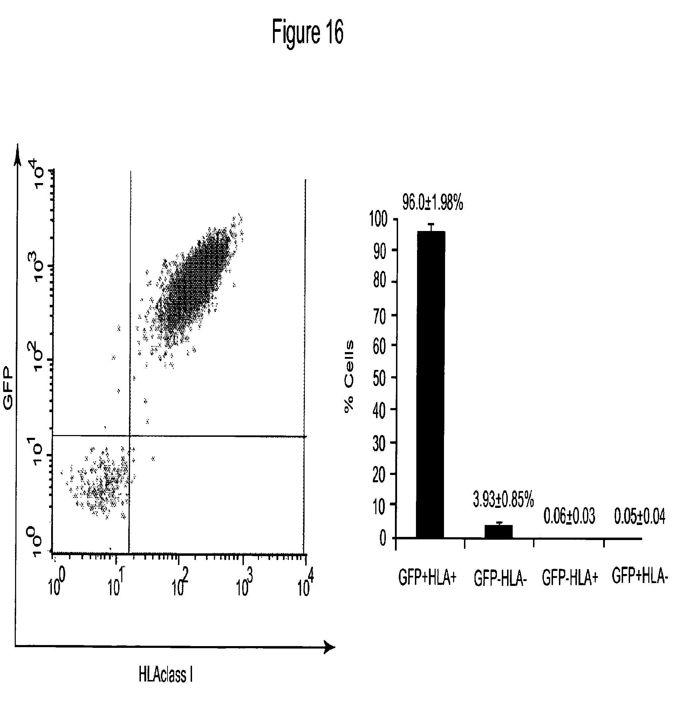

Figure 16 shows quantification of GFP/HLA subpopulations in tumour xenografts generated from injection of GFP-negative/HLA-negative sorted cells. Representative flow cytometry analysis plot and quantification of GFP/HLA subpopulations of cells in tumour xenografts show that two distinct populations of cells are observed: a major population of GFP-positive/HLA-positive cells and a smaller population of cells with a GFP-negative/HLA-negative phenotype.

### DETAILED DESCRIPTION OF THE INVENTION

A CSC that is refractory to standard chemotherapy has been discovered. Although chemotherapy eradicates certain tumor cell subpopulations including dividing cells, it does not deplete the identified CSC population. This provides an explanation as to why standard chemotherapy frequently results in initial tumour shrinkage, yet most cancers eventually recur because of regeneration by surviving CSCs. Identifying an agent that reduces or eradicates the number of CSCs as defined herein would advance the field of cancer therapy.

A method in accordance with the present invention has been developed to identify an agent that selectively decreases the number of cancer stem cells ("CSCs") having the phenotypical characteristics and functional chemoresistance of the CSCs that are identified herein. The *in vitro* screens of the present invention provide an assay for testing candidate agents to identify those that may reduce the survival or growth of the CSC or increase differentiation of the CSC. Implementation of the assays permits testing of a wide variety of candidate agents and facilitates the identification of agents with a putative CSC inhibitory effect.

Accordingly, one embodiment of the present invention is a method for identifying an agent that selectively decreases the number of cancer stem cells (CSCs). This method comprises:
a. contacting a CSC from a population of cells with a candidate agent; and
b. determining whether the candidate agent reduces the survival or growth of the CSC or increases differentiation of the CSC relative to a CSC that has not been contacted with the candidate agent.

A cancer stem cell ("CSC") is a cancer cell that is HLA⁻. Such cells are further defined by being at least one of CD24⁻, CD133⁻, Notch⁺, Gli1⁺, Gli2⁺, glial fibrillary acidic protein⁻ (GFAP⁻), Neurofil⁻, and cytokeratin⁻. Moreover, combinations of any or all of the foregoing are also contemplated. Such CSCs are further defined by having at least one of the following additional properties: capability to self-renew, undergo asymmetrical cell division, have tumorigenic capacity, have metastatic potential, have multi-differentiation properties, are sensitive to Notch and Hedgehog inhibitors, and have broad chemoresistance. Moreover, combinations of any or all of the foregoing are also contemplated. Further properties of CSCs are listed in, *e.g*., Table 1. The combination of any of the identified characteristics in Table 1 together with HLA⁻ may be sufficient to identify a CSC cell.

Table 1 summarizes a number of biomarkers that further define the cancer stem cell phenotype. This phenotype may include expression of developmental pathways and biomarkers of "embryonic stem cells" and "tissue/adult stem cells," such as certain homeobox regulatory factors (*e.g*., Sox2, Sox4), stem cell markers (*e.g.,* ScaI, Gata2, Gata3, Nestin), transcription factors (*e.g*., Notch-2, GliI, Gli2, nuclear beta-catenin), asymmetrical cell division (*e.g*., RMND5A), and membrane transporters associated with multidrug resistance (*e.g*., MDRI/P-glycoprotein, MRPI, MRP2, MRP3, ABC3). Of major relevance, these CSCs have in common the lack of expression of histocompatibility proteins, including all HLA class I and HLA class II molecules, which contributes to the evasion of host immune-surveillance, thus being critical for the metastatic spread.

As used herein, "HLA I⁻" means having no major histocompatibility complex (MHC) class I molecules. The MHC is a set of molecules displayed on cell surfaces that are responsible for lymphocyte recognition and antigen presentation. The MHC class I molecules present antigen to cytotoxic T-cells. MHC I molecules are found on almost all types of body cells.

As used herein, "HLA II⁻" means having no major histocompatibility complex (MHC) class II molecules. The MHC class II molecules present antigen to helper T-cells. MHC II molecules are only found on macrophages, dendritic cells and B cells. "HLA⁻" preferably means HLA I⁻ and HLA II⁻.

Identifying the HLA⁻ cells from a cancer cell population may be accomplished using any conventional or known technique that identifies cells based on, *e.g.,* expression, or non-expression, of particular cell surface markers and maintains the viability of the cell. Preferably, the identification is carried out by a Fluorescence-activated cell sorting (FACS) analysis as disclosed in more detail in the Examples.

As used herein, "capability to self-renew" means, *e.g*., that a CSC has the ability to go through numerous cycles of cell division while maintaining its undifferentiated state.

As used herein, "asymmetrical cell division" means, *e.g.,* capable of having a cell division that leads to two cells with different properties, such as for example one cell which maintains the CSC phenotype while the other is programmed to, *e.g.,* differentiate.

As used herein, "tumorigenic capacity" means, *e.g.,* a cell, such as a CSC, that has the ability to generate a tumor when transplanted into a host animal.

As used herein, "metastatic potential" means, *e.g.,* the ability of a cell, such as a CSC, to move to a secondary location in a body, *i.e.,* metastasize, and generate a tumor.

As used herein, "multidifferentiation properties" means, *e.g.,* the ability of a cell to differentiate into more than one cell type.

As used herein, "sensitivity to Notch and hedgehog inhibitors" means a cell, such as a CSC, which is modulated by inhibitors of the Notch and hedgehog pathways. Such inhibitors are known in the art. See, *e.g.,* K. Garber, JNCI, 99(17):1284-1285 (2007) (Notch inhibitors) and Martinson et al., U.S. Patent No. 7,695,965 (hedgehog inhibitors).

As used herein, "broad chemoresistance" means a cell, such as a CSC that is resistant to a range of chemical agents, such as, *e.g.,* agents used to treat cancers. As used herein, an agent that is used to treat cancer is to be broadly interpreted and may include any known chemotherapy compound, composition or combination thereof. For example, the agent may be a DNA damaging drug and/or an anti-mitotic agent. Further non-limiting examples of the agent include: microtubulin inhibitors, topoisomerase inhibitors, vinblastine, vincristine, vinorelbine, paclitaxel, mitoxantrone, cisplatin, docetaxel, colchicines analogs, harringtonine, homoharringtonine, camptothecine, camptothecine analogs, podophyllotoxin, and combinations thereof.

As used herein, "a population of cells" means a mixture of different cells. For example, the CSCs do not have to be isolated from the rest of the cell types, such as, *e.g.,* a population of cancer cells.

As used herein, "a population of cancer cells" or "a population of cancer/tumor cells" is to be broadly construed to include cancer/tumor cell lines, for example those which are readily available, through, *e.g.,* the ATCC, and may be immortal (*e.g.,* may be propagated indefinitely in vitro) and samples of, *e.g.,* non-immortal cancerous material ("non-immortal samples") obtained from a subject, such as a human, veterinary animal, or research animal such as a mouse, rat, etc. The cancerous material may be cells and/or tissue and/or fluid obtained, for example from a biopsy or other surgical procedure, from, *e.g.,* a solid tumor, a blood-based tumor, or a nervous system tumor. Non-limiting examples of the source of the cancerous material also include blood, plasma, urine, cerebrospinal fluid, ascites fluid, tumor ascites, and combinations thereof.

Thus, a population of cancer cells may be obtained by harvesting cancer cells from a cell culture using well known techniques, including those disclosed in the Examples below. In addition, a population of cancer cells may be obtained through the acquisition of a tumor sample from a cancer patient. The population of cancer cells can be primary cancer cells or metastatic cancer cells. The cancer cells can be derived from a variety of tissue sources, *e.g.,* prostate cancer cells, bladder cancer cells, colon cancer cells, breast cancer cells, lung cancer cells, melanoma, or sarcoma. Further non-limiting examples of other such cancers that are within the scope of the present invention include leukemia, lymphoma, and glioma.

The population of cells in accordance with the invention may also be a mammalian CSC line that is enriched for cells that are HLA I⁻ and HLA II⁻. "Mammalian" refers preferably to human, mouse, or other research mammal, such as *e.g.,* a rat. As used herein, "enriched" means a cancer cell line that has increased numbers of CSCs relative to a control cell line that has not been treated, with *e.g.,* an agent used to treat cancer. The increased numbers of CSCs may be transient, *e.g.,* some of the CSC may differentiate, or may be of a longer duration. Moreover, such a cell line may or may not be pure, e.g., substantially free of HLA⁺ cells.

As noted above, CSCs may have at least one of the following properties: CD24⁻, CD133⁻, Notch⁺, Gli1⁺, Gli2⁺, GFAP⁻, Neurofil⁻, and cytokeratin⁻. Moreover, combinations of any or all of the foregoing are also contemplated. The CSCs may further have at least one of the following properties: capability to self-renew, undergo asymmetrical cell division, have tumorigenic capacity, have metastatic potential, have multi-differentiation properties, sensitive to Notch and Hedgehog inhibitors, and have broad chemoresistance. Moreover, combinations of any or all of the foregoing are also contemplated.

In one aspect of this embodiment, the CSCs may be refractory to standard chemotherapy. As used herein, "refractory" means resistant. For example, a sample of cancer/tumor cells may be derived from a sample of cancerous material of a subject that is resistant to chemotherapy, or the CSCs may be obtained from a cancer cell line that is resistant to chemotherapy. Methods for generating such resistant cancer cell lines are known in the art and disclosed in further detail in the Examples.

In another aspect of this embodiment, a candidate agent is selected from the group consisting of a chemical, a biologic, and combinations thereof. As used herein, a "biologic" means a substance which is derived from or produced by a living organism or synthesized to mimic an *in vivo*-derived agent or a derivative or product thereof. A biologic may be, for example, a nucleic acid, a polypeptide, or a polysaccharide. A "chemical" means a substance that has a definite chemical composition and characteristic properties and that is not a biologic. Non-limiting examples of chemicals include small organic compounds and small inorganic compounds. Non-limiting examples of candidate agents include Hedgehog and Notch inhibitors as disclosed in the Examples herein.

In an additional aspect of this embodiment, the determining step further comprises comparing the percentage of CSCs in a population of cells which is treated with the candidate agent to the percentage of CSCs in a population of cells which is not treated with the candidate agent, wherein a candidate agent that decreases the percentage of CSCs in the population of treated cells as compared to the percentage of CSCs in the untreated cells is an agent that selectively decreases the number of CSCs.

In another aspect of this embodiment, the determining step comprises carrying out a procedure selected from the group consisting of flow cytometry analysis, a cell viability assay, a cell differentiation assay, a cell division assay, and combinations thereof.

As set forth above, flow cytometry, such as FACS, may be used to separate and/or identify CSCs from non-CSC. It may also be used to assess cell viability, differentiation, or division.

As used herein, a "cell viability" assay is an assay that identifies and/or separates live cells from dead cells. A non-limiting example of a cell viability assay is disclosed in the Examples.

As used herein, a "cell differentiation" assay is an assay that identifies and/or separates differentiated cells or non-differentiated cells based on, for example, cell differentiation markers, such as, *e.g.,* cytokeratin expression, as disclosed in the Examples.

As used herein, a "cell division" assay is an assay that identifies and and/or separates dividing cells from the non-dividing cells, for example, by performing a cell-cycle anaysis, as disclosed in the Examples.

In another aspect of the embodiment, the population of cells comprises CSCs and adult stem cells, and the agent decreases the number of CSCs in the population of cells without substantially decreasing the number of adult stem cells. As used herein, "adult stem cells" means undifferentiated cells, found throughout the body after embryonic development, such as the body of an adult human, that multiply by cell division to replenish dying cells and regenerate damaged tissues. The adult stem cells serve as a repair system for the body. Thus, an agent that does not substantially decrease the number of adult stem cells will not substantially affect the body's normal repair system.

The methods of the present invention may be carried out using a high throughput screen. A high throughput screen is preferred to maxize the capacity to screen for potential compounds or biologics that reduce the number of CSCs.

The phrase "high throughput screen" or a "high throughput screening method" as used herein defines a process in which large numbers of agents, *e.g.,* compounds, are tested rapidly and in parallel for biological activity against target molecules or target cells, such as, *e.g.,* a CSC. In certain embodiments, "large numbers of agents, *e.g.,* compounds" may be, for example, more than 100 or more than 300 or more than 500 or more than 1,000 compounds. Preferably, the process is an automated process.

In another aspect of this embodiment, the determining step may further comprise step i and at least one of steps ii, iii, and iv, as follows:
i. comparing the percentage of CSCs in a population of cells, which is treated with the candidate agent to the percentage of CSCs in a population of cells, which is not treated with the candidate agent;
ii. determining the percentage of viable cells in the treated and untreated population of cells;
iii. determining the percentage of non-CSC cells in the treated and untreated population of cells; and
iv. determining the percentage of dividing cells in the treated and untreated population of cells;
wherein a candidate agent that (1) decreases the percentage of CSCs and decreases cell viability; (2) decreases the percentage of CSCs without a decrease in cell viability; (3) decreases the percentage of CSCs and increases the percentage of non-CSCs; (4) decreases the percentage of CSCs and decreases cell division; or (5) decreases the percentage of CSCs without a decrease in cell division is an agent that selectively decreases the number of CSCs.

Preferably, the determining step comprises carrying out a procedure selected from the group consisting of flow cytometry analysis, a cell viability assay, a cell differentiation assay, a cell division assay, and combinations thereof.

Another embodiment of the present invention is a high throughput screening method for identifying agents that selectively decrease the percentage of cancer stem cells (CSCs) in a population of cells. This screening method comprises:
a. contacting a CSC from a population of cells with a candidate agent;
b. determining whether the candidate agent reduces the survival or growth or increases the differentiation of the CSC relative to a CSC that has not been contacted with the candidate agent by:
   i. comparing the percentage of CSCs in a population of cells which is treated with the candidate agent to the percentage of CSCs in a population of cells which is not treated with the candidate agent;
   ii. determining the percentage of viable cells in the treated and untreated population of cells,
   iii. determining the percentage of non-CSC cells in the treated and untreated population of cells; and
   iv. determining the percentage of dividing cells in the treated and untreated population of cells,
wherein a candidate agent that (1) decreases the percentage of CSCs and decreases cell viability; (2) decreases the percentage of CSCs without a decrease in cell viability; (3) decreases the percentage of CSCs and increases the percentage of non-CSCs; (4) decreases the percentage of CSCs and decreases cell division; or (5) decreases the percentage of CSCs without a decrease in cell division is an agent that selectively decreases the percentage of CSCs. The properties of the CSC are as disclosed above.
Step (i) of the present embodiment is preferably carried out by flow cytometry analysis.
Step (ii) of the present embodiment is preferably carried out by a cell viability assay.
Step (iii) of the present embodiment is preferably carried out by a cell differentiation assay.
Step (iv) of the present embodiment is preferably carried out by a cell division assay.

In another aspect of this embodiment, the candidate agent is selected from the group consisting of a chemical, a biologic, and combinations thereof.

In an additional aspect of this embodiment, the CSCs are refractory to standard chemotherapy.

A further embodiment of the invention is a high throughput screening method for identifying agents that selectively decrease the percentage of cancer stem cells (CSCs) in a population of cells, wherein the CSCs are refractory to standard chemotherapy. This method comprises:
a. contacting a CSC from a population of cells with a candidate agent, wherein the CSC has the following properties: HLA I⁻ and HLA II⁻;
b. determining whether the candidate agent reduces the survival or growth or increases differentiation of the CSC relative to a CSC that has not been contacted with the candidate agent by:
   i. comparing, using flow cytometry, the percentage of CSCs in the population of cells, which is treated with the candidate agent to the percentage of CSCs in a population of cells, which is not treated with the candidate agent;
   ii. determining, using a cell viability assay, the percentage of viable cells in the treated and untreated population of cells,
   iii. determining, using a cell differentiation assay, the percentage of non-CSC cells in the treated and untreated population of cells; and
   iv. determining, using a cell division assay, the percentage of dividing cells in the treated and untreated population of cells,
   wherein a candidate agent that (1) decreases the percentage of CSCs and decreases cell viability; (2) decreases the percentage of CSCs without a decrease in cell viability; (3) decreases the percentage of CSCs and increases the percentage of non-CSCs; (4) decreases the percentage of CSCs and decreases cell division; or (5) decreases the percentage of CSCs without a decrease in cell division is an agent that selectively decreases the percentage of CSCs. The properties of the CSC may be further defined, as disclosed above.

In one aspect of this embodiment, the candidate agent is selected from the group consisting of a chemical, a biologic, and combinations thereof.

A further embodiment of the present invention is an agent for selectively killing CSCs identified by the methods disclosed herein.

Another embodiment of the present invention is a pharmaceutical composition comprising a pharmaceutically acceptable carrier and the agent for selectively killing CSCs identified above or a pharmaceutically acceptable salt thereof.

Pharmaceutically acceptable salts as used herein are salts of the agent for selectively killing CSCs with physiologically compatible mineral acids, such as hydrochloric acid, sulphuric acid, sulphurous acid or phosphoric acid; or with organic acids, such as methanesulphonic acid, p-toluenesulphonic acid, acetic acid, lactic acid, trifluoroacetic acid, citric acid, fumaric acid, maleic acid, tartaric acid, succinic acid or salicylic acid. The term "pharmaceutically acceptable salts" refers to such salts. Agents in which an acidic group is present can further form salts with bases. Examples of such salts are alkaline, earth-alkaline and ammonium salts such as e.g., Na--, K--, Ca-- and trimethylammoniumsalt. The term "pharmaceutically acceptable salts" also refers to such salts.

A pharmaceutical composition of the present invention may be administered in any desired and effective manner: for oral ingestion, or as an ointment or drop for local administration to the eyes, or for parenteral or other administration in any appropriate manner such as intraperitoneal, subcutaneous, topical, intradermal, inhalation, intrapulmonary, rectal, vaginal, sublingual, intramuscular, intravenous, intraarterial, intrathecal, or intralymphatic. Further, a pharmaceutical composition of the present invention may be administered in conjunction with other treatments. A pharmaceutical composition of the present invention may be encapsulated or otherwise protected against gastric or other secretions, if desired.

The pharmaceutical compositions of the invention comprise one or more active ingredients, e.g. one or more agents identified by the methods of the present invention, in admixture with one or more pharmaceutically-acceptable carriers and, optionally, one or more other compounds, drugs, ingredients and/or materials. Regardless of the route of administration selected, the agents/compounds of the present invention are formulated into pharmaceutically-acceptable dosage forms by conventional methods known to those of skill in the art. See, e.g., Remington, The Science and Practice of Pharmacy (21^{st} Edition, Lippincott Williams and Wilkins, Philadelphia, PA.).

Pharmaceutically acceptable carriers are well known in the art (see, e.g., Remington, The Science and Practice of Pharmacy (21^{st} Edition, Lippincott Williams and Wilkins, Philadelphia, PA.) and The National Formulary (American Pharmaceutical Association, Washington, D.C.)) and include sugars (e.g., lactose, sucrose, mannitol, and sorbitol), starches, cellulose preparations, calcium phosphates (e.g., dicalcium phosphate, tricalcium phosphate and calcium hydrogen phosphate), sodium citrate, water, aqueous solutions (e.g., saline, sodium chloride injection, Ringer's injection, dextrose injection, dextrose and sodium chloride injection, lactated Ringer's injection), alcohols (e.g., ethyl alcohol, propyl alcohol, and benzyl alcohol), polyols (e.g., glycerol, propylene glycol, and polyethylene glycol), organic esters (e.g., ethyl oleate and tryglycerides), biodegradable polymers (e.g., polylactide-polyglycolide, poly(orthoesters), and poly(anhydrides)), elastomeric matrices, liposomes, microspheres, oils (e.g., corn, germ, olive, castor, sesame, cottonseed, and groundnut), cocoa butter, waxes (e.g., suppository waxes), paraffins, silicones, talc, silicylate, etc. Each pharmaceutically acceptable carrier used in a pharmaceutical composition of the invention must be "acceptable" in the sense of being compatible with the other ingredients of the formulation and not injurious to the subject. Carriers suitable for a selected dosage form and intended route of administration are well known in the art, and acceptable carriers for a chosen dosage form and method of administration can be determined using ordinary skill in the art.

The pharmaceutical compositions of the invention may, optionally, contain additional ingredients and/or materials commonly used in such pharmaceutical compositions. These ingredients and materials are well known in the art and include (1) fillers or extenders, such as starches, lactose, sucrose, glucose, mannitol, and silicic acid; (2) binders, such as carboxymethylcellulose, alginates, gelatin, polyvinyl pyrrolidone, hydroxypropylmethyl cellulose, sucrose and acacia; (3) humectants, such as glycerol; (4) disintegrating agents, such as agar-agar, calcium carbonate, potato or tapioca starch, alginic acid, certain silicates, sodium starch glycolate, cross-linked sodium carboxymethyl cellulose and sodium carbonate; (5) solution retarding agents, such as paraffin; (6) absorption accelerators, such as quaternary ammonium compounds; (7) wetting agents, such as cetyl alcohol and glycerol monostearate; (8) absorbents, such as kaolin and bentonite clay; (9) lubricants, such as talc, calcium stearate, magnesium stearate, solid polyethylene glycols, and sodium lauryl sulfate; (10) suspending agents, such as ethoxylated isostearyl alcohols, polyoxyethylene sorbitol and sorbitan esters, microcrystalline cellulose, aluminum metahydroxide, bentonite, agar-agar and tragacanth; (11) buffering agents; (12) excipients, such as lactose, milk sugars, polyethylene glycols, animal and vegetable fats, oils, waxes, paraffins, cocoa butter, starches, tragacanth, cellulose derivatives, polyethylene glycol, silicones, bentonites, silicic acid, talc, salicylate, zinc oxide, aluminum hydroxide, calcium silicates, and polyamide powder; (13) inert diluents, such as water or other solvents; (14) preservatives; (15) surface-active agents; (16) dispersing agents; (17) control-release or absorption-delaying agents, such as hydroxypropylmethyl cellulose, other polymer matrices, biodegradable polymers, liposomes, microspheres, aluminum monosterate, gelatin, and waxes; (18) opacifying agents; (19) adjuvants; (20) wetting agents; (21) emulsifying and suspending agents; (22), solubilizing agents and emulsifiers, such as ethyl alcohol, isopropyl alcohol, ethyl carbonate, ethyl acetate, benzyl alcohol, benzyl benzoate, propylene glycol, 1,3-butylene glycol, oils (in particular, cottonseed, groundnut, corn, germ, olive, castor and sesame oils), glycerol, tetrahydrofuryl alcohol, polyethylene glycols and fatty acid esters of sorbitan; (23) propellants, such as chlorofluorohydrocarbons and volatile unsubstituted hydrocarbons, such as butane and propane; (24) antioxidants; (25) agents which render the formulation isotonic with the blood of the intended recipient, such as sugars and sodium chloride; (26) thickening agents; (27) coating materials, such as lecithin; and (28) sweetening, flavoring, coloring, perfuming and preservative agents. Each such ingredient or material must be "acceptable" in the sense of being compatible with the other ingredients of the formulation and not injurious to the subject. Ingredients and materials suitable for a selected dosage form and intended route of administration are well known in the art, and acceptable ingredients and materials for a chosen dosage form and method of administration may be determined using ordinary skill in the art.

Pharmaceutical compositions suitable for oral administration may be in the form of capsules, cachets, pills, tablets, powders, granules, a solution or a suspension in an aqueous or non-aqueous liquid, an oil-in-water or water-in-oil liquid emulsion, an elixir or syrup, a pastille, a bolus, an electuary or a paste. These formulations may be prepared by methods known in the art, e.g., by means of conventional pan-coating, mixing, granulation or lyophilization processes.

Solid dosage forms for oral administration (capsules, tablets, pills, dragees, powders, granules and the like) may be prepared, e.g., by mixing the active ingredient(s) with one or more pharmaceutically-acceptable carriers and, optionally, one or more fillers, extenders, binders, humectants, disintegrating agents, solution retarding agents, absorption accelerators, wetting agents, absorbents, lubricants, and/or coloring agents. Solid compositions of a similar type may be employed as fillers in soft and hard-filled gelatin capsules using a suitable excipient. A tablet may be made by compression or molding, optionally with one or more accessory ingredients. Compressed tablets may be prepared using a suitable binder, lubricant, inert diluent, preservative, disintegrant, surface-active or dispersing agent. Molded tablets may be made by molding in a suitable machine. The tablets, and other solid dosage forms, such as dragees, capsules, pills and granules, may optionally be scored or prepared with coatings and shells, such as enteric coatings and other coatings well known in the pharmaceutical-formulating art. They may also be formulated so as to provide slow or controlled release of the active ingredient therein. They may be sterilized by, for example, filtration through a bacteria-retaining filter. These compositions may also optionally contain opacifying agents and may be of a composition such that they release the active ingredient only, or preferentially, in a certain portion of the gastrointestinal tract, optionally, in a delayed manner. The active ingredient can also be in microencapsulated form.

Liquid dosage forms for oral administration include pharmaceutically-acceptable emulsions, microemulsions, solutions, suspensions, syrups and elixirs. The liquid dosage forms may contain suitable inert diluents commonly used in the art. Besides inert diluents, the oral compositions may also include adjuvants, such as wetting agents, emulsifying and suspending agents, sweetening, flavoring, coloring, perfuming and preservative agents. Suspensions may contain suspending agents.

Pharmaceutical compositions for rectal or vaginal administration may be presented as a suppository, which may be prepared by mixing one or more active ingredient(s) with one or more suitable nonirritating carriers which are solid at room temperature, but liquid at body temperature and, therefore, will melt in the rectum or vaginal cavity and release the active compound. Pharmaceutical compositions which are suitable for vaginal administration also include pessaries, tampons, creams, gels, pastes, foams or spray formulations containing such pharmaceutically-acceptable carriers as are known in the art to be appropriate.

Dosage forms for the topical or transdermal administration include powders, sprays, ointments, pastes, creams, lotions, gels, solutions, patches, drops and inhalants. The active agent(s)/compound(s) may be mixed under sterile conditions with a suitable pharmaceutically-acceptable carrier. The ointments, pastes, creams and gels may contain excipients. Powders and sprays may contain excipients and propellants.

Pharmaceutical compositions suitable for parenteral administrations comprise one or more agent(s)/compound(s) in combination with one or more pharmaceutically-acceptable sterile isotonic aqueous or non-aqueous solutions, dispersions, suspensions or emulsions, or sterile powders which may be reconstituted into sterile injectable solutions or dispersions just prior to use, which may contain suitable antioxidants, buffers, solutes which render the formulation isotonic with the blood of the intended recipient, or suspending or thickening agents. Proper fluidity can be maintained, for example, by the use of coating materials, by the maintenance of the required particle size in the case of dispersions, and by the use of surfactants. These compositions may also contain suitable adjuvants, such as wetting agents, emulsifying agents and dispersing agents. It may also be desirable to include isotonic agents. In addition, prolonged absorption of the injectable pharmaceutical form may be brought about by the inclusion of agents which delay absorption.

In some cases, in order to prolong the effect of a drug (e.g., pharmaceutical formulation), it is desirable to slow its absorption from subcutaneous or intramuscular injection. This may be accomplished by the use of a liquid suspension of crystalline or amorphous material having poor water solubility.

The rate of absorption of the active agent/drug then depends upon its rate of dissolution which, in turn, may depend upon crystal size and crystalline form. Alternatively, delayed absorption of a parenterally-administered agent/drug may be accomplished by dissolving or suspending the active agent/drug in an oil vehicle. Injectable depot forms may be made by forming microencapsule matrices of the active ingredient in biodegradable polymers. Depending on the ratio of the active ingredient to polymer, and the nature of the particular polymer employed, the rate of active ingredient release can be controlled. Depot injectable formulations are also prepared by entrapping the drug in liposomes or microemulsions which are compatible with body tissue. The injectable materials can be sterilized for example, by filtration through a bacterial-retaining filter.

The formulations may be presented in unit-dose or multi-dose sealed containers, for example, ampules and vials, and may be stored in a lyophilized condition requiring only the addition of the sterile liquid carrier, for example water for injection, immediately prior to use. Extemporaneous injection solutions and suspensions may be prepared from sterile powders, granules and tablets of the type described above.

### Additional Definitions

The terminology used herein is for the purpose of describing particular embodiments only and is not intended to be limiting. As used in the specification and the appended claims, the singular forms "a," "an," and "the" include plural referents unless the context clearly dictates otherwise.

For recitation of numeric ranges herein, each intervening number there between with the same degree of precision is explicitly contemplated. For example, for the range of 6-9, the numbers 7 and 8 are contemplated in addition to 6 and 9, and for the range 6.0-7.0, the numbers 6.0, 6.1, 6.2, 6.3, 6.4, 6.5, 6.6, 6.7, 6.8, 6,9, and 7.0 are explicitly contemplated.

### Nucleic Acid

"Nucleic acid" or "oligonucleotide" or "polynucleotide" used herein mean at least two nucleotides covalently linked together. The depiction of a single strand also defines the sequence of the complementary strand. Thus, a nucleic acid also encompasses the complementary strand of a depicted single strand. Many variants of a nucleic acid may be used for the same purpose as a given nucleic acid. Thus, a nucleic acid also encompasses substantially identical nucleic acids and complements thereof. A single strand provides a probe that may hybridize to a target sequence under stringent hybridization conditions. Thus, a nucleic acid also encompasses a probe that hybridizes under stringent hybridization conditions.

Nucleic acids may be single stranded or double stranded, or may contain portions of both double stranded and single stranded sequences. The nucleic acid may be DNA, both genomic and cDNA, RNA, or a hybrid, where the nucleic acid may contain combinations of deoxyribo- and ribo-nucleotides, and combinations of bases including uracil, adenine, thymine, cytosine, guanine, inosine, xanthine hypoxanthine, isocytosine and isoguanine. Nucleic acids may be synthesized as a single stranded molecule or expressed in a cell (*in vitro* or *in vivo*) using a synthetic gene. Nucleic acids may be obtained by chemical synthesis methods or by recombinant methods.

The nucleic acid may also be a RNA such as a mRNA, tRNA, shRNA, siRNA, Piwi-interacting RNA, pri-miRNA, pre-miRNA, miRNA, or anti-miRNA, as described, *e.g*., in U.S. Patent Application Nos. 11/429,720, 11/384,049, 11/418,870, and 11/429,720 and Published International Application Nos. WO 2005/116250 and WO 2006/126040.

siRNA gene-targeting may be carried out by transient siRNA transfer into cells, achieved by such classic methods as lipid-mediated transfection (such as encapsulation in liposome, complexing with cationic lipids, cholesterol, and/or condensing polymers, electroporation, or microinjection). siRNA gene-targeting may also be carried out by administration of siRNA conjugated with antibodies or siRNA complexed with a fusion protein comprising a cell-penetrating peptide conjugated to a double-stranded (ds) RNA-binding domain (DRBD) that binds to the siRNA (see, e.g., U.S. Patent Application Publication No. 2009/0093026).

The nucleic acid may also be an aptamer, an intramer, or a spiegelmer. The term "aptamer" refers to a nucleic acid or oligonucleotide molecule that binds to a specific molecular target. Aptamers are derived from an *in vitro* evolutionary process (*e.g*., SELEX (Systematic Evolution of Ligands by EXponential Enrichment), disclosed in U.S. Pat. No. 5,270,163), which selects for target-specific aptamer sequences from large combinatorial libraries. Aptamer compositions may be double-stranded or single-stranded, and may include deoxyribonucleotides, ribonucleotides, nucleotide derivatives, or other nucleotide-like molecules. The nucleotide components of an aptamer may have modified sugar groups (*e.g*., the 2'-OH group of a ribonucleotide may be replaced by 2'-F or 2'-NH₂), which may improve a desired property, *e.g*., resistance to nucleases or longer lifetime in blood. Aptamers may be conjugated to other molecules, *e.g*., a high molecular weight carrier to slow clearance of the aptamer from the circulatory system. Aptamers may be specifically cross-linked to their cognate ligands, *e.g*., by photo-activation of a cross-linker (Brody, E. N. and L. Gold (2000) J. Biotechnol. 74:5-13).

The term "intramer" refers to an aptamer which is expressed *in vivo.* For example, a vaccinia virus-based RNA expression system has been used to express specific RNA aptamers at high levels in the cytoplasm of leukocytes (Blind, M. et al. (1999) Proc. Natl. Acad. Sci. USA 96:3606-3610).

The term "spiegelmer" refers to an aptamer which includes L-DNA, L-RNA, or other left-handed nucleotide derivatives or nucleotide-like molecules. Aptamers containing left-handed nucleotides are resistant to degradation by naturally occurring enzymes, which normally act on substrates containing right-handed nucleotides.

A nucleic acid will generally contain phosphodiester bonds, although nucleic acid analogs may be included that may have at least one different linkage, *e.g.*, phosphoramidate, phosphorothioate, phosphorodithioate, or O-methylphosphoroamidite linkages and peptide nucleic acid backbones and linkages. Other analog nucleic acids include those with positive backbones; non-ionic backbones, and non-ribose backbones, including those disclosed in U.S. Pat. Nos. 5,235,033 and 5,034,506. Nucleic acids containing one or more non-naturally occurring or modified nucleotides are also included within the definition of nucleic acid. The modified nucleotide analog may be located for example at the 5'-end and/or the 3'-end of the nucleic acid molecule. Representative examples of nucleotide analogs may be selected from sugar- or backbone-modified ribonucleotides. It should be noted, however, that also nucleobase-modified ribonucleotides, *i.e.* ribonucleotides, containing a non-naturally occurring nucleobase instead of a naturally occurring nucleobase such as uridines or cytidines modified at the 5-position, *e.g.* 5-(2-amino)propyl uridine, 5-bromo uridine; adenosines and guanosines modified at the 8-position, *e.g.* 8-bromo guanosine; deaza nucleotides, *e.g.* 7-deaza-adenosine; O- and N-alkylated nucleotides, e.g. N6-methyl adenosine are suitable. The 2'-OH-group may be replaced by a group selected from H, OR, R, halo, SH, SR, NH₂, NHR, NR₂ or CN, wherein R is C₁-C₆ alkyl, alkenyl or alkynyl and halo is F, Cl, Br or I. Modified nucleotides also include nucleotides conjugated with cholesterol through, e.g., a hydroxyprolinol linkage as disclosed in Krutzfeldt et al., Nature (Oct. 30, 2005), Soutschek et al., Nature 432:173-178 (2004), and U.S. Patent Application Publication No. 20050107325. Modified nucleotides and nucleic acids may also include locked nucleic acids (LNA), as disclosed in U.S. Patent Application Publication No. 20020115080. Additional modified nucleotides and nucleic acids are disclosed in U.S. Patent Application Publication No. 20050182005. Modifications of the ribose-phosphate backbone may be done for a variety of reasons, *e.g.,* to increase the stability and half-life of such molecules in physiological environments, to enhance diffusion across cell membranes, or as probes on a biochip. Mixtures of naturally occurring nucleic acids and analogs may be made; alternatively, mixtures of different nucleic acid analogs, and mixtures of naturally occurring nucleic acids and analogs may be made.

### Peptide, Polypeptide, Protein

The terms "peptide," "polypeptide," and "protein" are used interchangeably herein. In the present invention, these terms mean a linked sequence of amino acids, which may be natural, synthetic, or a modification, or combination of natural and synthetic. The term includes antibodies, antibody mimetics, domain antibodies, lipocalins, targeted proteases, and polypeptide mimetics. The term also includes vaccines containing a peptide or peptide fragment intended to raise antibodies against the peptide or peptide fragment.

"Antibody" as used herein includes an antibody of classes IgG, IgM, IgA, IgD, or IgE, or fragments or derivatives thereof, including Fab, F(ab')2, Fd, and single chain antibodies, diabodies, bispecific antibodies, and bifunctional antibodies. The antibody may be a monoclonal antibody, polyclonal antibody, affinity purified antibody, or mixtures thereof which exhibits sufficient binding specificity to a desired epitope or a sequence derived therefrom. The antibody may also be a chimeric antibody. The antibody may be derivatized by the attachment of one or more chemical, peptide, or polypeptide moieties known in the art. The antibody may be conjugated with a chemical moiety. The antibody may be a human or humanized antibody. These and other antibodies are disclosed in U.S. Published Patent Application No. 20070065447.

Other antibody-like molecules are also within the scope of the present invention. Such antibody-like molecules include, *e.g.,* receptor traps (such as entanercept), antibody mimetics (such as adnectins, fibronectin based "addressable" therapeutic binding molecules from, *e.g.,* Compound Therapeutics, Inc.), domain antibodies (the smallest functional fragment of a naturally occurring single-domain antibody (such as, *e.g.,* nanobodies; see, *e.g.,* Cortez-Retamozo et al., Cancer Res. 2004 Apr 15;64(8):2853-7)).

Suitable antibody mimetics generally can be used as surrogates for the antibodies and antibody fragments described herein. Such antibody mimetics may be associated with advantageous properties (*e.g.,* they may be water soluble, resistant to proteolysis, and/or be nonimmunogenic). For example, peptides comprising a synthetic beta-loop structure that mimics the second complementarity-determining region (CDR) of monoclonal antibodies have been proposed and generated. See, *e.g.,* Saragovi et al., Science. Aug. 16, 1991;253(5021):792-5. Peptide antibody mimetics also have been generated by use of peptide mapping to determine "active" antigen recognition residues, molecular modeling, and a molecular dynamics trajectory analysis, so as to design a peptide mimic containing antigen contact residues from multiple CDRs. See, *e.g.,* Cassett et al., Biochem Biophys Res Commun. Jul. 18, 2003;307(1):198-205. Additional discussion of related principles, methods, etc., that may be applicable in the context of this invention are provided in, *e.g.,* Fassina, Immunomethods. October 1994;5(2):121-9.

As used herein, "peptide" includes targeted proteases, which are capable of, *e.g.,* substrate-targeted inhibition of post-translational modification such as disclosed in, *e.g.,* U.S. Patent Application Publication No. 20060275823.

In the present invention, "peptide" further includes anticalins. Anticalins can be screened for agents that decrease the number of cancer stem cells. Anticalins are ligand-binding proteins that have been constructed based on a lipocalin scaffold (Weiss, G. A. and H. B. Lowman (2000) Chem. Biol. 7:R177-R184; Skerra, A. (2001) J. Biotechnol. 74:257-275). The protein architecture of lipocalins can include a beta-barrel having eight antiparallel beta-strands, which supports four loops at its open end. These loops form the natural ligand-binding site of the lipocalins, a site which can be re-engineered *in vitro* by amino acid substitutions to impart novel binding specificities. The amino acid substitutions can be made using methods known in the art, and can include conservative substitutions (*e.g*., substitutions that do not alter binding specificity) or substitutions that modestly, moderately, or significantly alter binding specificity.

In general, a polypeptide mimetic ("peptidomimetic") is a molecule that mimics the biological activity of a polypeptide, but that is not peptidic in chemical nature. While, in certain embodiments, a peptidomimetic is a molecule that contains no peptide bonds (that is, amide bonds between amino acids), the term peptidomimetic may include molecules that are not completely peptidic in character, such as pseudopeptides, semi-peptides, and peptoids. Examples of some peptidomimetics by the broader definition (*e.g*., where part of a polypeptide is replaced by a structure lacking peptide bonds) are described below. Whether completely or partially non-peptide in character, peptidomimetics according to this invention may provide a spatial arrangement of reactive chemical moieties that closely resembles the three-dimensional arrangement of active groups in a polypeptide. As a result of this similar active-site geometry, the peptidomimetic may exhibit biological effects that are similar to the biological activity of a polypeptide.

There are several potential advantages for using a mimetic of a given polypeptide rather than the polypeptide itself. For example, polypeptides may exhibit two undesirable attributes, *i.e.,* poor bioavailability and short duration of action. Peptidomimetics are often small enough to be both orally active and to have a long duration of action. There are also problems associated with stability, storage and immunoreactivity for polypeptides that may be reduced with peptidomimetics.

Polypeptides having a desired biological activity can be used in the development of peptidomimetics with similar biological activities. Techniques of developing peptidomimetics from polypeptides are known. Peptide bonds can be replaced by non-peptide bonds that allow the peptidomimetic to adopt a similar structure, and therefore biological activity, to the original polypeptide. Further modifications can also be made by replacing chemical groups of the amino acids with other chemical groups of similar structure, shape or reactivity. The development of peptidomimetics can be aided by determining the tertiary structure of the original polypeptide, either free or bound to a ligand, by NMR spectroscopy, crystallography and/or computer-aided molecular modeling. These techniques aid in the development of novel compositions of higher potency and/or greater bioavailability and/or greater stability than the original polypeptide (Dean (1994), BioEssays, 16: 683-687; Cohen and Shatzmiller (1993), J. Mol. Graph., 11: 166-173; Wiley and Rich (1993), Med. Res. Rev., 13: 327-384; Moore (1994), Trends Pharmacol. Sci., 15: 124-129; Hruby (1993), Biopolymers, 33: 1073-1082; Bugg et a/. (1993), Sci. Am., 269: 92-98.

### Polysaccharides

The term "polysaccharides" means polymeric carbohydrate structures, formed of repeating units (either mono- or di-saccharides) joined together by glycosidic bonds. The units of mono- or di-saccharides may be the same or different. Non-limiting examples of polysaccharides include starch, glycogen, cellulose, and chitin.

### Small organic or inorganic molecules

The phrase "small organic" or "small inorganic" molecule includes any chemical or other moiety, other than polysaccharides, polypeptides, and nucleic acids, that can act to affect biological processes. Small molecules can include any number of therapeutic agents presently known and used, or can be synthesized in a library of such molecules for the purpose of screening for biological function(s). Small molecules are distinguished from macromolecules by size. The small molecules of this invention usually have a molecular weight less than about 5,000 daltons (Da), preferably less than about 2,500 Da, more preferably less than 1,000 Da, most preferably less than about 500 Da.

As used herein, the term "organic compound" refers to any carbon-based compound other than macromolecules such as nucleic acids and polypeptides. In addition to carbon, organic compounds may contain calcium, chlorine, fluorine, copper, hydrogen, iron, potassium, nitrogen, oxygen, sulfur and other elements. An organic compound may be in an aromatic or aliphatic form. Non-limiting examples of organic compounds include acetones, alcohols, anilines, carbohydrates, mono-saccharides, di-saccharides, amino acids, nucleosides, nucleotides, lipids, retinoids, steroids, proteoglycans, ketones, aldehydes, saturated, unsaturated and polyunsaturated fats, oils and waxes, alkenes, esters, ethers, thiols, sulfides, cyclic compounds, heterocyclic compounds, imidizoles, and phenols. An organic compound as used herein also includes nitrated organic compounds and halogenated (*e.g*., chlorinated) organic compounds. Collections of small molecules, and small molecules identified according to the invention are characterized by techniques such as accelerator mass spectrometry (AMS; see Turteltaub et al., Curr Pharm Des 2000 6:991-1007, Bioanalytical applications of accelerator mass spectrometry for pharmaceutical research; and Enjalbal et al., Mass Spectrom Rev 2000 19:139-61, Mass spectrometry in combinatorial chemistry.)

Preferred small molecules are relatively easier and less expensively manufactured, formulated or otherwise prepared. Preferred small molecules are stable under a variety of storage conditions. Preferred small molecules may be placed in tight association with macromolecules to form molecules that are biologically active and that have improved pharmaceutical properties. Improved pharmaceutical properties include changes in circulation time, distribution, metabolism, modification, excretion, secretion, elimination, and stability that are favorable to the desired biological activity. Improved pharmaceutical properties include changes in the toxicological and efficacy characteristics of the chemical entity.

The following examples are provided to further illustrate the methods and compositions of the present invention. These examples are illustrative only and are not intended to limit the scope of the invention in any way.

### EXAMPLE 1

### PROSTATE CANCER CELLS RESISTANT TO DOCETAXEL DISPLAY A DEVELOPMENTAL MOLECULAR SIGNATURE CONSISTENT WITH CELLULAR STEMNESS.

Docetaxel is an anti-mitotic agent currently used as standard therapy in patients with hormone refractory prostate cancer (Petrylak *et al.,* 2004; Tannock *et al.,* 2004. However, all patients ultimately experience disease progression, and no other treatment controls the disease in this context. Due to the clinical relevance of this resistance phenomenon, an *in vitro* model of Docetaxel resistance using the well established prostate cancer hormone-independent cell lines DU145 and 22RV1 was generated to characterize the molecular alterations responsible for such an event. (Figures 1a-d).

DU145 and 22RV1 cells were exposed to increasing doses of Docetaxel, and the acquired chemoresistance was characterized and confirmed by cell viability, colony formation, annexin V, and poly-(ADP-ribose) polymerase (PARP) cleavage assays. (Figures 1a-d). The generated Docetaxel resistant (DR) cells showed cross-resistance to DNA damaging drugs (Mitoxantrone, Doxorubicin and Cisplatin), as well as other anti-mitotic agents (Vinorelbine and Paclitaxel), consistent with a multidrug resistant phenotype (data not shown).

Gene expression profiling, using oligonucleotide microarrays, was performed to compare sensitive (DU145 and 22RV1 - parental cells) and acquired resistant (DU145-DR and 22RV1-DR) prostate cancer cells. Genes with at least 2 fold increase or decrease in transcript expression were selected for further analysis. This analysis disclosed 1245 and 990 deregulated genes in DU145-DR and 22RV1-DR cells, respectively, of which 247 genes overlapped (Figure 2a left). Of these overlapping genes, 29.5% were up-regulated and 70.5% were down-regulated. Surprisingly, gene ontology category assessment of biological processes of the commonly deregulated 247 transcripts revealed that, besides the expected enrichment for cell proliferation, cell death, and drug response biological processes, developmental and immune surveillance categories were significantly represented (Figure 2a right). Genes of relevance in prostate cancer biology and stemness that were found up- or down-regulated included: a) epithelial differentiation biomarkers - cytokeratins (CK18 and 19), and prostate specific markers such as androgen receptor (AR), prostate specific antigen (PSA) and prostate specific membrane antigen (PSMA); b) immune-surveillance antigens - major histocompatibility complex class I (MHC class I); and c) stemness signaling pathways -Wnt/β-catenin, Notch and Hedgehog. Selected genes of relevance are shown in Tables 1 and 2 below. Transcript levels of certain cytostructural genes and signaling pathways, as well as clinicopathological and phenotypical characteristics of 20 metastatic prostate cancer patients' tissue samples are summarized in Table 3 below.

**TABLE 1**

| | Down regulated/ inactive | Up regulated/ active |
|---|---|---|
| Drug resistance | | MDRI (Pgp) |
| | | MRPI |
| | | MRP2 |
| | | MRP3 |
| | | ABC3 |
| Epithelial differentiation CK18 markers | CK8 | |
| | CK19 | |
| | ELF3 (ets domain transcription factor) | |
| | EMPI (epithelial membrane protein 1) | |
| Mesenchymal | Vimentin | |
| differentiation markers | α-Smooth muscle actin | |
| | MCAM (CDI46) | |
| Fibronectin Adhesion Molecules | ADAM8 | |
| | ADAMTS1 | |
| | AGRN (agrin) | |
| | ALCAM (CDI66) | |
| | AMIG02 | |
| | CLDNI (claudin 1) | |
| | CLDN4 (claudin 4) | |
| | CLDN7 (claudin 7) | |
| | CLDN10 (claudin 10) | |
| | CLDN11 (claudin 11) | |
| | CDH1 (e-cadherin) | |
| | CDH2 (n-cadherin) | |
| | CDH3 (p-cadherin) | |
| | CDH7 (cadherin 7) | |
| | CDCP1 (CUB domain containing protein 1) | |
| | ICAM1 | |
| | ITGA3 (integrin alpha 3) | |
| | ITGA5 (integrin alpha 5) | |
| | ITGA6 (integrin alpha 6) | |
| | JUP Uunction plakoglobin) | |
| | LAMA3 (laminin alpha 3) | |
| | LAMB3 (laminin beta 3) | |
| | LAMC2 (laminin C 2) | |
| | SDC1 (syndecan1) | |
| | SDC2 (syndecan2) | |
| | ZYX (zyxin) | |
| Developmental | AXIN1 (negative regulator WNT) | ABLIM3 |
| | | NOTCH2 |
| | DKK1 (negative regulator WNT) | HES1 |
| | | HEY1 |
| | WIFI (negative regulator WNT) | PTCH1 |
| | | GLI1 |
| | GPRC5A | GLI2 |
| | GPRC5C | BMP1 (Bone morphogenic protein 1) |
| | | GATA2 |
| | | NES (nestin) |
| | | NKX3.1 |
| Immunosurveillance | HLA-A | |
| | HLA-B | |
| | HLA-C | |
| | HLA-E | |
| | HLA-F | |
| | HLA-G | |
| | HLA-DR | |
| | HLA-DQ | |
| | HLA-DP | |
| | CD59 | |
| | CD74 | |
| | MR1 (major histocompatibility complex class I) | |
| | SECTM1 | |
| Cluster differentiation Markers | CD133 | |
| | CD24 | |
| | CD63 | |
| Signal transduction | AKAP1 (PKA& PKC) | ARHGEF10 (RhoPTTase) |
| | AKAP12 (PKA & PKC) | MZF1 |
| | ANXA3 (Inositol phosphate) | |
| | CALM1 (calmodulin) | |
| | CDS2 (phosphatidyl inositol) | |
| | EFNB2 (ephrin-B2) | |
| | EGFR | |
| | EHD1 (EGFR substrate) | |
| | ETS1 | |
| | ETS2 | |
| | FOSL1 | |
| | HGF (hepatic growth factor) | |
| | HSP90 (Heat shock protein 90) | |
| | HSP70 (Heat shock protein 70) | |
| | IFI16 (interferon, gamma inducible protein) | |
| | IFI35 (interferon, gamma inducible protein) | |
| | IFITM1 (IF inducible transmembrane protein) | |
| | IFITM2 (IF inducible transmembrane protein) | |
| | IFITM3 (IF inducible transmembrane protein) | |
| | IFNAR1 (intereferon receptor 1) | |
| | IFNAR2 (intereferon receptor 2) | |
| | IL6 (interleukin6) | |
| | IL6R (interleukin6 receptor) | |
| | IL8 (interleukin8) | |
| | LIMK1 (LIM domain kinase 1) | |
| | MET (hepatocyte growth factor receptor) | |
| | NMB (neuromedin) | |
| | STAT3 | |
| Cell Cycle | AURKA (Aurora Kinase A) | MCM5 |
| | G0S2 (G0G1 switch gene) | NSL1 (kinetochore component) |
| | KTN1 (kinesin receptor 1, MAP) | |
| | | RMND5A |
| Angiogenesis | OS9 | BAI2 (angiogenesis inhibitor) |
| | VEGFA | |
| | VEGFB | BAI3 (angiogenesis inhibitor) |
| | VEGFC | |
| | | BAIAP2 (angiogenesis inhibitor) |
| Metabolism | ALDH2 | AKR1C1 |
| | ARSJ (Sulfatase) | AKR1C2 |
| | B4GALTI (Galactose) | AKR1C3 |
| | GALC (galactosylceramide) | ALDH3A2 |
| | | ALDH3B1 |
| | | GCLC (glutamatecisteinligase) |
| Oxidative stress | | OGGI |
| | | OSGIN1 |
| | | OSGIN2 |
| Others | ADCK2 (aarf domain kinase 2) | ADNP homeobox 2 |
| | APP (Amyloid B precursor protein) | |
| | BACE2 (Amyloid B precursor cleaving enzyme) | |
| | CARS (cysteinyl-tRNA synthetase) | |
| | CREG1 (Inhibitor of EIA) | |
| | GSN (gelsolin, amyloid) | |

**TABLE 2**

| Gene name | x-fold change DU145/DR; 22RV1/DR | p value (t-test) |
|---|---|---|
| Keratin 19 | -29.8;-21.5 | <0.0001;<0.0001 |
| Keratin 18 | -2.7;-2.1 | <0.05;<0.05 |
| Androgen receptor | NE;-2.2 | -;<0.05 |
| Folate hydrolase (prostate-specific membrane antigen) 1 | NE;-5.4 | -;<0.0001 |
| kallikrein-related peptidase 3 | NE;-4.3 | -;<0.0001 |
| Major histocompatibility complex, class I, A | -2,1;-5.4 | <0.05;<0.0001 |
| Major histocompatibility complex, class I, B | -2.0;-3.9 | <0.05;<0.05 |
| Major histocompatibility complex, class I, C | -2.3;-4.4 | <0.05;<0.0001 |
| Major histocompatibility complex, class I, E | -2.1;-2.6 | <0.05;<0.05 |
| Major histocompatibility complex, class I, F | -2.4;-1.8 | <0.05;<NS |
| Major histocompatibility complex, class I, G | -2.1;-1.7 | <0.05;<NS |
| Dickkopf homolog 1 (Xenopus laevis) | -3.8;-3.2 | <0.05;<0.05 |
| Notch homolog 2 (Drosophila) | +4.2;+2.1 | <0.0001;<0.05 |
| Patched homolog 1 (Drosophila) | +3.2;+3.3 | <0.05;<0.05 |
| GLI family zinc finger 1 | +2.2;+2.3 | <0.05;<0.05 |
| GLI family zinc finger 2 | +2.1;+2.0 | <0.05;<0.05 |

| | | |
|---|---|---|
| Note: NE= Not expressed. NS=Not Significant. | | |

Genes that were found up- or down-regulated in Docetaxel resistant cells (DU145-DR and 22RV1-DR) when compared to their parental sensitive cells DU145 and 22RV1) are summarized in Table 2. Statistical analysis of the mean expression average difference of genes, which show ≥ 2 fold change based on a logarithmic normalization, was done using a t-test between matched sensitive and resistant cells. There was a decrease in the transcription levels of genes involved in epithelial differentiation, prostate specific markers and immune-surveillance markers. On the other hand, gene transcripts of developmental transcription factors were up-regulated.

**TABLE 3**

| **Patient** | **Metastatic site** | **Hormone status** | **CK18+19** | **% active β-catenin** | **% cleaved NOTCH-2** | **% Gli-1** | **% Gli-2** |
|---|---|---|---|---|---|---|---|
| | | | | | | | |
| 1 | Peritoneum | Independent | - | 66.6 | 77.7 | 87 | 92 |
| | | | + | 2 | 4 | 1.3 | 2 |
| 2 | Bone | Independent | - | 33.3 | 33.3 | 73 | 77 |
| | | | + | 0 | 0 | 0.5 | 0.5 |
| 3 | Brain | Dependent | | | 94 | 60 | 65 |
| | | | + | 4.5 | 36 | 1.2 | 2.1 |
| 4 | Lung | Independent | | 75 | 93 | 78 | 75 |
| | | | + | 2 | 3.5 | 1.2 | 1 |
| 5 | Bone | | | 66 | 75 | 67 | 67 |
| | | | + | 3 | 0 | 05 | 05 |
| 6 | Epidural | Independent | - | 75 | 77.7 | 58 | 66 |
| | | | + | 0.8 | 201 | 0.9 | 0.3 |
| 7 | Lymph | Independent | - | 30 | 81.8 | 36 | 38 |
| | Node | | + | 0.1 | 10.4 | 0 | 0 |
| 8 | Bone | Independent | - | 75 | 52 | 71 | 75 |
| | | | + | 4 | 0 | 1.8 | 2.1 |
| 9 | Lymph | Independent | | 80 | 2.3 | 65 | 72 |
| | Node | | + | 2.2 | 2.3 | 2.1 | 2.1 |
| 10 | Lymph | Independent | - | 30 | 37 | 55 | 63 |
| | node | | + | 0 | 0 | 0.9 | 0.8 |
| 11 | Testicular | Independent | - | 30 | 87 | 48 | 56 |
| | | | + | 0.1 | 5.3 | 1.2 | 1.5 |
| 12 | Lymph | Independent | - | 40 | 68 | 33.3 | 33.3 |
| | Node | | + | 0.3 | 1.5 | 0 | 0 |
| 13 | Lymph | Independent | - | 66 | 54 | 68 | 65 |
| | Node | | + | 0.1 | 4.5 | 1.4 | 1.5 |
| 14 | Lymph | Dependent | - | 75 | 100 | 63 | 75 |
| | Node | | + | 0.1 | 48 | 1.8 | 1.5 |
| 15 | Lymph | Dependent | | 50 | 76 | 33.3 | 33.3 |
| | Node | | + | 0.3 | 1.3 | 0 | 0 |
| 16 | Lymph | Independent | - | | 32 | 97 | 97 |
| | Node | | + | 40 | 0 | 33 | 47 |
| 17 | Lymph | Dependent | - | 40 | 40 | 55 | 66 |
| | Node | | + | 1 | 0 | 1.5 | 2 |
| 18 | Bone | Independent | - | 87 | 83 | 83 | 86 |
| | | | + | 9.7 | 4 | 18 | 15 |
| 19 | Lymph | Dependent | - | 90 | 86 | 79 | 83 |
| | Node | | + | 40 | 17 | 2.3 | 2.5 |
| 20 | Lymph | Independent | - | 88 | 94 | 71 | 67 |
| | Node | | + | 7 | 35 | 1.2 | 0.9 |

Protein validation of these genes by western blot (Figure 2c) and immunofluorescence assays (Figures 2d and 2f) confirmed that the acquired resistant cells had a decrease in epithelial differentiation proteins, prostate specific biomarkers and immune surveillance antigens, and displayed an activation (nuclear expression) of developmental signaling pathways (Figure 2d). Activated β-catenin, Gli1, Gli2 and cleaved NOTCH-2 showed significant increase in protein expression as well as nuclear immunolocalization, when comparing Docetaxel resistant cells to parental sensitive cells, which displayed mainly a cytoplasmic/membranous expression. Furthermore, these phenotype were found to be linked to the chemoresistant phenomenon, since reversed acquired resistant cells recuperate the differentiated phenotype (Figure 13).

With respect to studies on differentiation, the expression of cytokeratins (CKs) as epithelial markers, as well as prostate-related biomarkers, including AR, PSA and PSMA, were assessed. CKs have been previously reported to be specific for human differentiated epithelial cells, playing a role in the maintenance of cellular integrity while also functioning in signal transduction and cellular differentiation processes (Brulet *et al.,* 1980; Lu *et al.,* 1980; Oshima *et al.,* 1981; Tesar *et al.,* 2007). Low molecular weight CKs (e.g., CK18, and CK19) are specifically expressed in luminal normal human prostate cells and prostate cancer, whereas high molecular weight CKs (*e.g.,* CK5 and CK10) are identified in basal normal prostate cells and rarely observed in cancer cell populations (Ali *et al.,* 2008). Docetaxel resistant cells showed a significant decrease in both gene transcription and protein expression of low molecular weight CKs. DU145-DR cells showed a 6.25 and 16.6 fold decrease in the protein expression of CKs 19 and 18 respectively. Similarly, 22RV1-DR showed a 14.3 and 6.7 fold decrease in such CKs when quantified and compared to the sensitive parental cells. Immunofluorescence staining of CK19 and CK18 confirmed their decreased expression in the Docetaxel resistant cells (Figure 2). Moreover, high molecular weight CKs continued to be undetectable in the Docetaxel resistant cells as in their corresponding parental cells (data not shown), indicating that in the process of acquiring Docetaxel resistance, cells lose epithelial differentiation markers and do not undergo a shift from a luminal (low molecular weight CK) to a basal-like (high molecular weight CK) phenotype. Furthermore, 22RV1 cells, which express prostate-related differentiation markers including AR, PSMA and PSA, showed a dramatic decrease of gene and protein expression levels of such markers when exposed to Docetaxel (Figure 2). 22RV1-DR cells showed a 16.6, 20.0 and 11.1 fold decrease in the protein expression of AR, PSMA and PSA respectively. These results were further confirmed by immunofluorescence analysis (data not shown).

Regarding mechanisms of immune evasion, it was found that Docetaxel resistant cells showed deregulation of MHC class I molecules. It was observed that MHC class I antigens, which are critical for efficient antigen presentation to cytotoxic T lymphocytes and subsequent tumor cell lysis, were down-regulated at both gene transcription and protein level (Figure 2). Gene expression profiling revealed a significant down-regulation in all MHC class I antigens (A, B, C, E, F, G), a fact that was confirmed at the protein level by immunoblotting and immunofluorescence staining of MHC class I antigens A, B, C (Figure 2). Thus, these cells exhibit a phenotype that favors immune evasion, making them undetectable by the host immune system.

Regarding studies on stem cell phenotype, the identified deregulated expression of WNT/β-catenin, Notch and Hedgehog was characterized. These pathways have been implicated in self-renewal and differentiation of progenitor cells (Katoh *et al.,* 2007; McDonald *et al.,* 2006; van den Brink *et al*., 2004; Radtke *et al*., 2006; Leong *et al*., 2008; and Grigoryan *et al.,* 2008). In the prostate, these signaling pathways play essential roles in developmental patterning, epithelial regeneration, and prostate cancer tumorigenesis (Wang *et al*., *2006,* Karhadkar *et al., 2004).* Docetaxel resistant cells showed a significant decrease in both gene transcript and protein levels of the WNT inhibitor Dickkopf-I (DKKI), a well known inhibitor of the WNT/β-catenin signaling network (Fedi *et al.,* 1999). This decrease in DKK1 expression was linked to an increase in the expression of de-phosphorylated (active) β-catenin, which is the major key effector of WNT signaling. Immunofluorescence analyses demonstrated that parental Docetaxel sensitive cells displayed a membranous expression of β-catenin, associated to its function as an adhesion molecule, whereas Docetaxel resistant cells showed a pronounced nuclear localization of this protein (Figure 2), reported as necessary for the activation of the canonical WNT signaling pathway (Willert *et al.,* 2006). Moreover, Docetaxel resistant cells also exhibited an increase in the NOTCH signaling network. NOTCH2 gene transcript levels were significantly increased in the resistant cells and were linked to an increase in cleaved Notch2 protein expression that was associated with nuclear translocation of the protein, where it exerts its transcriptional activity (Figure 2). Finally, Docetaxel resistant cells had an increased expression of the Hedgehog receptor Patched and the glioma associated oncogene homolog transcription factors, Gli1 and Gli2. These findings were associated with an increased protein expression and nuclear translocation of the above mentioned transcription factors (Figure 2), a condition that has been related to Hedgehog pathway activation. Surprisingly, other reported stem cell surface markers, such as CD44 and CD133, were not found to be up-regulated in this study, as analyzed both at the gene transcript and protein levels (data not shown).

Based on the fact that the Docetaxel resistant cells exhibited a phenotype consistent with stemness, whether these cells displayed a higher tumor initiating capacity than the parental cells was tested. Subcutaneous injection in NOD/SCID mice of 10² and 10³ Docetaxel resistant cells (22RV1-DR and DU145-DR) gave rise to significantly more tumors than their parental sensitive cells (Figure 2e). Injection of 10² DU145-DR and 22RV1-DR cells induced tumor formation in 83.3±7.8% and 79.0±10.4% of the recipients, respectively; whereas the injection of parental DU145 and 22RV1 cells induced tumor formation in only 38.8±15.2% and 46.0±9.3%, respectively (p<0.0001). Moreover, although there was no difference in tumor formation when 10⁴ parental and resistant cells were injected, tumor latency was significantly shorter for the resistant cells. The tumor latency for DU145 parental cells was 59.2±4.9 days versus 43.2±2.6 days for resistant cells (p<0.0001). Similarly, the tumor latency was significantly (p<0.0001) longer for 22RV1 cells (54.9±1.5 days) as compared to the resistant cells (35.3±1.9 days). Thus, the stemness molecular signature of Docetaxel resistant cells was functionally reinforced by their high tumor initiating capacity.

### EXAMPLE 2

### IDENTIFICATION AND CHARACTERIZATION OF PROSTATE CANCER STEM-CELLS

Considering the stemness signature and the higher tumor initiating capacity of the generated Docetaxel resistant cells, it was investigated whether the chemoresistance phenomenon was due to a transition of sensitive to resistant cells with stemness characteristics, or alternatively if chemotherapy would select for pre-existing prostate cancer stem cells (Figure 3c). Because DU145 and 22RV1 Docetaxel resistant cells commonly displayed down-regulation of CK19 and CK18, and MHC class I antigens, whether cells with a CK-negative/HLA class I-negative phenotype were already present in the parental lines was determined. Immunofluorescence staining revealed the presence of a small CK-negative/HLA class I-negative subpopulation in both cell lines, which represented a 2.19 ± 0.95% and 3.58 ± 0.79% of the total population of DU145 and 22RV1 parental cells, respectively when quantified by flow cytometry (Figure 4b).

It was then studied whether the identified CK-negative/HLA class I-negative tumor cells could survive Docetaxel exposure, thus being responsible for the acquired chemoresistance phenomenon. For this purpose, DU145 parental cells were stably transfected with a plasmid containing the promoter of CK19 driving the expression of the green fluorescence protein (GFP). (Figures 14a and 14b) DU145 parental cells were stably transfected with a plasmid containing the promoter of CK19 driving the expression of the green fluorescence protein (GFP) (Figure 6a). Co-expression of CK19 and GFP was confirmed by flow cytometry and immunofluorescence (Figure 6b). Cells that expressed CK19 were GFP positive (GFP+), whereas cells that did not express CK19 were GFP negative (GFP-). Furthermore, stable insertion of the promoter construct in CK19/GFP negative cells was confirmed by PCR (Figure 6C). In addition, it was demonstrated that these CK19/GFP negative cells were HLA negative both by flow cytometry and immunofluorescence (Figures 6d).

Unsorted DU145-CK19 promoter-GFP stable cells were seeded and exposed to Docetaxel (10 nM) and live imaging was performed for periods of up to 48 hours. The resulting movies showed that Docetaxel exposure selected for cells with the GFP-negative phenotype which were able to divide and exit mitosis under therapy, whereas GFP-positive cells died after mitotic arrest (Figure 5b). Flow cytometry analysis revealed that Docetaxel treatment resulted in a significant shift in the proportion of surviving cells based on their respective phenotypes (Figure 5c). While initially cells displaying the GFP-positive phenotype represented 87.5 ± 10.4% of the total population, it was observed that after treatment this phenotype decreased, constituting only 28.3 ± 10.6% of the total cell population (p<0.001). In contrast, cells with the GFP-negative phenotype increased proportionally from 6.4 ± 4.3% to 73.1 ± 10.2% of the total population after treatment (p<0.001). Colony formation assays of DU145-CK19 promoter-GFP stable cells sorted by GFP/HLA class I expression confirmed these results, since only tumour cells with a GFP-negative/HLA class I-negative phenotype were able to form clones when continuously exposed to Docetaxel. (Figure 5d). Colony formation assays confirmed these results, since only tumor cells with a GFP-negative phenotype were able to form clones when continuously exposed to Docetaxel (Figure 5d). Thus, the results highlight the existence of a cell population with an undifferentiated phenotype (CK-negative/HLA class I-negative) which is spared by standard chemotherapy and could be responsible for tumor relapse.

It was also demonstrated that the subpopulation of tumor cells with a CK-negative/HLA class I-negative/GFP-negative phenotype had the unique stem cell property to differentiate through asymmetric cell division. When seeding unsorted DU145-CK19 promoter-GFP stable cells, it was observed that through such process GFP-negative cells give rise to daughter cells that enter the program of differentiation (become GFP-positive), while the GFP-negative cell retains its original identity (Figure 6a). In contrast, cells displaying a GFP-positive phenotype divided symmetrically, meaning that all daughter cells continue to possess the same characteristics. Flow cytometry analysis of DU145-CK19 promoter-GFP stable GFP sorted cells cultured during 4 weeks revealed that after this period of time, cells derived from GFP-negative sorted cells were majorly constituted by differentiated (GFP+) cells, whereas cells grown from GFP-positive sorted cells maintained the differentiated phenotype (Figure 6b). Thus this result explains the fact that the CK-negative/HLA class I-negative cells represent a minority of the tumor cell population inside the parental cell lines, whereas large sets of differentiated malignant cells constitute the major part of this total cell population.

Finally, the functional cancer stem cell property of tumor initiation in the identified subpopulations of cells was addressed. DU145-CK19 promoter-GFP stable cells were sorted by GFP/HLA class I expression and the obtained GFP sorted subpopulations of cells were injected subcutaneously into immunodeficient NOD/SCID mice and only the GFP-negative/HLA class I-negative cells exhibited tumor initiating capacity (Figure 6c). Injection of 10 cells with the GFP-negative phenotype produced tumors in 63.0 ± 14.6 % of recipients while no tumor formation was observed after injection the same amount of GFP-positive cells (Figure 6d).

In order to further confirm the observed findings in which only the DU145 CK-negative/HLA class I-negative/GFP-negative phenotype cells showed tumor initiating capacity, parental cell lines (DU145 and 22RV1) were sorted based on the expression of surface marker HLA class I antigen and their tumor initiating capacity was tested. (Figures 13 and 15). Similar to the results obtained with the GFP-negative cells, only the HLA class I-negative cells exhibited tumor initiating capacity after dilution assays. Injection of 10 HLA class I-negative DU145 and 22RV1 cells produced tumors in 83.3±19.1% and 100% of recipients, respectively, while no tumor formation was observed after 198 days of injection with 10 cells displaying a HLA class I-positive phenotype. Similar results were obtained after serial transplantation from HLA class I-negative generated tumor xenografts (data not shown).

Thus, the functional cancer stem cell property of tumor initiation was intrinsic to the subpopulation of cells with a CK-negative/HLA class I-negative phenotype. Furthermore, in addition to being the only cells endowed with tumour initiating capacity, HLA class I-negative cells also displayed a statistically significant higher clonability capacity when compared to HLA class I-positive cells (Figure 8). Most importantly, but not surprisingly, tumors generated from xenotransplanted GFP-negative cells displayed a differentiated (GFP-positive/CK-positive/HLA-positive) phenotype (Figure 6c), and retained a small population of GFP-negative/HLA-negative cells that accounted for 3.93±0.85% of the total tumour population (Figure 16).

Taken together, *in vitro* and *in vivo* results identify a cell population with a CK-negative/HLA class I-negative phenotype with tumor initiating capacity, which through asymmetrical cell division generates transit amplifying clonogens that overwhelmingly populate the evolving tumor with differentiated cells. Thus, confirming the discovery of a prostate cancer stem cell. Considering all of the results described above, including the stemness signature and the tumor initiating capacity of the newly generated Docetaxel resistant cells, it was hypothesized that chemotherapy induces an enrichment of prostate cancer stem cells (Figure 9a). Since the Docetaxel resistant cells displayed downregulation of epithelial differentiation markers, including CK19 and CK18, the CK-negative population of cells was initially quantified in the parental lines. Flow cytometry analysis revealed that both parental cells possessed scattered CK19 and CK18 negative subpopulations (Figure 6a). 22RV1 cells displayed 3.4% CK19 and 4.7% CK18 negative cells. Similarly, DU145 cells showed 2.1% CK19 and 2.3% CK18 negative cells. Moreover, 22RV1 had a 2.6% and DU145 had a 1.3% CK negative cells when co-stained with both CKs. Hence the immunofluorescence staining confirmed the presence of both a rare CK negative subpopulation, and a dominant subpopulation co-expressing both CKs.

### EXAMPLE 3

### IDENTIFICATION OF CANCER STEM CELLS IN HUMAN METASTATIC PROSTATE TUMOR SAMPLES

In view of the above results, it was investigated whether the identified prostate cancer stem cell population was present in human prostate cancer tissue samples. Immunohistochemical studies of metastases (n=20) and matched primary (n=6) human prostate cancer tissues revealed a scattered subpopulation of CK-negative tumoral cells (Figure 7a). These cells were also negative for HLA class I antigens (Figure 7b), displayed activation of key developmental transcription factors (Figure 7c) and lacked the expression of prostate-related differentiation markers (Figure 7d) corresponding to the stemness signature previously observed in *in vitro* studies.

All human prostate cancer specimens analyzed contained scattered subpopulations of CK-negative (CK18 and CK19) tumor cells, accounting for 0.05% to 0.3% and 0.4% to 1.8% of all tumor cells in primary and metastatic lesions, respectively (Figure 7a). Next, immunofluorescence-based double staining was performed to assess the association between CK expression and the markers of interest. In this analysis, it was consistently observed that CK expression was significantly associated to HLA class I expression (p<0.0001). More specifically, it was observed that the CK-negative tumor population did not express HLA class I antigens in 97.8 ± 0.7% of the cells. Nevertheless, all (100%) of the CK-positive cells displayed a positive HLA class I antigen phenotype (Figure 7b). Furthermore, it was persistently found that CK-negative/HLA-negative tumor cells had a significant (p<0.0001) increase of nuclear expression (activation) of developmental transcription factors when compared to differentiated CK-positive/HLA-positive cells. CK-negative/HLA-negative cells displayed nuclear expression of de-phosphorylated β-catenin in 63.9 ± 22.6% of cells, cleaved Notch2 in 72.8 ± 15.1%, Gli1 in 67.5 ± 17.3%, and Gli2 in 67 ± 17.3%, whereas CK-positive/HLA-positive cells expressed nuclear de-phosphorylated β-catenin in only 5.8 ± 11.9% of cells, cleaved Notch2 in 6.7 ± 7.9%, Gli1 in 1.2 ± 7.9%, and Gli2 in 1.5 ± 10.6% (Figure 7c). Moreover, it was also observed that the CK-negative/HLA class I-negative tumor cells showed no expression of nuclear AR, whereas CK-positive/HLA-positive cells displayed nuclear AR in 71.8 ± 14.3% of the cells (Figure 7d). Thus the fact that prostate cancer stem cells do not display a positive AR phenotype suggests that these cells may not be dependent on a functional AR signaling, which would explain how these cells might be responsible for the observed relapse after hormone-therapy, an issue to be pursued in future studies. Taken together, these results confirm the existence and the ability to identify a subpopulation of prostate cancer stem cells in human prostate cancer tissue samples.

Given that the cancer stem cell population was identified in both primary and metastatic prostate cancer, a series of experiments were designed aimed at investigating the tumorigenic ability of such population of cells from fresh human tumor samples. To assess whether prostate cancer cells with such phenotype were responsible for tumor initiation, tumors from 48 patients who underwent radical prostatectomy for primary prostate cancer were obtained. (Table 4). Cells were isolated by flow cytometry based in the expression of the cell surface marker HLA class I (Figure 9a). This analysis showed that all primary prostate cancer samples were mainly constituted by HLA class I-positive cells which accounted for a median of 98.9 ± 0.35% (range 98.7%-99.5%) of the total population whereas only a small percentage of cells median 1.2 ± 0.65% (range 0.5%-1.5%) showed an HLA class I-negative phenotype, being this result in agreement with the above reported immunohistochemical findings. Next, the same number (10, 10² and 10³) of HLA class I-negative, HLA class I-positive and unsorted cells mixed with Matrigel were injected into NOD/SCID mice. Overall, 4 (8.3%) of 48 xenograft tumors developed from the injection of primary prostate cancer cells after a median follow-up time of 34.5 weeks (range 21.0-45.3). 20.8 weeks (range 9.3-39.6), and only the HLA-negative cells could maintain their tumorigenic potential following serial transplantation when compared to the HLA-positive cells (Figure 9b and Table 5 below).

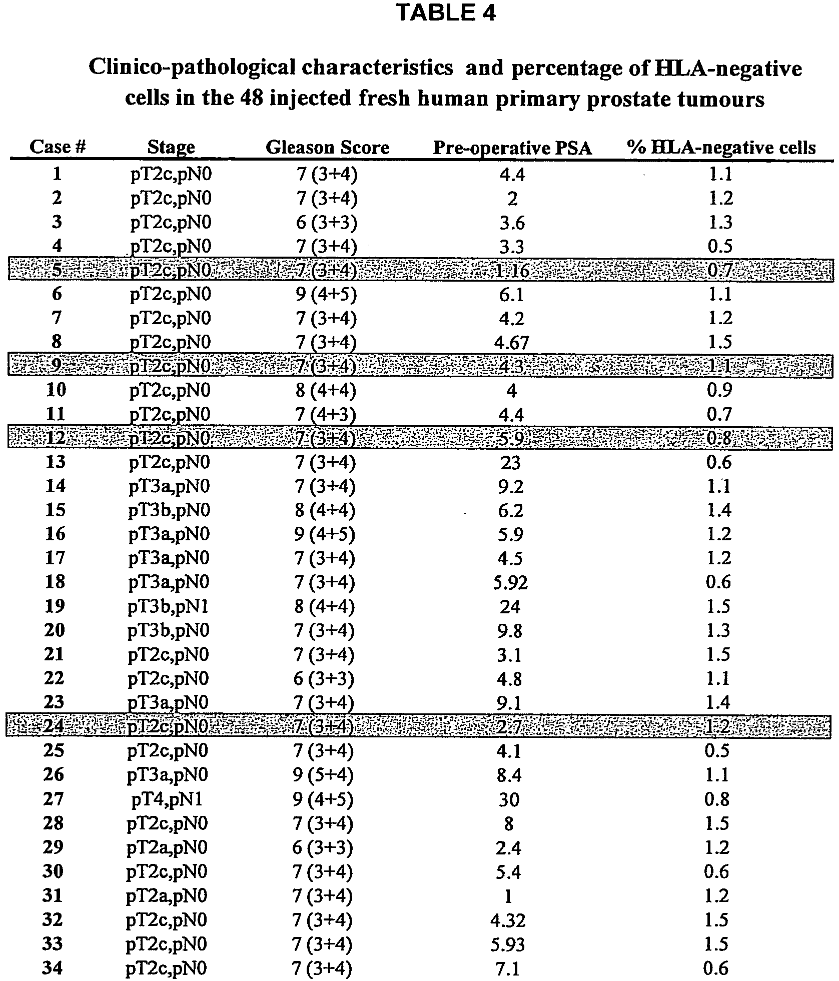

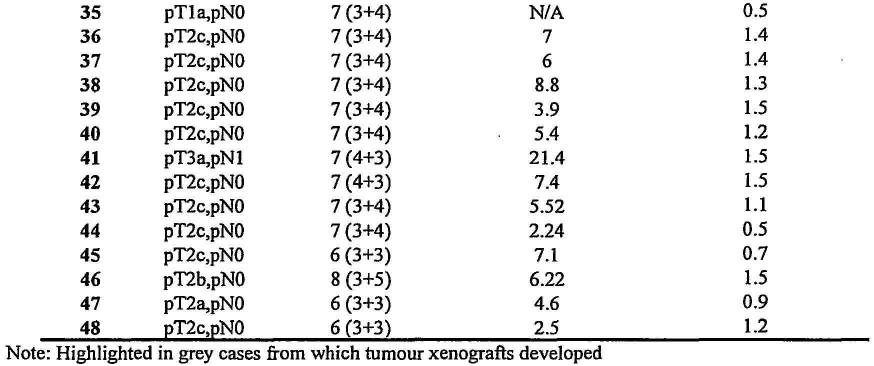

Table 5 summarizes the tumour initiating capacity measured by tumour incidence (tumours/injections) and tumour latencies in weeks (mean ± SD), when 10, 100 and 1000 HLA class I sorted and unsorted cells from primary prostate cancer tissues were injected. Four mice for each sorted cell population and cell dilution were injected twice in the upper flanks (HLA-negative) and lower flanks (HLA-positive). Unsorted cells from each tumour specimen were also injected.

**TABLE 5**

| | | **Primary injections** | | | | **Secondary injections** | | | |
|---|---|---|---|---|---|---|---|---|---|
| | | **Tumours/ Injections** | | **Tumour latency** | | **Tumours/Injections** | | **Tumour latency** | |
| Patient | HLA class I expression | Cells/injection | | Cells/injection | | Cells/injection | | Cells/injection | |
| | | 100 | 1000 | 100 | 1000 | 100 | 1000 | 100 | 1000 |
| #5 | negative | 5/8 | 7/8 | 20.2±3.4 | 12.6±3.9 | 8/8 | 8/8 | 19.9±2.7 | 13.8±2.8 |
| | positive | 0/8 | 3/8 | - | 26.8±4.0 | 0/8 | 1/8 | - | 23.8 |
| | unsorted | 2/8 | 5/8 | 27.8±4.6 | 21.0±2.5 | 4/8 | 7/8 | 23.4±3.5 | 23.1±6.4 |
| #9 | negative | 7/8 | 8/8 | 15.5±3.2 | 10.8±1.5 | 7/8 | 8/8 | 14.2±2.5 | 10.3±1.5 |
| | positive | 0/8 | 1/8 | - | 22.4 | 0/8 | 0/8 | - | - |
| | unsorted | 2/8 | 6/8 | 22.0±2.8 | 15.3±3.6 | 1/8 | 8/8 | 23.5 | 14.7±3.2 |
| #12 | negative | 6/8 | 8/8 | 19.4±2.0 | 12.6±3.6 | 8/8 | 8/8 | 20.3±4.5 | 16.6±2.5 |
| | positive | 0/8 | 0/8 | - | - | 0/8 | 0/8 | - | - |
| | unsorted | 1/8 | 5/8 | 23.4 | 22.2±3.6 | 0/8 | 3/8 | - | 19.5±1.5 |
| | | | | | | | | | |
| #24 | negative | 3/8 | 5/8 | 25.3±4.9 | 20.9±5.4 | 0/8 | 2/8 | - | 15.2±3.9 |
| | positive | 0/8 | 0/8 | - | - | 0/8 | 0/8 | - | - |
| | unsorted | 0/8 | 2/8 | - | 26.5±3.5 | 0/8 | 0/8 | - | - |

After primary injection of 10³ cells, it was observed that a significantly higher tumor initiating capacity was from the HLA-negative sorted cells (87.5±17.6%) when compared to the HLA-positive cells (12.5±17.6%), a fact that was linked to a lower tumor latency in the HLA-negative xenografts (14.2±4.5 versus 24.6±3.1 weeks). Moreover, further dilution into 10² and 10³ cells revealed that only the HLA-negative cells were endowed with tumor initiating capacity. These observations were confirmed by secondary transplantation experiments in which injection of 10² and 10³ sorted cells continued to demonstrate that tumor development was restricted to the HLA-negative subpopulation of cells (Figure 9b). Secondary injections of HLA sorted cells from the rarely generated xenografts from HLA-positive cells confirmed this result, since HLA-positive cells did not possess tumour initiating capacity. Histological and immuno-histochemical analyses revealed that tumors derived from HLA-negative sorted cells faithfully reproduced the phenotype of the original primary human tumor (Figure 9c), showing expression of HLA class I antigens in the majority of their tumor cells, as well as epithelial and prostate related markers (CK and AR). Thus these results show that the HLA-negative population is enriched in cells capable of initiating prostate cancer xenografts in NOD/SCID mice, and reproducing the molecular and phenotypic heterogeneity distinctive of most human cancers.

Moreover, it appears that this is a universal phenomenon, because HLA-negative tumor cells were isolated from a variety of fresh human solid neoplasms, including colon, breast, lung and bladder carcinomas which are responsible for tumor initiation when serially injected into NOD/SCID mice (Figure 9d, and Table 6 below). Overall, 4 of 10 (40%) colon, 2 of 10 (20%) lung, 2 of 12 (16.6%) breast and 2 of 18 (11.1%) bladder xenograft tumors developed from injection of fresh human tumor samples. Characteristics of other primary solid tumor types are shown in Table 7 below. Tumor initiating capacity and tumor latencies of HLA sorted cells from other solid cancer tissue types are shown in Table 8 below.

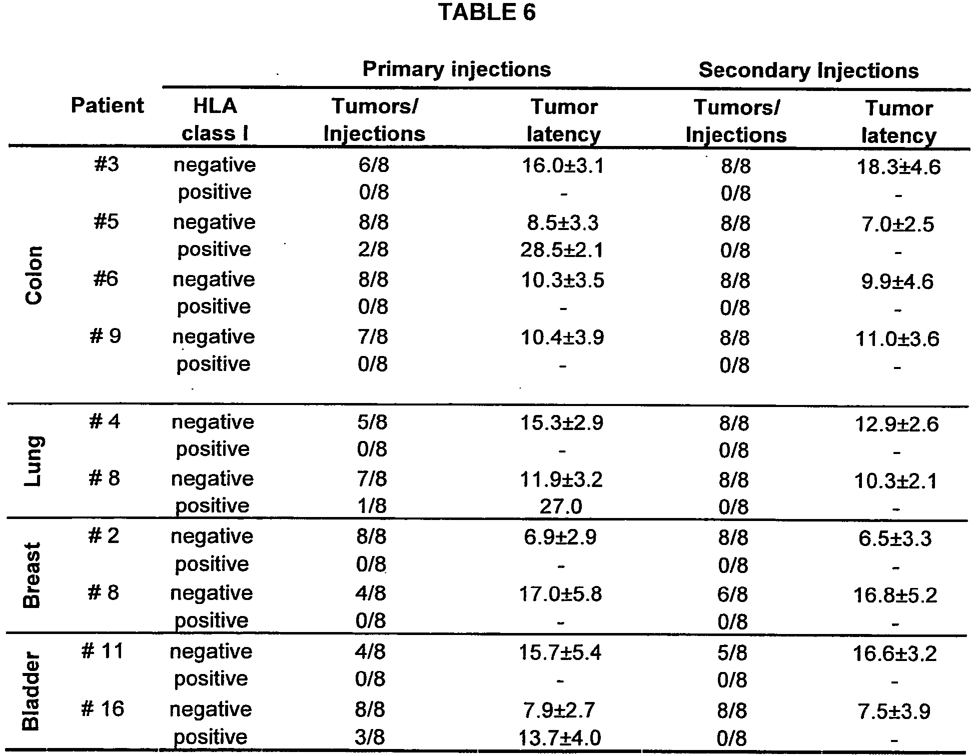

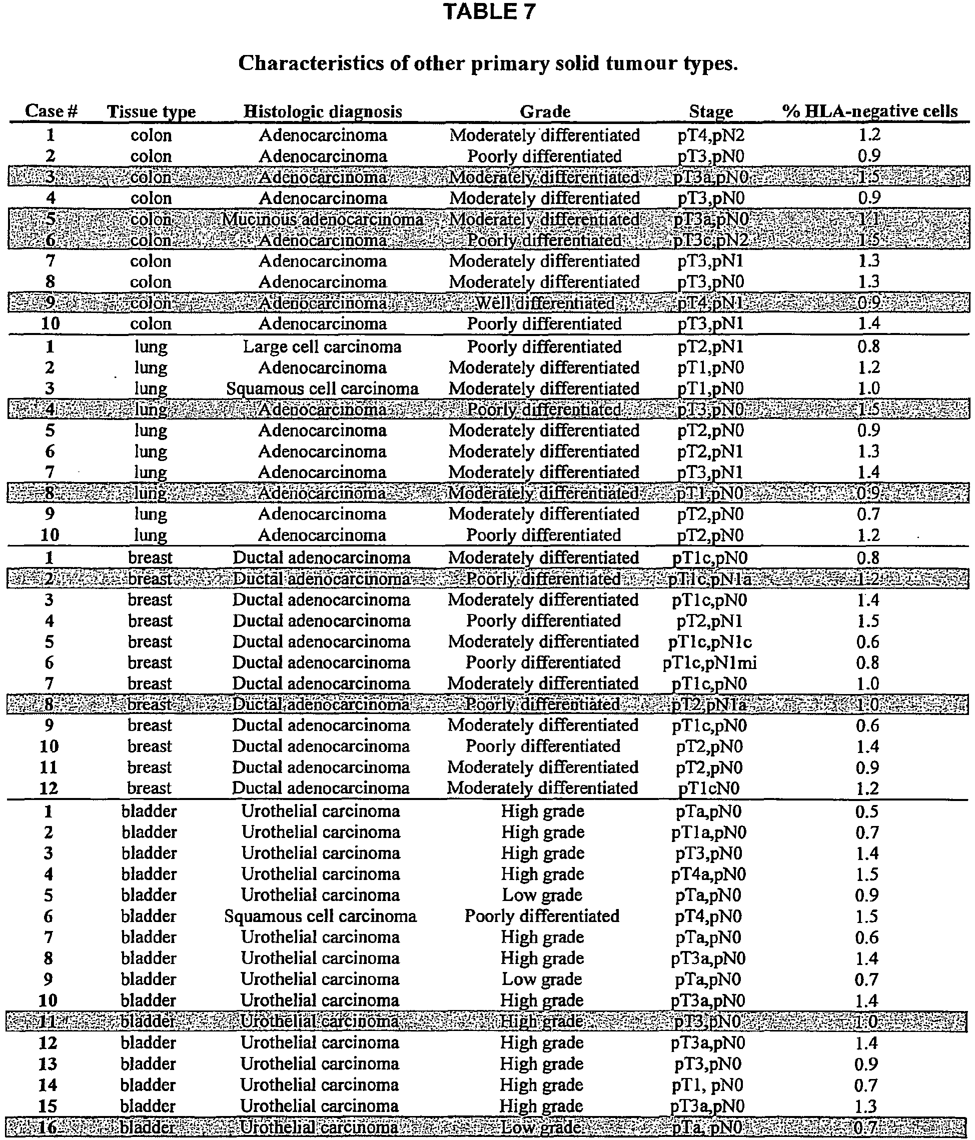

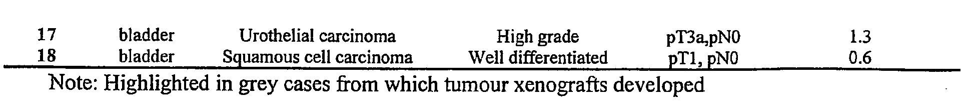

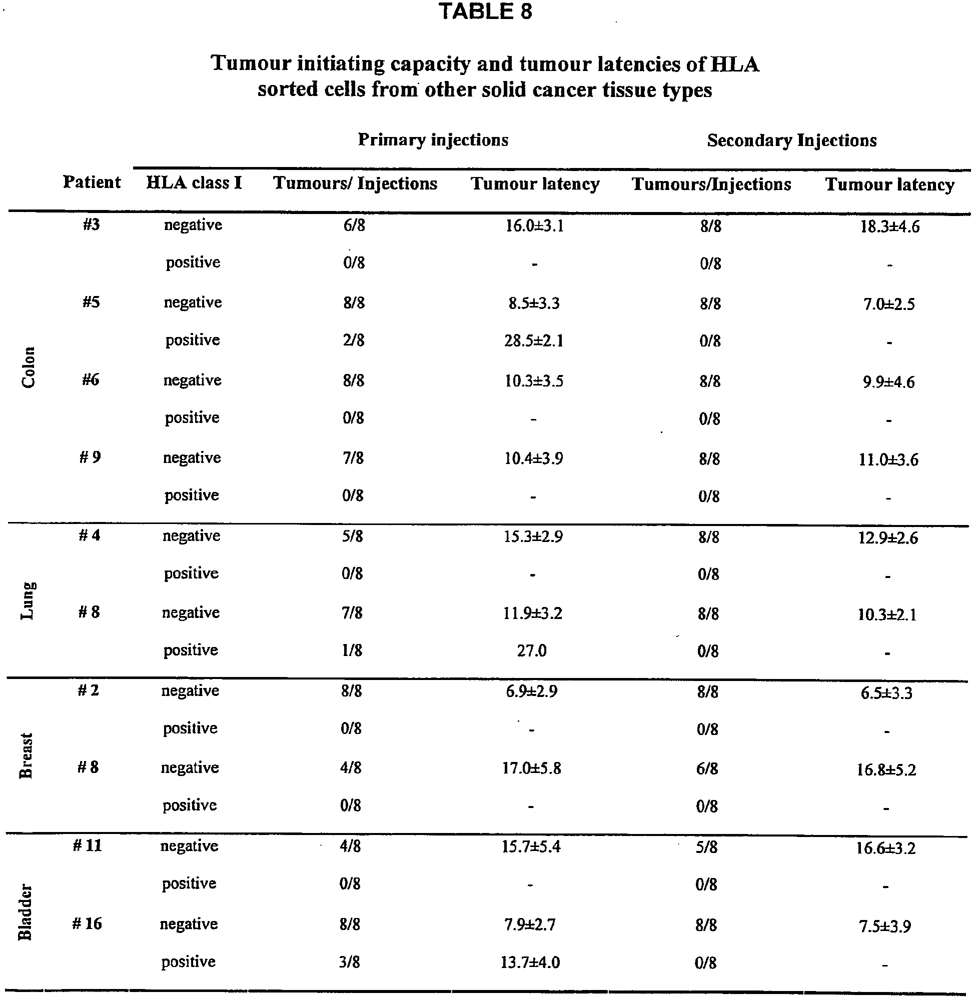

Table 8 summarizes the tumour initiating capacity measured by tumour incidence (tumours/injections) and tumour latencies in weeks (mean ± SD), when 100 HLA class I sorted cells from other primary tumour tissue types were injected. Four mice for each sorted cell population and cell dilution were injected twice in the upper flanks (HLA-negative) and lower flanks (HLA-positive).

After primary injection, tumor initiating capacity of 10² HLA-negative cells was significantly higher when compared to HLA-positive sorted cells. More importantly, following serial transplantation only the HLA-negative cells retained tumorigenic capacity, whereas HLA class I-positive cells did not. Moreover, secondary injections of HLA-positive cells sorted from xenografts generated from HLA-positive cells did not possess tumour initiating capacity. The generation of tumours from HLA class I-positive cells could occur because of possible contamination of HLA class I-negative cells during cell sorting, although it cannot be excluded that HLA class I-positive cells may have a low tumour initiating capacity that cannot be maintained after serial transplantation. Histological analysis of the developed xenograft tumors showed similar morphological characteristics than their corresponding human primary cancers (Figure 9e). Thus, an HLA-negative population with tumor initiating capacity has been identified in human epithelial tumor types in addition to prostate cancer.

Finally, the tumor initiating capacity of cells from fresh human tissue samples was not related to any of the analyzed clinico-pathological characteristics of the cancer patients, neither associated with the percentage of HLA-negative cells. Tumours with aggressive clinico-pathological characteristics (*e.g*., high grade, high tumour stage) or tumours with a high number/percentage of HLA-negative cells (*e.g*., 1.5%) did not possess a significantly higher tumour initiating capacity. Moreover, no association was observed between the percentage of HLA-negative cells and either tumour stage or tumour grade.

### EXAMPLE 4

### TARGETING THE HUMAN CANCER STEM CELL

Based on the fact that the identified cancer stem cells exhibited Hedgehog and Notch pathways activation, testing was done as to whether inhibition of such pathways could impair cancer stem cell homeostasis. For this purpose, Cyclopamine, a plant derived hedgehog pathway antagonist that acts at the level of Smo (Taipale *et al*., 2000; Karhadkar *et al*., 2004; Chen *et al.,* 2002), was utilized. Compound E is a highly active gamma-secretase inhibitor that blocks the proteolytic processing of Notch receptors (Seiffert *et al.,* 2000). For the *in vitro* experiments, DBZ, a highly active gamma-secretase inhibitor with established *in vivo* activity, was used as a substitute for Compound E in the *in vivo* experiments outlined below (van Es *et al.,* 2005).

Exposure of the CK-negative/HLA-negative cancer stem cell population from DU145 and 22RV1 to each individual inhibitor did not induce any major effect. However, when both agents were administered together a robust inhibition in cell cycle progression with an accumulation of cells in sub-G1 was observed, whereas this effect was minor in differentiated (CK-positive/HLA-positive) cells (Figure 10a). Because dexamethasone is used to reduce gut toxicity of gamma-secretase inhibitors in *in vivo* experiments (Real *et al*., 2009), the *in vitro* effects of dexamethasone in combination with the above mentioned inhibitors were analyzed. This analysis showed that dexamethasone did not change the cell cycle effects of Hedgehog and Notch inhibitors (data not shown). Moreover, the combined effect of these drugs was further confirmed by colony formation assays, since no colony formed after 21 days when Hedgehog and Notch inhibitors were combined (Figure 10b). Next, to determine whether the inhibition of these pathways could affect the tumor initiating capacity of the cancer stem cells *in vivo,* 10³ DU145 and 22RV1 CK-negative/HLA-negative cells were injected subcutaneously into NOD/SCID mice and treated with vehicle solution (Control), dexamethasone alone, dual combination (e.g. dexamethasone plus Cyclopamine) or triple combination (dexamethasone plus Cyclopamine plus DBZ) of the drugs. Mice treated with the triple combination showed a significant (p<0.05) delay in tumor first palpability of 5.1 weeks in DU145 and 3.8 weeks in 22RV1 xenografts, whereas this tumor delay was not significant in mice treated with Hedgehog or Notch inhibitors alone. (Figure 10c).

Finally, the tumor initiating inhibitory effects of these compounds was tested in xenografts derived from fresh human prostate cancer tissues. 10³ HLA-negative sorted cells from xenografts #5, #9 and #12 were injected into NOD/SCID mice and treated with the same schedules and combinations of Hedgehog and Notch inhibitors, as described above. As observed previously in the cell lines, only the combined treatment with Hedgehog and Notch inhibitors significantly (p<0.05) delayed tumor initiation (Figure 10d). Tumor xenografts in mice treated with the triple combination of drugs were first palpable after 18.3±3.1, 13.8±2.5 and 20.1±3.3 weeks, compared to 13.8±2.5, 10.3±1.5 and 16.6±2.5 weeks in their corresponding controls.

Taken together, these results show that the inhibition of these developmental pathways targets the cancer stem cell population delaying tumor initiation.

Two major hypotheses regarding tumor initiation have been postulated. The "stochastic model" which predicts that every neoplastic cell can generate an entirely new tumor; and the "cancer stem cell model" which proposes that tumor cells exist in a hierarchical state, and that only a few stem cells possess tumor initiating potential. The identification and functional characterization of a human cancer stem cell is disclosed, which fulfills the following stemness criteria: 1) self-renewal and differentiation through asymmetrical cell division, 2) tumor initiating capacity, 3) a negative histocompatibility signature, and 4) a multidrug resistance phenotype.

An HLA-negative phenotype is also shared by embryonic stem cells. It has been reported that human pre-implantation embryos are HLA class I and class II negative (Desoye *et al*., 1988). This phenomenon precludes rejection based on expression of paternal antigens, until a blood-tissue barrier develops, in this situation being the placenta. In the context of cancer stem cells, such a histocompatibility negative phenotype has major clinical implications, since it explains host mutation permissiveness, as well as tumor spread and metastatogenic capabilities, since cancer stem cells would escape immune-surveillance.

Concerning tumor initiation capacity, it is disclosed that only the identified CK-negative/HLA-negative cancer stem cells generate tumors *in vivo,* while the differentiated, CK-positive/HLA-positive progenies lack such property. However, it appears that these cancer stem cells exhibit genetic memory independent of certain stroma interactions, since subcutaneous injections confer the tissue-of-origin phenotype without the need for orthotopic implantation, a phenomenon that needs to be further investigated. Moreover, due to its homogenous phenotype, it was hypothesized that these cells are genetically stable, a property facilitated by their quiescent state and asymmetrical division. This genetic stability would allow for the identification of "driver" mutations, since molecular heterogeneity would be essentially a product of the tumor expanding and differentiated cancer cell populations, and probably not as critical for tumorigenesis. A new molecular classification of human tumors could probably be derived from analysis of such "driver" mutations in these cancer stem cell populations.

In sum, a newly defined prostate cancer stem cell in human cancer cell lines and tissue samples has been identified and characterized. Moreover, this population of cells was isolated using HLA class I surface marker, and its tumor initiating capacity was demonstrated. Further, it was observed that similar CSC populations are also present in human breast, colon, lung and bladder carcinomas, a fact that gives further universality to these findings. The discovery of this human cancer stem cell has important clinical implications in diagnostic and predictive laboratory assays, as well as for development of novel therapeutic strategies. In this context, treatment with Notch and Hedgehog inhibitors attenuates tumor formation in experimental animal models.

### EXAMPLE 5

### MATERIALS AND METHODS

### Inhibitors and drugs

Docetaxel, Mitoxantrone, Doxorubicin, Cisplatin, Vinorelbine, Paclitaxel, Dexamethasone, Cyclopamine and Compound E were obtained from Sigma-Aldrich (St. Louis, MO). DBZ [(2S)-2-[2-(3,5-difluorophenyl)-acetylamino]-N-(5-methyl-6-oxo-6,7-dihydro-5H-dibenzo[b,-d]azepin-7-yl)-propionamide] was obtained from Syncom (Groningen, The Netherlands).

### Cell culture, generation and characterization of acquired Docetaxel resistant cells

Human hormone-independent prostate cancer cell lines, DU-145 and 22RV1, were obtained from American Type Culture Collection (ATCC) and maintained in RPMI 1640 medium (Gibco, Invitrogen Corp., Carlsbad, CA) supplemented with 10% FBS without antibiotics. Cells were grown at 37°C in a humidified atmosphere with 5% CO₂. DU145 and 22RV1 cells were selected in order to generate a prostate cancer Docetaxel resistance model. This selection was based on the fact that both cell lines are hormone-refractory, a condition treated with Docetaxel in the clinical setting, and that they also exhibit distinct hormone-refractory phenotypes. While 22RV1 cells are still dependent of androgen receptor signaling and express prostate specific markers (e.g., PSMA, androgen receptor), DU145 do not. Thus, the generation of acquired Docetaxel resistance in these two phenotypically distinct cell lines facilitated an approach to study the molecular processes involved in the acquisition of Docetaxel resistance. Docetaxel resistant clones, DU-145-DR and 22RV1-DR, were selected by culturing cells with Docetaxel in a dose-escalation manner. Initial culture was at 5 nM Docetaxel. After the sensitive clones were no longer present and the surviving DU-145 and 22RV1 cells repopulated the flask, the concentration of Docetaxel was increased to 10 nM and subsequently to 25 nM, 50 nM, 100 nM and 250 nM. 22RV1-DR cells were further exposed to 500 nM Docetaxel. After exposure to each increasing dose of Docetaxel, the remaining surviving cells were maintained in culture medium containing the last selection escalating dose of Docetaxel. The last drug selection concentration at which the cells were exposed was 250 nM for DU-145-DR and 500 nM for 22RV1-DR, in order to avoid reversibility of the acquired Docetaxel resistance phenotype. The process of acquired drug resistance took 9 months for DU-145-DR and 6.5 months for 22RV1-DR. In parallel, parental DU-145 and 22RV1 cells were exposed to DMSO (vehicle solution of Docetaxel) in the same dose-escalation manner.

Cells were exposed to increasing doses of Docetaxel, and the acquired chemoresistance was characterized and confirmed by cell viability, colony formation, annexin V, and poly-(ADP-ribose) polymerase (PARP) cleavage assays (Figures 1a-d). The generated Docetaxel resistant (DR) cells showed cross-resistance to DNA damaging drugs (Mitoxantrone, Doxorubicin and Cisplatin), as well as other anti-mitotic agents (Vinorelbine and Paclitaxel), consistent with a multidrug resistant phenotype (data not shown). It was also observed that the Docetaxel chemoresistance was reversible (Figures 12a and b). Removal of the drug from culture medium induced a significant decrease in drug resistance. After 12 weeks of culturing Docetaxel resistant cells without drug, cell viability decreased significantly. Docetaxel IC-50 concentrations for DU145-DR cells decreased from 1µM to 25 nM and in 22RV1-DR cells IC-50 drug concentrations decreased from 10µM to 50nM (Figure 12a). This decrease in docetaxel resistance after drug removal was confirmed by colony formation assays (Figure 12b). Thus, in order to maintain the Docetaxel resistance phenotype, cells were continuously cultured under the last drug selection concentration.

### Cell viability and colony formation assays

Cell viability was analyzed using the Cell titer 96 Aquos Non-Reactive Cell Proliferation Assay (MTS) kit (Promega Corp., Madison, WI). Cells were seeded at a density of 10⁴ in 96-well culture dishes and 24 hours later medium was removed and replaced with new medium alone (control) or medium containing drugs. After 72 hours, color absorbance was measured on a microplate spectrophotometer (Molecular Dynamics) at 450 nm (test wavelength) and 620 nm (reference wavelength). The percentage of surviving cells was estimated by dividing the A 450 nm - A 620 nm of treated cells by the A 450 nm - A 620 nm of control cells. Clonogenic survival assays in response to drug treatment were performed by plating 10³ cells in 35 mm culture dishes. After 24 hours, cells were left untreated (control) or treated with drugs. Next day, medium was changed and the cells kept growing in fresh medium without any drug or under continuous exposure to drugs. For these continuous exposure experiments, medium plus drugs were replaced every 3 days until clones of drug-resistant cells appeared. Cells were then fixed with 4% paraformaldehyde in PBS, stained with crystal violet solution and formed colonies were visually counted.

### Analysis of apoptosis by flow cytometry

Cells (10⁵) were left untreated (control) or treated with drugs for 72 hours. Adherent and detached cells were pooled, washed and labeled with annexin-V-FITC and propidium iodide using the annexin-V-FLUOS Staining Kit (Roche, Nutley, NJ) according to manufacturer's instructions. Samples were acquired with a FACscan Flow Cytometer (BD Biosciences, San Jose, CA) and analyzed with CellQuest Pro software (BD Biosciences) to determine the percentage of cells displaying annexin V staining.

### Cell cycle analysis

Cells were treated with drugs for 72 hours, harvested and fixed in 70% ethanol and stored at 4°C. Before analysis, cells were washed with PBS, centrifuged and incubated for 30 min at room temperature in a staining solution containing 0.1% Triton X, 0.2 mg/ml RNAse and 0.02 mg/ml propidium iodide. DNA content was acquired with a FACscan Flow Cytometer (BD Biosciences) and analyzed with CellQuest Pro software (BD Biosciences).

### cDNA microarray analysis

22RV1, 22RV1-DR, DU-145 and DU-145-DR gene expression profiles were analyzed. Total RNA from each sample was isolated by Tryzol (Invitrogen) and purified by RNeasy mini kit and RNase-free DNase set (Qiagen Inc., Valencia, CA) according to the manufacturer's protocols. RNA quality of all samples was tested by RNA electrophoresis and RNA LabChip analysis (Agilent Technologies, Inc., Santa Clara, CA) to ensure RNA integrity. Samples were prepared for analysis with Affymetrix Human U133 Plus 2.0 arrays according to the manufacturer's instructions. Gene expression levels of samples were normalized and analyzed with Microarray Suite, MicroDB, and Data Mining tool software (Affymetrix, Santa Clara, CA). The absolute call (present, marginal, or absent) and average difference of 22.215 expressions in a sample, and the absolute call difference, fold change, average difference of gene expression between two or three samples were normalized and identified using this software package. Statistical analysis of the mean expression average difference of genes, which show ≥2-fold change based on a log normalization, was done using a *t* test between Docetaxel sensitive and resistant samples. Genes that were not annotated or not easily classified were excluded from the functional clustering analysis.

### Gene ontology analysis

Genes differentially expressed in the Docetaxel resistant cells compared to the parental sensitive cells generated a list of commonly deregulated transcripts. This list was assessed by the DAVID Bioinformatics Resources, a web-based statistical hypergeometric test applied for enrichment analysis of gene ontology (GO) categories, which are, biological process, molecular function, and cellular component. GO categories enriched on the highest hierarchical level (≥ level 5) at statistical significance (p<0.01) were taken into consideration.

### Western Blot analysis

Whole cell extracts were prepared in sample buffer and analyzed by immunoblotting. Primary antibodies against poly (ADP-ribose) polymerase (PARP) (BD Pharmingen, San Jose, CA), cleaved PARP (BD Pharmingen), cytokeratin 19 (Abcam), cytokeratin 18 (Abcam, Cambridge, MA), androgen receptor (Sigma-Aldrich), prostate specific membrane antigen (Abcam), prostate specific antigen (Epitomics, Burlingame, CA), pan-HLA class I (Abcam), DKK1 (Orbigen, BioCarta LLC, San Diego, CA), activated ß-Catenin (Millipore, Billerica, MA), ß-Catenin (BD Transduction), activated Notch2 (Abcam), PTCH (Abcam), Gli1 (Santa Cruz Antibody, Santa Cruz, CA), Gli2 (Abcam), and ß-Actin (Sigma-Aldrich) were used in immunoblot assays using standard procedures. Protein expression was quantified by comparing band expression using Quantity One software (Bio-Rad, Hercules, CA).

### Immunohistochemistry and immunofluorescence analyses

Immunofluorescence analyses were conducted on prostate cancer cell lines and formalin fixed paraffin-embedded tissue sections from human cancers and tumor xenografts. Primary antibodies included a combination of cytokeratin 19 and 18 (Abcam), pan-HLA class I (Abcam), green fluorescence protein (Abcam) and the following transcription factors: active ß-Catenin (Millipore), activated Notch2 (Abcam), Gli1 (Santa Cruz), Gli2 (Abcam) and androgen receptor (DAKO, Fort Collins, CO). Secondary antibodies used were Alexa Fluor® 594 (Invitrogen) and Alexa Fluor® 488 (Invitrogen). Prostate cancer cells (10⁵) were plated in 35 mm culture dishes and 24 hours later stained by standard immunofluorescence procedures. Tissue sections (5 µm) were deparaffinized and submitted to standard peroxidase based immunohistochemistry and immunofluorescence procedures. Quantification of the expression of cytokeratins, HLA class I antigen, transcription factors and androgen receptor was performed by evaluating tumoral cells. Percentage of positive and negative cells was determined in 10 high power fields.

### Generation of the cytokeratin 19-green fluorescent protein (GFP) reporter plasmid

CK19 gene promoter region was amplified from DU145 cells genomic DNA by PCR with specific primer sets (Fw 5'-AACGCATGCTTTGGGGGGATG-3' (SEQ ID NO: 1) and Rv 5'-TCCCCCTTTACTCGGCCCCCAC-3' (SEQ ID NO: 2)) as described previously (Tripathi *et al.,* 2005. The PCR products were digested with *Ase I* and *Hind III* and cloned into pEGFPN1 vector (Clontech, Mountain View, CA) previously digested with the same enzymes. As a result, the CMV promoter was removed from the original vector and the GFP expression was under control of the CK19 promoter. The final construct was confirmed by digestion and sequencing analysis. DU145 cells were transfected with pCK19-GFP construct using Lipofectamine Plus 2000 (Invitrogen). After 24 hours, medium was replaced with fresh medium and stably expressing cells selected in the presence of G418 (Invitrogen). Positive clones were confirmed by direct microscopy and immunofluorescence and also by PCR amplification of GFP coding region using specific primers (Fw 5'-TTCCTGCGTTATCCCCTGATTC-3' (SEQ ID NO: 3) and Rv 5'-GCTCCTCCGGCCCTTGCTCACCAT-3' (SEQ ID NO: 4)).

### Live Cell Imaging

Time-lapse videomicroscopy was used to assess asymmetrical cell division and Docetaxel subpopulation sensitivity of DU145 cells stably transfected with the pCK19-GFP promoter. Cells growing in 6-well plates at low confluence were placed in the stage inside an incubator chamber at 37°C, 50% humidity and in an atmosphere of 5% CO₂. Unattended time-lapse movies of randomly chosen GFP+ and GFP- DU145 cells were performed with a Nikon Eclipse Ti inverted microscope. NIS Elements AR (Nikon Inc., Melville, NY) software was used to collect and process data. Imaging was performed using a 10x objective and images were captured using 200-ms exposure times for GFP and 20-ms for bright field every 30 minutes.

### Analysis of subpopulations of cells by flow cytometry

Flow cytometry analysis of subpopulations of prostate cancer cells were carried out following standard procedures. Intracellular CK19 and CK18 expression was performed in single-cell suspensions fixed with 70% ethanol, whereas the expression of cell surface HLA class I and GFP was determined in fresh cell samples (without fixation). Primary antibodies against CK19 (Abcam), CK18 (Abcam), HLA class I (Abcam), HLA class I conjugated to phycoerythrin (Abcam) and GFP (Abcam) were used. Secondary antibodies, when used, corresponded to Alexa Fluor® 594 (Invitrogen) and Alexa Fluor® 488 (Invitrogen). Samples were acquired with a FACscan Flow Cytometer (BD Biosciences) and analyzed with a CellQuest Pro software (BD Biosciences). A minimum of 10⁴ cells were measured per sample.

### Mice procedures

Animal use and care was in strict compliance with institutional guidelines established by the University of Columbia, Institutional Animal Care and Use Committee. Xenograft experiments were performed with 5-6 weeks old mice (NOD.CB17-Prkdc^{scid}) obtained from Jackson Laboratories as recipients.

### Human primary and metastatic prostate cancer tissue samples

Formalin-fixed paraffin-embedded human primary and metastatic prostate cancer tissue samples were provided by the tumor bank of Columbia University Cancer Center. All samples were collected under informed consent and under the supervision of the Columbia University Medical Center Institutional Review Board. Tissue sections with cancer were selected by reviewing Hematoxylin & Eosin (H&E) stained slides.

### Tumour initiating capacity of cancer cells from prostate cell lines and fresh human samples

To compare the tumor initiating capacity of Docetaxel sensitive parental cells and Docetaxel resistant cells, GFP positive and GFP negative sorted cells, or HLA-positive and HLA-negative sorted cells, various numbers of cells (*e.g.* 10, 10², 10³, 10⁴) were subcutaneously injected in 200 µl of medium:Matrigel (1:1) into male mice. GFP cell subpopulations of prostate cancer cells were sorted following standard procedures. For HLA class I cell isolation, single suspensions of fresh cells where blocked with PBS + FBS 5% and stained with an HLA class I antibody directly conjugated to phycoerythrin (Abcam). To assess the tumor initiating capacity of human cancer cells from fresh tumor tissue samples, portions of tumors were obtained from patients who underwent surgical procedures at Columbia University medical Center through an Institutional Review Board approved protocol. Forty-eight primary prostate cancers, 10 primary colon cancers, 10 primary lung cancers, 12 primary breast cancers and 18 primary bladder cancers were processed. Specimens were mechanically dissociated and filtered to obtain a single-cell suspension and exposed to red cell lysis buffer (Sigma-Aldrich) to remove red blood cells. Cells were stained with directly conjugated fluorescent antibodies to human CD45 (Abcam), human CD31 (eBiosciences) and human HLA-class I (Abcam). For xenograft tumors, primary fluorescent conjugated antibodies to mouse CD45 (eBiosciences), mouse CD31 (Biolegend, San Diego, CA) and human HLA-class I (Abcam) were used to select live human cancer cells. Cells were suspended in 10 µg/ml DAPI to label dead cells and sorted on FACSAria Cell Sorting System (BD Biosciences). Different dilutions (10, 100 and 1,000 injected cells) of human prostate cancer HLA sorted cells (HLA class I-negative and HLA class I-positive) and unsorted cells, and 100 HLA sorted cells from other human cancer samples (primary injections) and derived xenografts (secondary injections) were injected into NOD/SCID mice. Four mice for each sorted cell population and cell dilution were injected. Four injections were performed in each mouse for sorted cells, two in the upper flanks for HLA class I-negative cells and two in the lower flanks for HLA class I-positive cells. Unsorted cells from each tumour specimen were also injected in NOD/SCID mice. Secondary injections of HLA sorted cells were performed from tumours generated from HLA class I-negative sorted cells and the rarely observed tumours originated from the HLA class I-positive fraction of cells. Tumour initiation was measured by tumor incidence (number of tumors/number of injections) and latency (time from injection to first tumor palpability). Tumour formation was evaluated regularly by palpation of injection sites. In cases where a tumor became palpable at only one injection site, that tumor was surgically removed to allow continued evaluation of other injection sites. Mice were monitored for up to 36 weeks. Animals with no sign of tumor burden were also examined on necropsy to confirm that there was no tumor development. Tumors harvested were fixed in formalin, and paraffin sections were made for H&E staining and immunofluorescence studies when necessary.

### In vitro effects of Notch and Hedgehog inhibitors

The *in vitro* effects of Notch and Hedgehog inhibitors on HLA-negative and HLA-positive sorted cell lines were analyzed by cell cycle and colony formation assays (described above). Cells were exposed to vehicle solution (Control), dexamethasone (1 µM), Cyclopamine (1 µM), Compound E (1 µM) and a dual (e.g. dexamethasone plus Cyclopamine) or triple (dexamethasone plus Cyclopamine plus Compound E) combination of the drugs.

### Effects of Notch and Hedgehog inhibitors in tumor initiation

To analyze whether the inhibition of these developmental pathways could affect the tumor initiating capacity of the cancer stem cells *in vivo,* 10³ HLA-negative sorted cells from cell lines and human prostate tumor xenografts were inoculated subcutaneously into NOD/SCID mice. Mice were treated with vehicle solution (Control), dexamethasone (15 mg/kg/ip. daily), Cyclopamine (50 µg/kg/sc daily) plus dexamethasone, DBZ (10 µM/kg/ip. daily) plus dexamethasone or a combination of the 3 drugs. Dexamethasone and Cyclopamine were administered continuously; however, DBZ was administered daily (days 1 to 15 every 4 weeks) in order to avoid gut toxicity. For the in vivo cell lines studies, three independent experiments in 8 mice for treatment arm (e.g., Cyclopamine) were performed, whereas for the human prostate tumours 8 mice were included for each treatment arm. Mice were monitored every day until tumors formed. Animals were sacrificed if they showed any evidence of distress or if they lost more than 20% of their original body weight. Generated tumors were harvested and histologically confirmed.

### Characterization of the chemo-resistant phenotype

Regarding studies on differentiation, the expression of cytokeratins (CKs) as epithelial markers was assessed, as well as prostate-related biomarkers, including AR, PSA and PSMA. CKs have been previously reported to be specific for human differentiated epithelial cells, playing a role in the maintenance of cellular integrity while also functioning in signal transduction and cellular differentiation processes. Low molecular weight CKs (e.g., CK18, and CK19) are specifically expressed in luminal normal human prostate cells and prostate cancer, whereas high molecular weight CKs (e.g., CK5 and CK10) are identified in basal normal prostate cells and rarely observed in cancer cell populations. In the model of the present invention, Docetaxel resistant cells showed a significant decrease in both gene transcription and protein expression of low molecular weight CKs. DU145-DR cells showed a 6.25 and 16.6 fold decrease in the protein expression of CKs 19 and 18 respectively. Similarly, 22RV1-DR showed a 14.3 and 6.7 fold decrease in such CKs when quantified and compared to the sensitive parental cells. Immunofluorescence staining of CK19 and CK18 confirmed their decreased expression in the Docetaxel resistant cells (Figure 2). Moreover, high molecular weight CKs continued to be undetectable in the Docetaxel resistant cells as in their corresponding parental cells (data not shown), indicating that in the process of acquiring Docetaxel resistance, cells lose epithelial differentiation markers and do not undergo a shift from a luminal (low molecular weight CK) to a basal-like (high molecular weight CK) phenotype. Furthermore, 22RV1 cells, which express prostate-related differentiation markers including AR, PSMA and PSA, showed a dramatic decrease of gene and protein expression levels of such markers when exposed to Docetaxel (Figure 2). 22RV1-DR cells showed a 16.6, 20.0 and 11.1 fold decrease in the protein expression of AR, PSMA and PSA respectively. These results were further confirmed by immunofluorescence analysis (data not shown).

Regarding mechanisms of immune evasion, it was found that Docetaxel resistant cells showed deregulation of MHC class I molecules. In this context, previous work from our group already reported the identification of MHC class I antigens negative tumour cell subpopulations in human primary and metastatic carcinomas (Cordon-Cardo, *et al.,* 1991). In the present study, it was found that MHC class I antigens, which are critical for efficient antigen presentation to cytotoxic T lymphocytes and subsequent tumour cell lysis, were down-regulated at both gene transcription and protein level (Figure 2). Gene expression profiling revealed a significant down-regulation in all MHC class I antigens (A, B, C, E, F, G), a fact that was confirmed at the protein level by immunoblotting and immunofluorescence staining of MHC class I antigens A, B, C (Figure 2). Moreover, Docetaxel resistant cells showed a down-regulation in gene transcript levels of known NK ligands, such as MICA/B, PVR, and PVRL2, as shown in Table 9. Thus, these cells exhibit a phenotype that favors immune evasion, making them undetectable by the host immune system.

Natural killer (NK) ligands gene expression that were deregulated in Docetaxel resistant cells (DU145-DR and 22RV1-DR) when compared to their parental sensitive cells (DU145 and 22RV1) are summarized in Table 9. Statistical analysis of the mean expression average difference of genes, which show ≥ 2 fold change based on a logarithmic normalization, was done using a t-test between matched sensitive and resistant cells. There is a decrease in the transcription levels of most NK ligands genes.

**TABLE 9**

| **Gene transcription levels of natural killer (NK) cell ligands.** | | |
|---|---|---|
| Gene name | x-fold change DU145/DR; 22RV1/DR | p value |
| MHC class I polypeptide-related sequence A | -1.4; -1.7 | NS;NS |
| MHC class I polypeptide-related sequence A///B | -2.2 ; -1.8 | <0.05;NS |
| MHC class I polypeptide-related sequence B | -2.2 ; -1.5 | <0.05;NS |
| Poliovirus receptor | -4.1; -2.1 | <0.0001;<0.05 |
| poliovirus receptor-related 2 (herpesvirus entry mediator B) | -2.5;-1.7 | <0.05;NS |

| | | |
|---|---|---|
| Note: NS=Not Significant | | |

Regarding studies on stem cell phenotype, the identified deregulated expression of WNT/β-catenin was characterized, Notch and Hedgehog, which have been implicated in self-renewal and differentiation of progenitor cells (Katoh *et al.,* 2007; McDonald *et al.,* 2006; van den Brink *et al.,* 2004; Radtke *et al.,* 2006; Leong *et al.,* 2008; and Grigoryan *et al.,* 2008). In the prostate, these signaling pathways have been reported to play essential roles in developmental patterning, epithelial regeneration, and prostate cancer tumourigenesis (Wang *et al.,* 2006; Karhadkar *et al.,* 2004). Docetaxel resistant cells showed a significant decrease in both gene transcript and protein levels of Dickkopf-1 (DKK1), a well known inhibitor of the WNT/β-catenin signaling network. This decrease in DKK1 expression was linked to an increase in the expression of de-phosphorylated (active) β-catenin, which is the major key effector of WNT signaling. Immunofluorescence analyses demonstrated that parental Docetaxel sensitive cells displayed a membranous expression of β-catenin, associated with its function as an adhesion molecule, whereas Docetaxel resistant cells showed a pronounced nuclear localization of this protein (Figure 2), reported as necessary for the activation of the canonical WNT signaling pathway. Moreover, Docetaxel resistant cells also exhibited an increase in the NOTCH signaling network. NOTCH2 gene transcript levels were significantly increased in the resistant cells and were linked to an increase in cleaved Notch2 protein expression that was associated with nuclear translocation of the protein, where it exerts its transcriptional activity (Figure 2). Finally, Docetaxel resistant cells had an increased expression of the Hedgehog receptor Patched and the glioma associated oncogene homolog transcription factors, Gli1 and Gli2. These findings were associated with an increased protein expression and nuclear translocation of the above mentioned transcription factors (Figure 2), a condition that has been related to Hedgehog pathway activation. Surprisingly, other reported stem cell surface markers, such as CD44 and CD133, were not found to be up-regulated in the model, as analyzed both at the gene transcript and protein levels (data not shown).

Furthermore, it was observed that the reversibility of the resistant phenomenon for both DU145-DR and 22RV1-DR cells was linked to an increase in the expression of differentiation markers. Docetaxel reversed resistant cells (cultured without the drug during 12 weeks) showed higher protein expression levels of low molecular weight cytokeratins (CK19 and CK18) and HLA-class I when compared to Docetaxel acquired resistant cells, achieving levels similar to those observed in parental sensitive cells (Figure 13).

### Generation and characterization of an epithelial differentiation reporter model

DU145 parental cells were stably transfected with a plasmid containing the promoter of CK19 driving the expression of the green fluorescence protein (GFP) (Figure 14a). Co-expression of CK19 and GFP in DU145-CK19 promoter-GFP stable cells was confirmed by immunofluorescence (Figure 14a). Flow cytometry quantification showed two distinct populations of cells, being the majority of cells positive for both GFP and CK19 (94.3±3.8%) and a discrete population of cells negative for both markers (5.6±4.1%). Few scattered cells outside these two main populations were observed which could represent transiting cells from one compartment to the other. Furthermore, stable insertion of the promoter construct in CK19/GFP negative cells was confirmed by PCR (Figure 4c). The expression of HLA class I in DU145-CK19 promoter-GFP stable cells was further characterized (Figure 14b). Not surprisingly, cells that expressed GFP were also HLA-positive (91.6±5.5%) and cells that did not express GFP displayed an HLA-negative phenotype (7.0±4.95). Thus these results validate the use of GFP as a reporter of epithelial differentiation and further demonstrate, as shown previously in Figure 4b, the existence of a subpopulation of cells that lack differentiation markers (CK19/GFP) and HLA class I antigens.

### Tumour initiation studies on HLA class I sorted cell lines

In order to further confirm that HLA-class I expression can be used as a cell surface marker that identifies cells with the cancer stem cell functional property of tumour initiation, parental cell lines DU145 and 22RV1 were sorted for HLA-class I and their tumour initiating capacity tested in NOD/SCID mice (Figure 15). Similar to the results obtained with the DU145-CK19 promoter-GFP stable GFP-negative cells, only the HLA class I-negative cells exhibited tumour initiating capacity after dilution assays. Injection of 10 HLA class I-negative DU145 and 22RV1 cells produced tumours in 83.3±19.1% and 100% of recipients, respectively, while no tumour formation was observed after 198 days of injection with 10 cells displaying a HLA class I-positive phenotype. Of note, tumour initiating capacity and tumours latencies of HLA class I-negative cells from 22RV1 and DU145 were different, although these differences did not reach statistical significance. The differences between tumourigenic cell lines could be explained by the fact that other molecular pathways may play a role in the engraftment and growth of human cells in mice. Similar results were obtained after serial transplantation from HLA class I-negative generated tumour xenografts (data not shown).

### Clonability studies on HLA class I sorted cell lines

In order to address the clonability capacity of HLA class I sorted cells from DU145 and 22RV1 parental cell lines, dilution colony formation assays were performed. HLA- class I-negative cells displayed a statistically significant higher clonability than HLA class I-positive cells. Specifically, HLA class I-negative sorted cells from DU145 generated colonies in 31.6±7.5%, 17.1±3.6% and 22.0±4.9% when 10, 100 and 1000 cells were plated, respectively. In contrast, HLA class I-positive cells generated colonies in 5.0±8.3%, 8.0±3.3% and 5.5±1.5% when 10, 100 and 1000 cells were plated (Figure 8). Similar results were observed with 22RV1 HLA class I sorted parental cells (data not shown).

### Identification of prostate cancer stem cells in human tissues

Immunohistochemical studies of metastases (n=20) and matched primary (n=6) human prostate cancer tissues revealed that all specimens contained scattered subpopulations of CK-negative (CK18 and CK19) tumour cells, accounting for 0.05% to 0.3% and 0.4% to 1.8% of all tumour cells in primary and metastatic lesions, respectively (Figure 7a). Immunofluorescence-based double staining was then performed to assess the association between CK expression and the markers of interest. In this analysis, it was consistently observed that CK expression was significantly associated with HLA class I expression (p<0.0001). More specifically, it was observed that the CK-negative tumour population did not express HLA class I antigens in 97.8 ± 0.7% of the cells, whereas all (100%) of the CK-positive cells displayed a positive HLA class I antigen phenotype (Figure 4b). A small population of CK-negative cells that displayed a HLA class I-positive phenotype was identified, which could represent tumour cells that undergo transition from an HLA class I-negative/CK-negative phenotype to a differentiated phenotype. Furthermore, it was consistently found that CK-negative/HLA-negative tumour cells had a significant (p<0.0001) increase of nuclear expression (activation) of developmental transcription factors when compared to differentiated CK-positive/HLA-positive cells. CK-negative/HLA-negative cells displayed nuclear expression of de-phosphorylated β-catenin in 63.9 ± 22.6% of cells, cleaved Notch2 in 72.8 ± 15.1%, Gli1 in 67.5 ± 17.3%, and Gli2 in 67 ± 17.3%, whereas CK-positive/HLA-positive cells expressed nuclear de-phosphorylated β-catenin in only 5.8 ± 11.9% of cells, cleaved Notch2 in 6.7 ± 7.9%, Gli1 in 1.2 ± 7.9%, and Gli2 in 1.5 ± 10.6% (Figure 7c). Moreover, it was also observed that the CK-negative/HLA class I-negative tumour cells showed no expression of nuclear AR, whereas CK-positive/HLA-positive cells displayed nuclear AR in 71.8 ± 14.3% of the cells (Figure 7d). Thus the fact that prostate cancer.stem cells do not display a positive AR phenotype suggests that these cells may not be dependent on a functional AR signaling, which would explain how these cells might be responsible for the observed relapse after hormone-therapy. Taken together, these results confirm their existence and the ability to identify a subpopulation of prostate cancer stem cells in human prostate cancer tissue samples.

### Statistical analyses

Experimental data is expressed as means ± SD. Statistical analysis by Student's t-test was performed. Values were considered statistically significant at p<_ 0.05.

### EXAMPLE 6

### IN VITRO HIGH THROUGHPUT ASSAY

Based on the functional chemoresistance and phenotipical characteristics of the CSCs disclosed above, an *in vitro* high throughput assay was designed to screen compounds targeting this CSC population, and to determine whether CSCs are either selectively killed or differentiated. Implementation of this assay permits testing of a wide variety of drugs and facilitates the identification of compounds with a putative CSC inhibitory effect.

The *in vitro* high throughput assay consists of the seeding of the above mentioned CSCs into 96 well plates. Cells are then exposed to the candidate agents, e.g., drugs, of interest, and readouts are obtained at desired time points (*e.g*., 24 hours, 72 hours, etc). Readouts consist in comparing the percentage of cells that express the CSC phenotype (*e.g.*, lack of HLA, CD24, etc) in treated versus untreated populations by flow cytometry analysis. In parallel, cell viability assays, which compare the percentage of viable ("surviving") cells between treated and untreated cells, are also performed. Results are interpreted as follows: candidate agents, e.g., drugs, that target the CSC population should inhibit the growth or kill chemoresistant cells which lack CSCs. Four possible results may be obtained with this assay: outcome 1, a decrease in the percentage of CSCs with a decrease in cell viability; outcome 2, a decrease in CSC without a decrease in cell viability; outcome 3, an increase in CSC populations with a decrease in cell viability; and outcome 4, no decrease in either the percentage of CSC nor in cell viability. Outcome 1 may be interpreted that the CSC population has been targeted by the candidate agent, *e.g.,* drug. Outcome 2 may be interpreted that the CSC population has been differentiated. Outcome 3 may be interpreted that neoplastic cells, but not the CSC population, has been targeted by the candidate agent, *e.g.,* drug. Outcome 4 may be interpreted that no effect is produced by the candidate agent, *e.g.,* drug, in either CSC or neoplastic differentiated cells.

Candidate agents, *e.g.,* drugs that produce CSC depletion (outcome 1) or CSC differentiation (outcome 2) would be considered for further experimentation and characterization by means of standard *in vitro* and *in vivo* assays. Briefly, these assays aimed at confirming and validating early screening results, include: a) *in vitro -* apoptotic methods (e.g., annexin V), colony formation, and differentiation studies based on their correponding phenotypes (e.g., flow cytometry and immunohistochemical analyses); and b) *in vivo -* dilutional tumor initiation assays in immunocompromised mice.

This high throughput screen is validated using the Notch and Hedgehog inhibitors disclosed above.

Although illustrative embodiments of the present invention have been described herein, it should be understood that the invention is not limited to those described.

### Cited Documents

1. Al-Hajj, M., et al., Prospective identification of tumorigenic breast cancer cells. Proc Natl Acad Sci USA 100, 3983-3988 (2003).
2. Ali, T. Z. et al., False positive labeling of prostate cancer with high molecular weight cytokeratin: p63 a more specific immunomarker for basal cells. Am J Surg Pathol 32, 1890-1895 (2008).
3. Alix-Panabieres, C. et al., Full-length cytokeratin-19 is released by human tumor cells: a potential role in metastatic progression of breast cancer. Breast Cancer Res. 11 (3):R39 (2009).
4. Alison, M. R. et al., Number crunching in the cancer stem cell market. Breast Cancer Res. 11(2):302 (2009).
5. Bao, S. et al., Glioma stem cells promote radioresistance by preferential activation of the DNA damage response. Nature 444, 756-760 (2006).
6. Brulet, P. et al., Monoclonal antibodies against trophectoderm-specific markers during mouse blastocyst formation. Proc Natl Acad Sci USA 77, 4113-4117 (1980).
7. Cariati, M. et al., Alpha-6 integrin is necessary for the tumorigenicity of a stem cell-like subpopulation within the MCF7 breast cancer cell line. Int J Cancer 122, 298-304 (2008).
8. Chen, J. K. et al., Inhibition of Hedgehog signaling by direct binding of cyclopamine to Smoothened. Genes Dev 16, 2743-2748, (2002).
9. Chen, R. et al., A hierarchy of self-renewing tumor-initiating cell types in glioblastoma. Cancer Cell 17, 362-375 (2009).
10. Collins, A. T. et al., Prospective identification of tumorigenic prostate cancer stem cells. Cancer Res 65, 10946-10951 (2005).
11. Cordon-Cardo, C. et al., Expression of HLA-A,B,C antigens on primary and metastatic tumor cell populations of human carcinomas. Cancer Res 51, 6372-6380 (1991).
12. Costello, R. T. et al., Human acute myeloid leukemia CD34+/CD38- progenitor cells have decreased sensitivity to chemotherapy and Fas-induced apoptosis, reduced immunogenicity, and impaired dendritic cell transformation capacities. Cancer Res 60, 4403-4411 (2000).
13. Dalerba, P. et al., Cancer stem cells: models and concepts. Annu Rev Med 58, 267-284 (2007).
14. Dean, M. et al., Tumor stem cells and drug resistance. Nat Rev Cancer 5, 275-284 (2005).
15. Desoye, G. et al., Lack of HLA class I and class II antigens on human preimplantation embryos. J Immunol 140, 4157-4159 (1988).
16. Fedi, P. et al., Isolation and biochemical characterization of the human Dkk-I homologue, a novel inhibitor of mammalian Wnt signaling. J Biol Chem 274, 19465-19472 (1999).
17. Fillmore, C. M. et al., Human breast cancer cell lines contain stem-like cells that self-renew, give rise to phenotypically diverse progeny and survive chemotherapy. Breast Cancer Res 10, R25 (2008).
18. Grigoryan, T. et al., Deciphering the function of canonical Wnt signals in development and disease: conditional loss- and gain-of-function mutations of beta-catenin in mice. Genes Dev 22, 2308-2341 (2008).
19. Gunaratne, P. H., Embryonic stem cell microRNAs: defining factors in induced pluripotent (iPS) and cancer (CSC) stem cells? Curr. Stem Cell Res. Ther. 4(3):168-177 (2009).
20. Gupta, P. B. et al., Identification of selective inhibitors of cancer stem cells by high-throughput screening. Cell 138, 645-659 (2009).
21. Guzman, M. L. et al., Preferential induction of apoptosis for primary human leukemic stem cells. Proc Natl Acad Sci USA 99,16220-16225 (2002).
22. Jordan, C. T. et al., Cancer stem cells. N Engl J Med 355, 1253-1261 (2006).
23. Karhadkar, S. S. et al., Hedgehog signalling in prostate regeneration, neoplasia and metastasis. Nature 431, 707-712 (2004).
24. Katoh, M., Networking of WNT, FGF, Notch, BMP, and Hedgehog signaling pathways during carcinogenesis. Stem Cell Rev 3, 30-38 (2007).
25. Leong, K. G. et al., The Notch pathway in prostate development and cancer. Differentiation 76, 699-716 (2008).
26. Li, H. et al., PC3 human prostate carcinoma cell holoclones contain self-renewing tumor-initiating cells. Cancer Res 68, 1820-1825 (2008).
27. Lu, H. et al., Type II keratins precede type I keratins during early embryonic development. Eur J Cell Biol 84, 709-718 (2005).
28. McDonald, S. A. et al., Clonal expansion in the human gut: mitochondrial DNA mutations show us the way. Cell Cycle 5, 808-811 (2006).
29. Nishii, T. et al., Cancer stem cell-like SP cells have a high adhesion ability to the peritoneum in gastric carcinoma. Cancer Sci. 100(8):1397-1402 (2009).
30. O'Brien, C. A. et al., A human colon cancer cell capable of initiating tumor growth in immunodeficient mice. Nature 445, 106-110 (2007).
31. Oshima, R. G., Identification and immunoprecipitation of cytoskeletal proteins from murine extra-embryonic endodermal cells. J Biol Chern 256, 8124-8133 (1981).
32. Patrawala, L. et al., Highly purified CD44+ prostate cancer cells from xenograft human tumors are enriched in tumorigenic and metastatic progenitor cells. Oncogene 25, 1696-1708 (2006).
33. Patrawala, L. et al., Hierarchical organization of prostate cancer cells in xenograft tumors: the CD44+alpha2beta1+ cell population is enriched in tumor-initiating cells. Cancer Res 67, 6796-6805 (2007).
34. Petersen, O. W., et al., Epithelial progenitor cell lines as models of normal breast morphogenesis and neoplasia. Cell Prolif. 36 Suppl. 1:33-44 (2003).
35. Petrylak, D. P. et al., Docetaxel and estramustine compared with mitoxantrone and prednisone for advanced refractory prostate cancer. N Engl J Med 351, 1513-1520 (2004).
36. Petrylak, D. P. et al., P-glycoprotein expression in primary and metastatic transitional cell carcinoma of the bladder. Ann Oncol 5, 835-840 (1994).
37. Ponti, D. et al., Isolation and in vitro propagation of tumorigenic breast cancer cells with stem/progenitor cell properties. Cancer Res 65, 5506-5511 (2005).
38. Quintana, E. et al., Efficient tumor formation by single human melanoma cells. Nature 456, 593-598 (2008).
39. Radtke, F. et al., From gut homeostasis to cancer. Curr Mol Med 6, 275-289 (2006).
40. Rahman, R. et al., Cellular immortality in brain tumours: an integration of the cancer stem cell paradigm. 1792(4):280-288 (2009).
41. Real, P. J. et al., Gamma-secretase inhibitors reverse glucocorticoid resistance in T cell acute lymphoblastic leukemia. Nat Med 15, 50-58, (2009).
42. Reya, T. et al., Stem cells, cancer, and cancer stem cells. Nature 414, 105-111 (2001).
43. Ricci-Vitiani, L. et al., Identification and expansion of human colon-cancer-initiating cells. Nature 445, 111-115 (2007).
44. Ricci-Vitiani, L. et al., Colon cancer stem cells. J. Mol. Med. 87(11):1097-1104 (2009).
45. Rosen, J. M. et al., The increasing complexity of the cancer stem cell paradigm. Science 324, 1670-1673 (2009).
46. Sanchez-Carbayo, M. et al., Expression profiling of osteosarcoma cells transfected with MDR1 and NEO genes: regulation of cell adhesion, apoptosis, and tumor suppression-related genes. Lab Invest 83, 507-517 (2003).
47. Seiffert, D. et al., Presenilin-1 and -2 are molecular targets for gamma-secretase inhibitors. J Biol Chem 275, 34086-34091, (2000).
48. Sharma, S. V. et al., A chromatin-mediated reversible drug-tolerant state in cancer cell subpopulations. Cell 141, 69-80 (2010)
49. Shmelkov, S. V. et al., CD133 expression is not restricted to stem cells, and both CD133+ and CD133- metastatic colon cancer cells initiate tumors. J Clin Invest 118, 2111-2120 (2008).
50. Singh, S. K. et al., Identification of human brain tumor initiating cells. Nature 432, 396-401 (2004).
51. Takaishi, S. et al., Identification of gastric cancer stem cells using the cell surface marker CD44. Stem Cells 27(5):1006-1020 (2009).
52. Taipale, J. et al., Effects of oncogenic mutations in Smoothened and Patched can be reversed by cyclopamine. Nature 406, 1005-1009, (2000).
53. Tannock, I. F. et al., Docetaxel plus prednisone or mitoxantrone plus prednisone for advanced prostate cancer. N Engi J Med 351, 1502-1512 (2004).
54. Tesar, P. J. et al., New cell lines from mouse epiblast share defining features with human embryonic stem cells. Nature 448, 196-199 (2007).
55. van den Brink, G. R. et al., Indian Hedgehog is an antagonist of Wnt signaling in colonic epithelial cell differentiation. Nat Genet 36,277-282 (2004).
56. Tripathi, M. K. et al., Negative regulation of the expressions of cytokeratins 8 and 19 by SLUG repressor protein in human breast cells. Biochem Biophys Res Commun 329, 508-515 (2005)
57. Trumpp, A. et al., Mechanisms of Disease: cancer stem cells--targeting the evil twin. Nat Clin Pract Oncol 5, 337-347 (2008).
58. van Es, J. H. et al., Notch/gamma-secretase inhibition turns proliferative cells in intestinal crypts and adenomas into goblet cells. Nature 435, 959-963, (2005).
59. Vermeulen, L. et al., Cancer stem cells--old concepts, new insights. Cell Death Differ 15, 947-958 (2008).
60. Wang, X. D. et al., Notch signaling is required for normal prostatic epithelial cell proliferation and differentiation. Dev Biol 290, 66-80 (2006).
61. Willert, K. et al., Wnt signaling: is the party in the nucleus? Genes Dev 20, 1394-1404 (2006).
62. Wu, M. et al., Imaging hematopoietic precursor division in real time. Cell Stem Cell (2007*).*

## Claims

1. A method for identifying an agent that selectively decreases the number of cancer stem cells (CSCs) comprising:
a. contacting a CSC from a population of cells with a candidate agent; and b. determining whether the candidate agent reduces the survival or growth of the CSC or increases differentiation of the CSC relative to a CSC that has not been contacted with the candidate agent; wherein the CSC is a cancer cell that is HLA⁻.

2. The method according to claim 1 , wherein the CSC further has at least one of the following properties: CD24⁻, CD133⁻, Notch⁺, Gli1⁺, Gli2⁺, cytokeratin-, Neurofil⁻, and Glial fibrillary acidic protein⁻ (GFAP-).

3. The method according to any one of claims 1 or 2, wherein the CSC further has at least one of the following properties: capability to self-renew, undergoes asymmetrical cell division, has tumorigenic capacity, has metastatic potential, has multi-differentiation properties, broad chemoresistance, and sensitivity to Notch and Hedgehog inhibitors.

4. The method according to claim 1 , wherein the CSCs are refractory to standard chemotherapy.

5. The method according to claim 1 , wherein the candidate agent is selected from the group consisting of a chemical, a biologic, and combinations thereof.

6. The method according to claim 1, wherein the determining step further comprises comparing the percentage of CSCs in a population of cells which is treated with the candidate agent to the percentage of CSCs in a population of cells which is not treated with the candidate agent, wherein a candidate agent that decreases the percentage of CSCs in the population of treated cells as compared to the percentage of CSCs in the untreated cells is an agent that selectively decreases the number of CSCs.

7. The method according to claim 1 , wherein the determining step comprises carrying out a procedure selected from the group consisting of flow cytometry analysis, a cell viability assay, a cell differentiation assay, a cell division assay, and combinations thereof.

8. The method according to claim 1 , wherein the population of cells comprises CSCs and adult stem cells and the agent decreases the number of CSCs in the population of cells without substantially decreasing the number of adult stem cells.

9. The method according to claim 1 , which is a high throughput screen.

10. The method according to claim 1 , wherein the determining step further comprises step i and at least one of steps ii, iii, and iv: i. comparing the percentage of CSCs in a population of cells, which is treated with the candidate agent to the percentage of CSCs in a population of cells, which is not treated with the candidate agent;
ii. determining the percentage of viable cells in the treated and untreated population of cells,
iii. determining the percentage of non-CSC cells in the treated and untreated population of cells; and
iv. determining the percentage of dividing cells in the treated and untreated population of cells,
wherein a candidate agent that (1) decreases the percentage of CSCs and decreases cell viability; (2) decreases the percentage of CSCs without a decrease in cell viability; (3) decreases the percentage of CSCs and increases the percentage of non-CSCs; (4) decreases the percentage of CSCs and decreases cell division; or (5) decreases the percentage of CSCs without a decrease in cell division is an agent that selectively decreases the number of CSCs.

11. The method according to claim 10, wherein the determining step comprises carrying out a procedure selected from the group consisting of flow cytometry analysis, a cell viability assay, a cell differentiation assay, a cell division assay, and combinations thereof.

12. A high throughput screening method for identifying agents that selectively decrease the percentage of cancer stem cells (CSCs) in a population of cells comprising:
a. contacting a CSC from a population of cells with a candidate agent;
b. determining whether the candidate agent reduces the survival or growth or increases the differentiation of the CSC relative to a CSC that has not been contacted with the candidate agent by:
i. comparing the percentage of CSCs in a population of cells which is treated with the candidate agent to the percentage of CSCs in a population of cells which is not treated with the candidate agent;
ii. determining the percentage of viable cells in the treated and untreated population of cells;
iii. determining the percentage of non-CSC cells in the treated and untreated population of cells; and
iv. determining the percentage of dividing cells in the treated and untreated population of cells;
wherein a candidate agent that (1) decreases the percentage of CSCs and decreases cell viability; (2) decreases the percentage of CSCs without a decrease in cell viability; (3) decreases the percentage of CSCs and increases the percentage of non-CSCs; (4) decreases the percentage of CSCs and decreases cell division; or (5) decreases the percentage of CSCs without a decrease in cell division is an agent that selectively decreases the percentage of CSCs; wherein the CSC is a cancer cell that is HLA⁻.

13. The method according to claim 12, wherein the CSC further has at least one of the following properties: CD24⁻, CD133⁻, Notch⁺, Gli1⁺, Gli2⁺, cytokeratin⁻ , Neurofil⁻, and GFAP⁻.

14. The method according to any one of claims 12 or 13, wherein the CSC further has at least one of the following properties: capability to self-renew, undergoes asymmetrical cell division, has tumorigenic capacity, has metastatic potential, has multi-differentiation properties, broad chemoresistance, and sensitivity to Notch and Hedgehog inhibitors.

15. The method according to claim 12, wherein step (i) is carried out by flow cytometry analysis.

16. The method according to claim 12, wherein step (ii) is carried out by a cell viability assay.

17. The method according to claim 12, wherein step (iii) is carried out by a cell differentiation assay.

18. The method according to claim 12, wherein step (iv) is carried out by a cell division assay.

19. The method according to claim 12, wherein the candidate agent is selected from the group consisting of a chemical, a biologic, and combinations thereof.

20. The method according to claim 12, wherein the CSCs are refractory to standard chemotherapy.

21. A high throughput screening method for identifying agents that selectively decrease the percentage of cancer stem cells (CSCs) in a population of cells, wherein the CSCs are refractory to standard chemotherapy, comprising:
a. contacting a CSC from a population of cells with a candidate agent, wherein the CSC has the following properties: HLA Γ and HLA II⁻;
b. determining whether the candidate agent reduces the survival or growth or increases differentiation of the CSC relative to a CSC that has not been contacted with the candidate agent by:
i. comparing, using flow cytometry, the percentage of CSCs in the population of cells, which is treated with the candidate agent to the percentage of CSCs in a population of cells, which is not treated with the candidate agent;
ii. determining, using a cell viability assay, the percentage of viable cells in the treated and untreated population of cells;
iii. determining, using a cell differentiation assay, the percentage of non-CSC cells in the treated and untreated population of cells; and
IV. determining, using a cell division assay, the percentage of dividing cells in the treated and untreated population of cells;
wherein a candidate agent that (1) decreases the percentage of CSCs and decreases cell viability; (2) decreases the percentage of CSCs without a decrease in cell viability; (3) decreases the percentage of CSCs and increases the percentage of non-CSCs; (4) decreases the percentage of CSCs and decreases cell division; or (5) decreases the percentage of CSCs without a decrease in cell division is an agent that selectively decreases the percentage of CSCs.

22. The method according to claim 21 , wherein the candidate agent is selected from the group consisting of a chemical, a biologic, and combinations thereof.

## Patentansprüche

1. Verfahren zur Identifizierung eines Mittels, das selektiv die Zahl von Krebsstammzellen (cancer stem cells, CSCs) verringert, umfassend:
a. In-Kontakt-Bringen einer CSC aus einer Zellpopulation mit einem Kandidatenmittel und b. Bestimmen, ob das Kandidatenmittel das Überleben oder das Wachstum der CSC verringert oder eine Differenzierung der CSC erhöht, und zwar im Vergleich zu einer CSC, die nicht mit dem Kandidatenmittel in Kontakt gebracht wurde; wobei die CSC eine Krebszelle ist, die HLA- ist.

2. Verfahren nach Anspruch 1, wobei die CSC außerdem wenigstens eine der folgenden Eigenschaften hat: CD24-, CD133⁻, Notch⁺, Gli1⁺, Gli2⁺, Zyto-keratin⁻, Neurofil- und saures Gliagaserprotein⁻ (Glial fibrillary acidic protein-(GFAP-)).

3. Verfahren nach einem der Ansprüche 1 oder 2, wobei die CSC außerdem wenigstens eine der folgenden Eigenschaften hat: Fähigkeit zur Selbsterneuerung, macht eine asymmetrische Zellteilung durch, hat tumorigene Kapazität, hat metastatisches Potential, hat Multidifferenzierungseigenschaften, eine breite Chemoresistenz und Sensitivität gegenüber Notch- und Hedgehog-Inhibitoren.

4. Verfahren nach Anspruch 1, wobei die CSCs gegenüber Standard-Chemotherapie refraktär sind.

5. Verfahren nach Anspruch 1, wobei das Kandidatenmittel aus der Gruppe, bestehend aus einer Chemikalie, einem Biologikum und Kombinationen davon, ausgewählt wird.

6. Verfahren nach Anspruch 1, wobei der Bestimmungsschritt außerdem Vergleichen des Prozentgehalts von CSCs in einer Zellpopulation, die mit dem Kandidatenmittel behandelt ist, zu dem Prozentgehalt von CSCs in einer Zellpopulation, die nicht mit dem Kandidatenmittel behandelt ist, umfasst, wobei ein Kandidatenmittel, das den Prozentgehalt von CSCs in der Population behandelter Zellen im Vergleich zu dem Prozentgehalt von CSCs in den unbehandelten Zellen verringert, ein Mittel ist, das die Zahl von CSCs selektiv verringert.

7. Verfahren nach Anspruch 1, wobei der Bestimmungsschritt Durchführen eines Verfahrens, ausgewählt aus der Gruppe, bestehend aus Durchflusszytometrieanalyse, einem Assay auf Lebensfähigkeit, einem Zelldifferenzierungsassay, einem Zellteilungsassay und Kombinationen davon, umfasst.

8. Verfahren nach Anspruch 1, wobei die Zellpopulation CSCs und adulte Stammzellen umfasst und das Mittel die Zahl der CSCs in der Zellpopulation verringert, ohne die Zahl adulter Stammzellen wesentlich zu verringern.

9. Verfahren nach Anspruch 1, das ein Screen mit hohem Durchsatz ist.

10. Verfahren nach Anspruch 1, wobei der Bestimmungsschritt außerdem Schritt i und wenigstens einen der Schritte ii, iii und iv umfasst:
i. Vergleichen des Prozentgehalts von CSCs in einer Zellpopulation, die mit dem Kandidatenmittel behandelt ist, mit dem Prozentgehalt von CSCs in einer Zellpopulation, die nicht mit dem Kandidatenmittel behandelt ist;
ii. Bestimmen des Prozentgehalts von lebensfähigen Zellen in der behandelten und unbehandelten Zellpopulation,
iii. Bestimmen des Prozentgehalts von Nicht-CSC-Zellen in der behandelten und unbehandelten Zellpopulation und
iv. Bestimmen des Prozentgehalts von sich teilenden Zellen in der behandelten und unbehandelten Zellpopulation,
wobei ein Kandidatenmittel, das (1) den Prozentgehalt von CSCs verringert und die Zelllebensfähigkeit verringert; (2) den Prozentgehalt von CSCs ohne eine Verringerung der Zelllebensfähigkeit verringert; (3) den Prozentgehalt von CSCs verringert und den Prozentgehalt von Nicht-CSCs erhöht; (4) den Prozentgehalt von CSCs verringert und die Zellteilung verringert oder (5) den Prozentgehalt von CSCs verringert ohne eine Verringerung der Zellteilung, ein Mittel ist, das die Zahl von CSCs selektiv verringert.

11. Verfahren nach Anspruch 10, wobei der Bestimmungsschritt Durchführen eines Verfahrens, ausgewählt aus der Gruppe, bestehend aus einer Durchflusszytometrieanalyse, einem Assay auf Zelllebensfähigkeit, einem Zelldifferenzierungsassay, einem Zellteilungsassay und Kombinationen davon, umfasst.

12. Hochdurchsatz-Screeningverfahren zur Identifizierung von Mitteln, die selektiv den Prozentgehalt von Krebsstammzellen (CSCs) in einer Zellpopulation verringern, umfassend:
a. In-Kontakt-Bringen einer CSC aus einer Zellpopulation mit einem Kandidatenmittel;
b. Bestimmen, ob das Kandidatenmittel das Überleben oder das Wachstum der CSC reduziert oder die Differenzierung der CSC erhöht, und zwar im Vergleich zu einer CSC, die nicht mit dem Kandidatenmittel in Kontakt gebracht wurde, durch:
i. Vergleichen des Prozentgehalts von CSCs in einer Zellpopulation, die mit dem Kandidatenmittel behandelt ist, mit dem Prozentgehalt von CSCs in einer Zellpopulation, die nicht mit dem Kandidatenmittel behandelt ist;
ii. Bestimmen des Prozentgehalts von lebensfähigen Zellen in der behandelten und unbehandelten Zellpopulation;
iii. Bestimmen des Prozentgehalts von Nicht-CSC-Zellen in der behandelten und unbehandelten Zellpopulation und
iv. Bestimmen des Prozentgehalts von sich teilenden Zellen in der behandelten und unbehandelten Zellpopulation;
wobei ein Kandidatenmittel, das (1) den Prozentgehalt von CSCs verringert und die Zelllebensfähigkeit verringert; (2) den Prozentgehalt von CSCs ohne eine Verringerung der Zelllebensfähigkeit verringert; (3) den Prozentgehalt von CSCs verringert und den Prozentgehalt von Nicht-CSCs erhöht; (4) den Prozentgehalt von CSCs verringert und die Zellteilung verringert oder (5) den Prozentgehalt von CSCs verringert ohne eine Verringerung der Zellteilung, ein Mittel ist, das die Zahl von CSCs selektiv verringert; wobei die CSC eine Krebszelle ist, welche HLA⁻ ist.

13. Verfahren nach Anspruch 12, wobei die CSC außerdem wenigstens eine der folgenden Eigenschaften hat: CD24-, CD133⁻, Notch⁺, Gli1⁺, Gli2⁺, Zyto-keratin⁻, Neurofil⁻ und GFAP⁻.

14. Verfahren nach einem der Ansprüche 12 oder 13, wobei die CSC außerdem wenigstens eine der folgenden Eigenschaften hat: Fähigkeit zur Selbsterneuerung, macht eine asymmetrische Zellteilung durch, hat tumorigene Kapazität, hat metastatisches Potential, hat Multidifferenzierungseigenschaften, eine breite Chemoresistenz und Sensitivität gegenüber Notch- und Hedgehog-Inhibitoren.

15. Verfahren nach Anspruch 12, wobei Schritt (i) durch Durchflusszytometrie-analyse durchgeführt wird.

16. Verfahren nach Anspruch 12, wobei Schritt (ii) durch einen Assay auf Zelllebensfähigkeit durchgeführt wird.

17. Verfahren nach Anspruch 12, wobei Schritt (iii) durch einen Zelldifferenzierungsassay durchgeführt wird.

18. Verfahren nach Anspruch 12, wobei Schritt (iv) durch einen Zellteilungsassay durchgeführt wird.

19. Verfahren nach Anspruch 12, wobei das Kandidatenmittel aus der Gruppe, bestehend aus einer Chemikalie, einem Biologikum und Kombinationen davon, ausgewählt wird.

20. Verfahren nach Anspruch 12, wobei die CSCs gegenüber einer Standard-Chemotherapie refraktär sind.

21. Hochdurchsatz-Screeningverfahren zur Identifizierung von Mitteln, die den Prozentgehalt von Krebsstammzellen (CSCs) in einer Zellpopulation verringern, wobei die CSCs gegenüber einer Standard-Chemotherapie refraktär sind, umfassend:
a. In-Kontakt-Bringen einer CSC aus einer Zellpopulation mit einem Kandidatenmittel, wobei die CSC die folgenden Eigenschaften hat: HLA I⁻ und HLA II⁻;
b. Bestimmen, ob das Kandidatenmittel das Überleben oder das Wachstum der CSC reduziert oder die Differenzierung der CSC erhöht, und zwar relativ zu einer CSC, die nicht mit dem Kandidatenmittel in Kontakt gebracht wurde, durch:
i. Vergleichen, unter Verwendung von Durchflusszytometerie, den Prozentgehalt von CSCs in der Zellpopulation, die mit dem Kandidatenmittel behandelt ist, mit dem Prozentgehalt von CSCs in einer Zellpopulation, die nicht mit dem Kandidatenmittel behandelt ist;
ii. Bestimmen, unter Verwendung eines Assays auf Zelllebensfähigkeit, den Prozentgehalt von lebensfähigen Zellen in der behandelten und unbehandelten Zellpopulation;
iii. Bestimmen, unter Verwendung eines Zelldifferenzierungsassays, den Prozentgehalt von Nicht-CSC-Zellen in der behandelten und unbehandelten Zellpopulation und
iv. Bestimmen, unter Verwendung eines Zellteilungsassays, den Prozentgehalt von sich teilenden Zellen in der behandelten und unbehandelten Zellpopulation;
wobei das Kandidatenmittel, das (1) den Prozentgehalt von CSCs verringert und die Zelllebensfähigkeit verringert; (2) den Prozentgehalt von CSCs ohne eine Verringerung der Zelllebensfähigkeit verringert; (3) den Prozentgehalt von CSCs verringert und den Prozentgehalt von Nicht-CSCs erhöht; (4) den Prozentgehalt von CSCs verringert und die Zellteilung verringert oder (5) den Prozentgehalt von CSCs verringert ohne eine Verringerung der Zellteilung, ein Mittel ist, das die Zahl von CSCs selektiv verringert.

22. Verfahren nach Anspruch 21, wobei das Kandidatenmittel aus der Gruppe, bestehend aus einer Chemikalie, einem Biologikum und Kombinationen davon, ausgewählt wird.

## Revendications

1. Procédé pour identifier un agent qui diminue sélectivement le nombre de cellules souches cancéreuses (CSC), comprenant les étapes consistant à :
a. mettre en contact une CSC provenant d'une population de cellules avec un agent candidat ; et b. déterminer si l'agent candidat diminue la survie ou la croissance de la CSC, ou accroît la différenciation de la CSC par rapport à une CSC qui n'a pas été mise en contact avec l'agent candidat ; dans lequel la CSC est une cellule cancéreuse qui est HLA⁻.

2. Procédé selon la revendication 1, dans lequel la CSC possède en outre au moins l'une des propriétés suivantes : CD24⁻, CD133⁻, Notch⁺, Gli1⁺, Gli2⁺, cytokératine⁻, Neurofil⁻ et protéine acide fibrillaire gliale (GFAP⁻).

3. Procédé selon la revendication 1 ou 2, dans lequel la CSC possède en outre au moins l'une des propriétés suivantes : une capacité d'auto-renouvellement, subit une division cellulaire asymétrique, présente une capacité tumorigène, possède un potentiel métastatique, a des propriétés de multi-différenciation, une large chimiorésistance et sensibilité aux inhibiteurs Notch et Hedgehog.

4. Procédé selon la revendication 1, dans lequel les CSC sont réfractaires à une chimiothérapie standard.

5. Procédé selon la revendication 1, dans lequel l'agent candidat est choisi dans le groupe constitué par un produit chimique, un produit biologique, et des combinaisons de ceux-ci.

6. Procédé selon la revendication 1, dans lequel l'étape de détermination comprend en outre la comparaison du pourcentage de CSC dans une population de cellules qui est traitée à l'aide de l'agent candidat et du pourcentage de CSC dans une population de cellules qui ne sont pas traitées à l'aide de l'agent candidat, dans lequel un agent candidat qui diminue le pourcentage de CSC dans la population des cellules traitées par rapport au pourcentage de CSC dans les cellules non traitées est un agent qui diminue sélectivement le nombre de CSC.

7. Procédé selon la revendication 1, dans lequel l'étape de détermination comprend la réalisation d'une procédure choisie dans le groupe constitué par une analyse par cytométrie de flux, un dosage de viabilité cellulaire, un dosage de différenciation cellulaire, un dosage de division cellulaire, et des combinaisons de ceux-ci.

8. Procédé selon la revendication 1, dans lequel la population de cellules comprend des CSC et des cellules souches adultes, et l'agent diminue le nombre de CSC dans la population de cellules sensiblement sans diminuer le nombre de cellules souches adultes.

9. Procédé selon la revendication 1, qui est un crible à haut débit.

10. Procédé selon la revendication 1, dans lequel l'étape de détermination comprend en outre l'étape i et au moins l'une des étapes ii, iii et iv consistant à : i. comparer le pourcentage de CSC dans une population de cellules qui est traitée à l'aide de l'agent candidat et le pourcentage de CSC dans une population de cellules qui n'est pas traitée à l'aide de l'agent candidat ;
ii. déterminer le pourcentage de cellules viables dans les populations de cellules traitée et non traitée ;
iii. déterminer le pourcentage de cellules non-csc dans les populations de cellules traitée et non traitée ; et
iv. déterminer le pourcentage de cellules en division dans les populations de cellules traitée et non traitée,
dans lequel un agent candidat qui (1) diminue le pourcentage de CSC et diminue la viabilité des cellules ; (2) diminue le pourcentage de CSC sans une diminution de la viabilité des cellules ; (3) diminue le pourcentage de CSC et augmente le pourcentage de non-CSC ; (4) diminue le pourcentage de CSC et diminue la division cellulaire ; ou (5) diminue le pourcentage de CSC sans diminution de la division cellulaire est un agent qui diminue de manière sélective le nombre de CSC.

11. Procédé selon la revendication 10, dans lequel l'étape de détermination comprend la réalisation d'une procédure choisie dans le groupe constitué par une analyse par cytométrie de flux, un dosage de viabilité cellulaire, un dosage de différenciation cellulaire, un dosage de division cellulaire, et des combinaisons de ceux-ci.

12. Procédé de criblage à haut débit pour identifier des agents qui diminuent de manière sélective le pourcentage de cellules souches cancéreuses (CSC) dans une population de cellules, comprenant les étapes consistant à :
a. mettre en contact une CSC provenant d'une population de cellules avec un agent candidat ;
b. déterminer si l'agent candidat diminue la survie ou la croissance, ou augmente la différenciation de la CSC par rapport à une CSC qui n'a pas été mise en contact avec l'agent candidat, en :
i. comparant le pourcentage de CSC dans une population de cellules qui est traitée à l'aide de l'agent candidat et le pourcentage de CSC dans une population de cellules qui n'est pas traitée à l'aide de l'agent candidat ;
ii. déterminant le pourcentage de cellules viables dans les populations de cellules traitée et non traitée ;
iii. déterminer le pourcentage de cellules non-csc dans les populations de cellules traitée et non traitée ; et
iv. déterminer le pourcentage de cellules en division dans les populations de cellules traitée et non traitée ;
dans lequel un agent candidat qui (1) diminue le pourcentage de CSC et diminue la viabilité des cellules ; (2) diminue le pourcentage de CSC sans une diminution de la viabilité des cellules ; (3) diminue le pourcentage de CSC et augmente le pourcentage de non-CSC ; (4) diminue le pourcentage de CSC et diminue la division cellulaire ; ou (5) diminue le pourcentage de CSC sans diminution de la division cellulaire est un agent qui diminue de manière sélective le nombre de CSC ;
où la CSC est une cellule cancéreuse qui est HLA⁻.

13. Procédé selon la revendication 12, dans lequel la CSC possède en outre au moins l'une des propriétés suivantes : CD24⁻, CD133⁻, Notch⁺, Gli1⁺, Gli2⁺, cytokératine⁻, Neurofil⁻ et GFAP⁻.

14. Procédé selon l'une quelconque des revendications 12 ou 13, dans lequel la CSC possède en outre au moins l'une des propriétés suivantes : une capacité d'auto-renouvellement, subit une division cellulaire asymétrique, présente une capacité tumorigène, possède un potentiel métastatique, a des propriétés de multi-différenciation, une large chimiorésistance et sensibilité aux inhibiteurs Notch et Hedgehog.

15. Procédé selon la revendication 12, dans lequel l'étape (i) est effectuée par cytométrie de flux.

16. Procédé selon la revendication 12, dans lequel l'étape (ii) est effectuée par un dosage de viabilité cellulaire.

17. Procédé selon la revendication 12, dans lequel l'étape (iii) est effectuée par un dosage de différenciation cellulaire.

18. Procédé selon la revendication 12, dans lequel l'étape (iv) est effectuée par un dosage de division cellulaire.

19. Procédé selon la revendication 12, dans lequel l'agent candidat est choisi dans le groupe constitué par un produit chimique, un produit biologique, et des combinaisons de ceux-ci.

20. Procédé selon la revendication 12, dans lequel les CSC sont réfractaires à une chimiothérapie standard.

21. Procédé de criblage à haut débit pour identifier des agents qui diminuent de manière sélective le pourcentage de cellules souches cancéreuses (CSC) dans une population de cellules, où les CSC sont réfractaires à une chimiothérapie standard, comprenant les étapes consistant à :
a. mettre en contact une CSC provenant d'une population de cellules avec un agent candidat, la CSC ayant les propriétés suivantes : HLA I⁻ et HLA II⁻ ;
b. déterminer si l'agent candidat diminue la survie ou la croissance, ou augmente la différenciation de la CSC par rapport à une CSC qui n'a pas été mise en contact avec l'agent candidat, en :
i comparant, en utilisant une cytométrie de flux, le pourcentage de CSC dans la population de cellules qui est traitée à l'aide de l'agent candidat et le pourcentage de CSC dans une population de cellules qui n'est pas traitée à l'aide de l'agent candidat ;
ii déterminant, en utilisant un dosage de viabilité cellulaire, le pourcentage de cellules viables dans les populations de cellules traitée et non traitée ;
iii. déterminant, en utilisant un test de différenciation cellulaire, le pourcentage de cellules non-CSC dans les populations de cellules traitée et non traitée ; et
iv. déterminant, en utilisant un dosage de division cellulaire, le pourcentage de cellules en division dans les populations de cellules traitée et non traitée ;
dans lequel un agent candidat qui (1) diminue le pourcentage de CSC et diminue la viabilité cellulaire ; (2) diminue le pourcentage de CSC sans une diminution de la viabilité cellulaire ; (3) diminue le pourcentage de CSC et augmente le pourcentage de non-CSC ; (4) diminue le pourcentage de CSC et diminue la division cellulaire ; ou (5) diminue le pourcentage de CSC sans diminution de la division cellulaire est un agent qui diminue sélectivement le pourcentage de CSC.

22. Procédé selon la revendication 21, dans lequel l'agent candidat est choisi dans le groupe constitué par un produit chimique, un produit biologique, et des combinaisons de ceux-ci.


### SEQUENCE LISTING

<110> The Trustees of Columbia University in the City of New York
   Cordon-Cardo, Carlos
<120> ASSAY FOR SCREENING COMPOUNDS THAT SELECTIVELY DECREASE THE NUMBER OF CANCER STEM CELLS
<130> IC035795/0311192
<160> 4
<170> PatentIn version 3.3
<210> 1
   <211> 21
   <212> DNA
   <213> Artificial
<220>
   <223> synthetic construct; CK19 promoter forward primer
<400> 1
   aacgcatgct ttggggggat g 21
<210> 2
   <211> 22
   <212> DNA
   <213> Artificial
<220>
   <223> synthetic construct; CK19 promoter reverse primer
<400> 2
   tcccccttta ctcggccccc ac 22
<210> 3
   <211> 22
   <212> DNA
   <213> Artificial
<220>
   <223> artificial construct; GFP coding region forward primer
<400> 3
   ttcctgcgtt atcccctgat tc 22
<210> 4
   <211> 24
   <212> DNA
   <213> Artificial
<220>
   <223> sythetic construct; GFP coding region reverse primer
<400> 4
   gctcctccgg cccttgctca ccat 24